# EUROPEAN PATENT APPLICATION

(11) **EP 4 516 798 A1**
(43) Date of publication of application: **05.03.2025**
(21) Application number: 23795375.7
(22) Date of filing: 25.04.2023
(51) Int. Cl.: C07F 9/6561, A61K 31/675, A61P 31/14, A61P 31/16

(54) **NUCLEOSIDE DRUG FOR TREATING OR PREVENTING CORONAVIRUS INFECTION, AND USE THEREOF**

(30) Priority: 25.04.2022 CN 202210465512; 25.04.2022 US 202263363499 P; 30.09.2022 CN 202211217036; 01.11.2022 CN 202211358613; 01.11.2022 US 202263381846 P
(71) Applicant: Miracure Biotechnology Limited, Haidian, Beijing 100192 (CN)
(72) Inventor: LI, Xiaolin, Beijing 100192 (CN); QI, Longwu, San Diego, California 92121 (US); XU, Shusen, Beijing 100192 (CN); DU, Nana, Beijing 100192 (CN)
(74) Representative: Gille Hrabal Partnerschaftsgesellschaft mbB Patentanwälte
(86) International application number: PCT/CN2023/090488
(87) International publication number: WO 2023/207942

(57) **Abstract**

The present invention relates to the technical field of medicines, and in particular to a nucleoside drug for treating or preventing coronavirus infection, and a use thereof. The present invention further relates to a method for preparing a prodrug of a nucleoside drug. Compared with other nucleoside drugs such as remdesivir for injection, the nucleoside drug of formula (I) or formula (II) provided in the present invention is more suitable for oral administration to individuals in need.

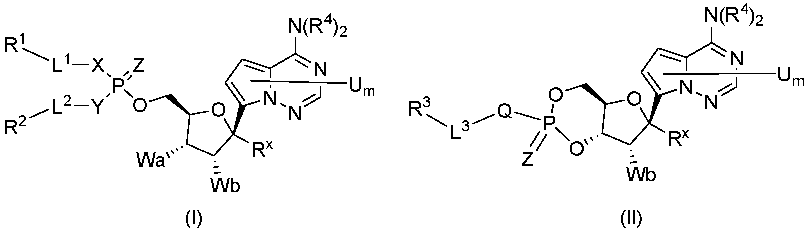

## Description

The present application claims the priorities to the following patent applications filed with the China National Intellectual Property Administration: CN202210465512.X filed on April 25, 2022, 202211217036.6 file on September 30, 2022 and CN202211358613.3 filed on November 1, 2022; and to the following patent applications filed with the United States Patent and Trademark Office: No. 63/363,499 filed on April 25, 2022 and No. 63/381,846 filed on November 1, 2022.

### TECHNICAL FIELD

The invention belongs to the technical field of medicine, and particularly relates to nucleoside drugs for the treatment or prevention of coronavirus infection and uses thereof. The present invention also relates to a method for preparing prodrugs of a nucleoside drug.

### BACKGROUND

Severe Acute Respiratory Syndrome Coronavirus 2 (SARS-CoV-2, also known as Novel Coronavirus for short) is an enveloped virus containing a relatively large positive-sense RNA encapsulated by a nucleocapsid protein (N). Three transmembrane proteins are integrated into the viral lipid envelope: the spike protein (S) and two smaller proteins, i.e., the membrane protein (M) and the envelope protein (E). The infection of novel Coronavirus begins with the binding of the spike protein (S protein) on the surface of the virus to the Angiotensin Converting Enzyme (ACE2) receptor on the cell surface, and mediates subsequent viral uptake and fusion.

After the coronavirus enters the host cell, it disintegrates and releases the nucleocapsid and viral RNA into the cytoplasm. The open reading frame (ORF1a/b) at the 5' end of the viral RNA encodes a polyprotein (pp1a and pp1a/b). pp1a and pp1ab can be cleaved by papain-like protease (PLpro) and 3CL protease (3CLpro, also known as Mpro) to generate nonstructural proteins (NSPs), such as RNA-dependent RNA polymerase (RdRp) and helicase. Viral RNA-dependent RNA polymerase (RdRp), also known as non-structural protein No. 12 (nsp 12), can assemble with multiple other non-structural proteins to form an efficient RNA synthesis "machine" to complete the genetic material RNA genome transcription and replication. As the core component of this transcriptional replication machine, RNA polymerase is one of the most important targets of antiviral drugs. Disrupting its function is expected to prevent virus replication and ultimately achieve therapeutic purposes. The drug reaearch and development and validation of the pharmacodynamic mechanism for the drug targets of the novel coronavirus, especially the RNA-dependent RNA polymerase, are crucial.

Recently, there is no therapeutic drugs with clear curative effect for SARS-CoV-2 infection, and clinical treatment is mainly based on isolation, antiviral, symptomatic support, etc. However, these treatments still cannot meet the clinical needs. Anti-coronavirus oral drug, Molnupiravir, from Merck has been approved by the European Medicines Agency for emergency use, and it is also the world's first approved anti-coronavirus oral drug that can be taken at home. Although monopiravir has approved by the U.S. FDA, it still has side effects and is prone to drug resistance. Therefore, it will be of great significance to carry out targeted drug research against SARS-CoV-2.

Remdesivir, as a drug with broad-spectrum anti-RNA virus activity, has a wide range of applications in the prevention and treatment of RNA virus diseases, but its use is limited due to its liver toxicity side effects and its inability to take orally.

### SUMMARY

In view of the deficiencies in the prior art and the needs in the art, the object of the present invention is to provide a series of medicinal compounds, which have virus inhibition mechanisms similar to those of remdesivir, but have higher inhibition effect on coronavirus, especially SARS-CoV-2 virus than remdesivir is stronger, and/or have the advantages of low toxicity, good safety, and can be taken orally, so that it can be used as a clinically effective medicinal compound for preventing, alleviating and/or treating coronavirus infection. This object is achieved by the subject matters described in the following aspects of the present application.

In the first aspect, the present disclosure provides a compound of formula (X) or (XI) or an isotopically-labeled compound, optical isomer, geometric isomer, tautomer or mixture of isomers, or a pharmaceutically acceptable salt, prodrug, metabolite, polymorph or solvate thereof, 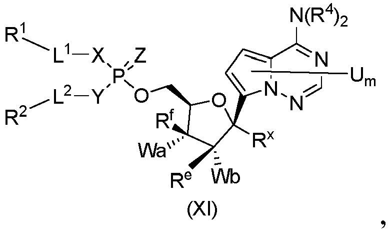 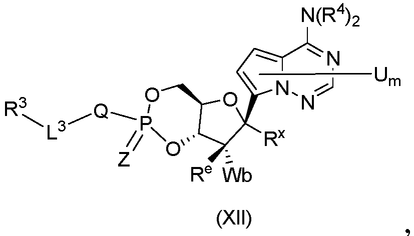 wherein,
U, independently of each other, represents H, D, halogen, C₁-C₁₂ alkyl or C₁-C₁₂ haloalkyl; m, independently of each other, represents an integer from 0 to 3, preferably represents 0, 1 or 2, more preferably represents 0 or 1;
R^{x}, independently of each other, represents H, OH, -OC₁-C₁₂ alkyl, CN, nitro, amino, halogen, C₁-C₁₂ haloalkyl;
Wa and Wb, independently of each other, represent H, OH, the following groups unsubstituted or substituted with one, two or more R^{w}: C₁-C₁₂ alkyl, C₂-C₁₂ alkenyl, C₂-C₁₂ alkynyl, C₁-C₁₂ alkyloxy, C₂-C₁₂ alkenyloxy, C₂-C₁₂ alkynyloxy, C₁-C₁₂ alkylcarbonyloxy, C₂-C₁₂ alkenylcarbonyloxy, C₂-C₁₂ alkynylcarbonyloxy, 5-12 membered heteroarylamino, benzimidamido, C₆-C₁₄ aryl, C₁-C₁₂ alkyloxycarbonyloxy, C₁-C₁₂ alkylcarbonylthio, C₆-C₂₀ aryl-C₁-C₁₂ alkylcarbonyloxy, C₃-C₁₂ cycloalkylcarbonyloxy, C₆-C₁₄ arylcarbonyloxy, C₃-C₁₂ cycloalkyloxycarbonyl, carboxyl, amino, 5-12 membered heteroarylcarbonyloxy, C₆-C₂₀ arylcarbonyloxy, 5-12 membered heterocyclylcarbonyloxy; wherein, R^{w}, independently of each other, represents di(C₁-C₆ alkyl)aminosulfonyl, C₁-C₁₂ alkyl, hydroxyl, carboxyl, amino, halogen;
Q, X, Y and Z, independently of each other, represent O, S or NH,
L¹ and L², independently of each other, represents a single bond, the following groups unsubstituted or optionally substituted with one, two or more R^{a}: C₁-C₁₂ alkylene, C₂-C₁₂ alkenylene, C₂-C₁₂ alkynylene, C₃-C₁₂ cycloalkylene, C₆-C₁₄ arylene, 5-12 membered heteroarylene, -(O-C₁-C₁₂ alkylene)ₙ-, -(C₁-C₁₂ alkylene-O-)ₙ-, C₁-C₁₂ alkylene-NH-C₁-C₁₂ alkylene, C₁-C₁₂ alkylene-C₆-C₁₄ arylene, C₆-C₁₄ arylene-C₁-C₁₂ alkylene; wherein, R^{a}, independently of each other, represents C₁-C₆ alkyl, C₁-C₆ haloalkyl, phenyl, aminoC₁-C₆ alkyl, di(C₁-C₆ alkyl)aminoC₁-C₆ alkyl, benzyloxycarbonylamino; n, independently of each other, represents an integer from 1 to 6, preferably an integer from 4 to 6;
R¹ and R², independently of each other, represents H, the following groups unsubstituted or substituted with one, two or more R^{b}: C₁-C₁₂ alkyl, C₂-C₁₂ alkenyl, C₂-C₁₂ alkynyl, C₁-C₁₂ alkyloxy, C₂-C₁₂ alkenyloxy, C₂-C₁₂ alkynyloxy, C₁-C₁₂ alkylcarbonyloxy, C₂-C₁₂ alkenylcarbonyloxy, C₂-C₁₂ alkynylcarbonyloxy, C₁-C₁₂ alkyloxycarbonyl, C₂-C₁₂ alkenyloxycarbonyl, C₂-C₁₂ alkynyloxycarbonyl, 5-12 membered heteroarylamino, benzimidamido, C₆-C₁₄ aryl, C₁-C₁₂ alkyloxycarbonyloxy, C₁-C₁₂ alkylcarbonylthio, C₆-C₂₀ aryl-C₁-C₁₂ alkylcarbonyloxy, C₃-C₁₂ cycloalkylcarbonyloxy, C₆-C₁₄ arylcarbonyloxy, C₃-C₁₂ cycloalkyloxycarbonyl, carboxyl, amino, 5-12 membered heteroarylcarbonyloxy, C₆-C₂₀ arylcarbonyloxy, 5-12 membered heterocyclylcarbonyloxy; wherein, R^{b}, independently of each other, represents benzyloxycarbonylamino, C₁-C₆ alkyl, C₁-C₆ haloalkyl, hydroxyl, carboxyl, amino, halogen, preferably Cl;
L³ represents a single bond, C₁-C₁₂ alkylene unsubstituted or optionally substituted with one, two or more group R^{c}; wherein R^{c}, independently of each other, represents C₁-C₆ alkyl, C₁-C₆ haloalkyl, hydroxyl, carboxyl, amino, halogen;
R³ represents H, the following groups unsubstituted or substituted with one, two or more group R^{d}: C₁-C₁₂ alkyl, C₂-C₁₂ alkenyl, C₂-C₁₂ alkynyl, C₁-C₁₂ alkyloxy, C₂-C₁₂ alkenyloxy, C₂-C₁₂ alkynyloxy, C₁-C₁₂ alkylcarbonyloxy, C₁-C₁₂ alkyloxycarbonyl, 5-12 membered heteroarylcarbonyloxy, C₆-C₂₀ arylcarbonyloxy, C₁-C₁₂ alkyloxycarbonyloxy, 5-12 membered heterocyclylcarbonyloxy, carboxyl, amino, C₁-C₁₂ alkylcarbonylthio, C₆-C₂₀ aryl-C₁-C₁₂ alkylcarbonyloxy, C₃-C₁₂ cycloalkylcarbonyloxy, C₆-C₁₄ aryloxycarbonyloxy, C₃-C₁₂ cycloalkyloxycarbonyl, C₃-C₁₂ cycloalkyloxycarbonyloxy; wherein, R^{d}, independently of each other, represents di(C₁-C₆ alkyl)aminosulfonyl, preferably di-(n-propyl)aminosulfonyl, C₁-C₆ alkyl, hydroxyl, carboxyl, amino, halogen, preferably Cl;
R⁴ represents H, R¹-L¹-X or R²-L²-Y;
R^{e} and R^{f}, independently of each other, represents C₁-C₁₂ alkyl, C₂-C₁₂ alkenyl, C₂-C₁₂ alkynyl, C₁-C₁₂ alkyloxy, C₂-C₁₂ alkenyloxy, C₂-C₁₂ alkynyloxy, halogen, OH, CN, -NO₂.

In the second aspect, the disclosure provides a compound of formula (I) or (II) or an isotopically-labeled compound, optical isomer, geometric isomer, tautomer or mixture of isomers, or a pharmaceutically acceptable salt, prodrug, metabolite, polymorph or solvate thereof, 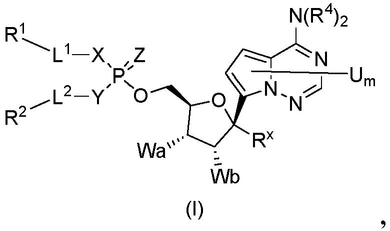 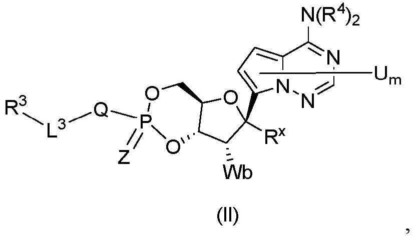 wherein,
U, independently of each other, represents H, D, halogen, C₁-C₁₂ alkyl or C₁-C₁₂ haloalkyl; m, independently of each other, represents an integer from 0 to 3, preferably represents 0, 1 or 2, more preferably represents 0 or 1;
R^{x}, independently of each other, represents H, OH, -OC₁-C₁₂ alkyl, CN, nitro, amino, halogen, C₁-C₁₂ haloalkyl;
Wa and Wb, independently of each other, represent H, OH, the following groups unsubstituted or substituted with one, two or more R^{w}: C₁-C₁₂ alkyl, C₂-C₁₂ alkenyl, C₂-C₁₂ alkynyl, C₁-C₁₂ alkyloxy, C₂-C₁₂ alkenyloxy, C₂-C₁₂ alkynyloxy, C₁-C₁₂ alkylcarbonyloxy, C₂-C₁₂ alkenylcarbonyloxy, C₂-C₁₂ alkynylcarbonyloxy, 5-12 membered heteroarylamino, benzimidamido, C₆-C₁₄ aryl, C₁-C₁₂ alkyloxycarbonyloxy, C₁-C₁₂ alkylcarbonylthio, C₆-C₂₀ aryl-C₁-C₁₂ alkylcarbonyloxy, C₃-C₁₂ cycloalkylcarbonyloxy, C₆-C₁₄ arylcarbonyloxy, C₃-C₁₂ cycloalkyloxycarbonyl, carboxyl, amino, 5-12 membered heteroarylcarbonyloxy, C₆-C₂₀ arylcarbonyloxy, 5-12 membered heterocyclylcarbonyloxy; wherein, R^{w}, independently of each other, represents di(C₁-C₆ alkyl)aminosulfonyl, C₁-C₁₂ alkyl, hydroxyl, carboxyl, amino, halogen;
Q, X, Y and Z, independently of each other, represent O, S or NH,
L¹ and L², independently of each other, represents a single bond, the following groups unsubstituted or optionally substituted with one, two or more R^{a}: C₁-C₁₂ alkylene, C₂-C₁₂ alkenylene, C₂-C₁₂ alkynylene, C₃-C₁₂ cycloalkylene, C₆-C₁₄ arylene, 5-12 membered heteroarylene, -(O-C₁-C₁₂ alkylene)ₙ-, -(C₁-C₁₂ alkylene-O-)ₙ-, C₁-C₁₂ alkylene-NH-C₁-C₁₂ alkylene, C₁-C₁₂ alkylene-C₆-C₁₄ arylene, C₆-C₁₄ arylene-C₁-C₁₂ alkylene; wherein, R^{a}, independently of each other, represents C₁-C₆ alkyl, C₁-C₆ haloalkyl, phenyl, aminoC₁-C₆ alkyl, di(C₁-C₆ alkyl)aminoC₁-C₆ alkyl, benzyloxycarbonylamino; n, independently of each other, represents an integer from 1 to 6, preferably an integer from 4 to 6;
R¹ and R², independently of each other, represents H, the following groups unsubstituted or substituted with one, two or more R^{b}: C₁-C₁₂ alkyl, C₂-C₁₂ alkenyl, C₂-C₁₂ alkynyl, C₁-C₁₂ alkyloxy, C₂-C₁₂ alkenyloxy, C₂-C₁₂ alkynyloxy, C₁-C₁₂ alkylcarbonyloxy, C₂-C₁₂ alkenylcarbonyloxy, C₂-C₁₂ alkynylcarbonyloxy, C₁-C₁₂ alkyloxycarbonyl, C₂-C₁₂ alkenyloxycarbonyl, C₂-C₁₂ alkynyloxycarbonyl, 5-12 membered heteroarylamino, benzimidamido, C₆-C₁₄ aryl, C₁-C₁₂ alkyloxycarbonyloxy, C₁-C₁₂ alkylcarbonylthio, C₆-C₂₀ aryl-C₁-C₁₂ alkylcarbonyloxy, C₃-C₁₂ cycloalkylcarbonyloxy, C₆-C₁₄ arylcarbonyloxy, C₃-C₁₂ cycloalkyloxycarbonyl, carboxyl, amino, 5-12 membered heteroarylcarbonyloxy, C₆-C₂₀ arylcarbonyloxy, 5-12 membered heterocyclylcarbonyloxy; wherein, R^{b}, independently of each other, represents benzyloxycarbonylamino, C₁-C₆ alkyl, C₁-C₆ haloalkyl, hydroxyl, carboxyl, amino, halogen, preferably Cl;
L³ represents a single bond, C₁-C₁₂ alkylene unsubstituted or optionally substituted with one, two or more group R^{c}; wherein R^{c}, independently of each other, represents C₁-C₆ alkyl, C₁-C₆ haloalkyl, hydroxyl, carboxyl, amino, halogen;
R³ represents H, the following groups unsubstituted or substituted with one, two or more group R^{d}: C₁- C₁₂ alkyl, C₂-C₁₂ alkenyl, C₂-C₁₂ alkynyl, C₁-C₁₂ alkyloxy, C₂-C₁₂ alkenyloxy, C₂-C₁₂ alkynyloxy, C₁-C₁₂ alkylcarbonyloxy, C₁-C₁₂ alkyloxycarbonyl, 5-12 membered heteroarylcarbonyloxy, C₆-C₂₀ arylcarbonyloxy, C₁-C₁₂ alkyloxycarbonyloxy, 5-12 membered heterocyclylcarbonyloxy, carboxyl, amino, C₁-C₁₂ alkylcarbonylthio, C₆-C₂₀ aryl-C₁-C₁₂ alkylcarbonyloxy, C₃-C₁₂ cycloalkylcarbonyloxy, C₆-C₁₄ aryloxycarbonyloxy, C₃-C₁₂ cycloalkyloxycarbonyl, C₃-C₁₂ cycloalkyloxycarbonyloxy; wherein, R^{d}, independently of each other, represents di(C₁-C₆ alkyl)aminosulfonyl, preferably di-(n-propyl)aminosulfonyl, C₁-C₆ alkyl, hydroxyl, carboxyl, amino, halogen, preferably Cl;
R⁴ represents H, R¹-L¹-X or R²-L²-Y.

In some preferred embodiments in the disclosure, the compound of formula (I) or (II) has a structure of formula (I-1) or (II-I), 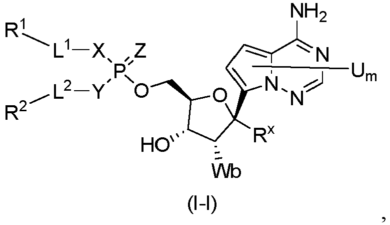 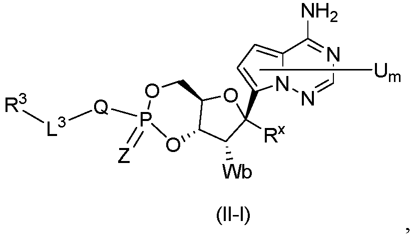 wherein,
U, independently of each other, represents H, D, halogen, C₁-C₁₂ alkyl or C₁-C₁₂ haloalkyl; m, independently of each other, represents an integer from 0 to 3, preferably represents 0, 1 or 2, more preferably represents 0 or 1;
R^{x}, independently of each other, represents H, OH, -OC₁-C₁₂ alkyl, CN, nitro, amino, halogen, C₁-C₁₂ haloalkyl;
Wb, independently of each other, represents H or OH;
Q, X, Y and Z, independently of each other, represent O, S or NH,
L¹ and L², independently of each other, represents a single bond, the following groups unsubstituted or optionally substituted with one, two or more R^{a}: C₁-C₁₂ alkylene, C₂-C₁₂ alkenylene, C₂-C₁₂ alkynylene, C₃-C₁₂ cycloalkylene, C₆-C₁₄ arylene, 5-12 membered heteroarylene, -(O-C₁-C₁₂ alkylene)ₙ-, C₁-C₁₂ alkylene-NH-C₁-C₁₂ alkylene; wherein, R^{a}, independently of each other, represents C₁-C₆ alkyl, C₁-C₆ haloalkyl, phenyl, aminoC₁-C₆ alkyl, di(C₁-C₆ alkyl)aminoC₁-C₆ alkyl; n, independently of each other, represents an integer from 1 to 6, preferably an integer from 4 to 6;
R¹ and R², independently of each other, represents H, the following groups unsubstituted or substituted with one, two or more R^{b}: C₁-C₁₂ alkyl, C₂-C₁₂ alkenyl, C₂-C₁₂ alkynyl, C₁-C₁₂ alkyloxy, C₂-C₁₂ alkenyloxy, C₂-C₁₂ alkynyloxy, C₁-C₁₂ alkylcarbonyloxy, C₁-C₁₂ alkyloxycarbonyl, 5-12 membered heteroarylamino, benzimidamido, C₆-C₁₄ aryl, C₁-C₁₂ alkyloxycarbonyloxy, carboxyl, amino, 5-12 membered heteroarylcarbonyloxy, 5-12 membered heterocyclylcarbonyloxy; wherein, R^{b}, independently of each other, represents C₁-C₆ alkyl, C₁-C₆ haloalkyl, hydroxyl, carboxyl, amino, halogen, preferably Cl;
L³ represents a single bond, C₁-C₁₂ alkylene unsubstituted or optionally substituted with one, two or more group R^{c};
R³ represents H, the following groups unsubstituted or substituted with one, two or more group R^{d}: C₁-C₁₂ alkyl, C₂-C₁₂ alkenyl, C₂-C₁₂ alkynyl, C₁-C₁₂ alkyloxy, C₂-C₁₂ alkenyloxy, C₂-C₁₂ alkynyloxy, C₁-C₁₂ alkylcarbonyloxy, C₁-C₁₂ alkyloxycarbonyl, 5-12 membered heteroarylcarbonyloxy, C₆-C₂₀ arylcarbonyloxy, C₁-C₁₂ alkyloxycarbonyloxy, 5-12 membered heterocyclylcarbonyloxy, carboxyl, amino; wherein, R^{d}, independently of each other, represents di(C₁-C₆ alkyl)aminosulfonyl, preferably di-(n-propyl)aminosulfonyl, C₁-C₆ alkyl, hydroxyl, carboxyl, amino, halogen, preferably Cl.

In some preferred embodiments in the disclosure, R⁴, independently of each other, represents H.

In some preferred embodiments in the disclosure, one R⁴ represents H and the other R⁴ represents R¹-L¹-X or R²-L²-Y.

In some preferred embodiments in the disclosure, the compound of formula (I) or (II) has a structure of the following formula (IA) or (IIA): 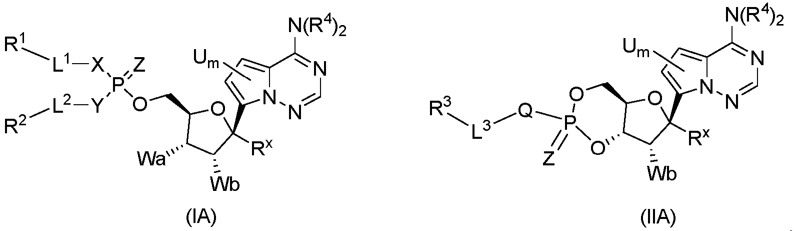 wherein, R¹, R², R³, R⁴, R^{x}, L¹, L², L³, Q, U, Wa, Wb, X, Y, Z are as defined in formula (I) or (II), preferably, U represents deuterium (D).

In some preferred embodiments in the disclosure, the compound of formula (I) or (II) has a structure of the following formula (IB) or (IIB): 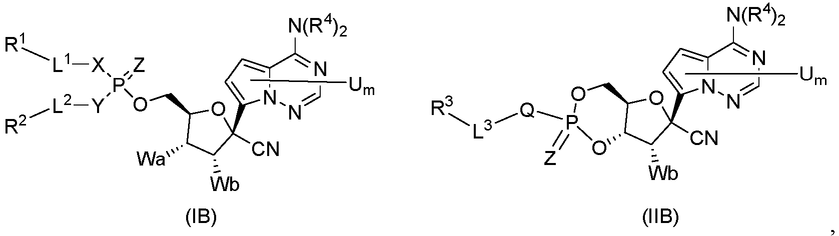 wherein, R¹, R², R³, R⁴, L¹, L², L³, Q, U, Wa, Wb, X, Y, Z are as defined in formula (I) or (II), preferably, U represents deuterium(D).

In some preferred embodiments in the disclosure, the compound of formula (I) or (II) has a structure of the following formula (IC) or (IIC): 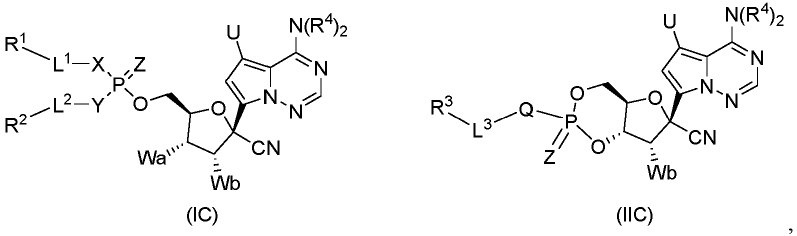 wherein, R¹, R², R³, R⁴, L¹, L², L³, Q, U, Wa, Wb, X, Y, Z are as defined in formula (I) or (II), preferably, U represents deuterium(D).

In some preferred embodiments in the disclosure, Wa and Wb in the fomula (I), (I-I), (IA), (IB) or (IC) represent OH. In some other embodiments, Wb in the fomula (II), (II-I), (IIA), (IIB) or (IIC) represent OH.

In some embodiments, Wa and Wb, independently of each other, represent H, OH, the following groups unsubstituted or substituted with one, two or more R^{w}: C₁-C₆ alkylcarbonyloxy, C₂-C₆ alkenylcarbonyloxy, C₂-C₆ alkynylcarbonyloxy, C₃-C₁₂ cycloalkylcarbonyloxy, 5-12 membered heteroarylcarbonyloxy, C₆-C₂₀ arylcarbonyloxy, 5-12 membered heterocyclylcarbonyloxy; wherein, R^{w}, independently of each other, represents di(C₁-C₆ alkyl)aminosulfonyl, preferably di(n-propyl)aminosulfonyl, C₁-C₆ alkyl, hydroxyl, carboxyl, amino, halogen; preferably Wa and Wb, independently of each other, represent OH, acetoxy, isopropanoyloxy, isobutyryloxy, 2-propylpentanoyloxy, 2,2-dimethylpropanoyloxy, 4-(di(n-propyl)aminosulfonyl)benzoyloxy, pyridine-3-carbonoyloxy, N-methylpyrrolidin-2-ylcarbonoyloxy, prolyloxy, cyclopentylcarbonoyloxy.

In some preferred embodiments in the disclosure, L¹ and L², independently of each other, preferably represents a single bond, C₁-C₆ alkylene, C₆-C₁₀ arylene, C₃-C₆ cycloalkylene, 5-8 membered heteroarylene, -(O-C₁-C₆ alkylene)ₙ-, C₁-C₆ alkylene-NH-C₁-C₆ alkylene; more preferably, L¹ and L², independently of each other, represents a single bond, methylene, ethylene, propylene, phenylene, cyclopentylene, cyclohexylene, oxadiazolylene, pyrazolylene, imidazolylene, thiazolylene, -(OCH₂CH₂)ₙ-, C₁-C₆ alkylene-NH-C₁-C₃ alkylene; most preferably, L¹ and L², independently of each other, represents a single bond, methylene, 1,1-ethylene, 2,2-propylene, 2-phenyl-1,1-ethylene, 1,4-phenylene, 1,2,4-oxadiazol-3,5-diyl, -(OCH₂CH₂)₄-, pentylene-N(C₂H₅)-CH₂CH₂-.

In some preferred embodiments in the disclosure, R¹ and R², independently of each other, preferably represents H, the following groups unsubstituted or substituted with one, two or more R^{b}: C₁-C₆ alkyl, C₆-C₁₂ alkylcarbonyloxy, C₁-C₆ alkyloxycarbonyl, 5-12 membered heteroarylamino, benzimidamido, C₆-C₁₄ aryl, C₁-C₆ alkyloxycarbonyloxy, C₃-C₆ cycloalkylcarbonyloxy, C₃-C₆ cycloalkyloxycarbonyl, carboxyl, amino, 5-12 membered heteroarylcarbonyloxy, 5-12 membered heterocyclylcarbonyloxy, wherein R^{b}, independently of each other, represents benzyloxycarbonylamino, C₁-C₆ alkyl, C₁-C₆ haloalkyl, hydroxy, carboxyl, amino, fluoro, chloro or bromo; more preferably, R¹ and R², independently of each other, represents H, methyl, heptylcarbonyloxy, methyloxycarbonyl, ethyloxycarbonyl, propyloxycarbonyl, butyloxycarbonyl, hexyloxycarbonyl, quinolinylamino, benzimidamido, propyloxycarbonyloxy, carboxyl, amino, nicotinoyloxy, N-methyl-hydroxylpyrrolidinylcarbonyl, cyclopentylcarbonoyloxy, cyclopentyloxycarbonoyl; most preferably, R¹ and R² are independently of each other selected from the following atoms or groups:
H, phenyl, methyl, carboxyl, amino,

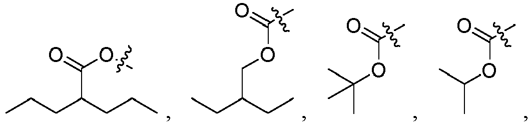

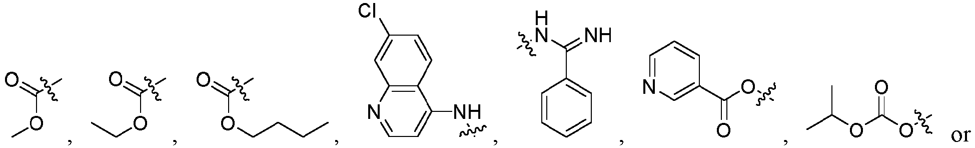

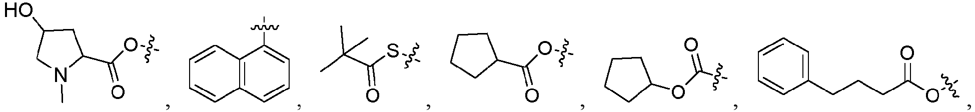

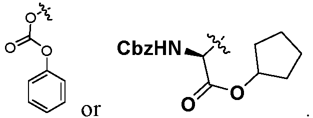

In some preferred embodiments in the disclosure, R¹ and R² are both

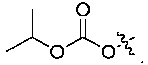

In some preferred embodiments in the disclosure, L³ preferably represents a single bond or C₁-C₆ alkylene unsubstituted or optionally substituted with one, two or more group R^{c}, wherein R^{c}, independently of each other, represents C₁-C₆ alkyl, C₁-C₆ haloalkyl, hydroxy, carboxyl, amino, halogen; more preferably, L³ represents a single bond, methylene or ethylene; most preferably, L³ represents a single bond, methylene, 1,1-ethylene.

In some preferred embodiments in the disclosure, R³ preferably represents H, the following groups unsubstituted or substituted with one, two or more group R^{d}: C₁-C₆ alkyl, C₆-C₁₂ alkylcarbonyloxy, C₁-C₆ alkyloxycarbonyl, 5-12 membered heteroarylcarbonyloxy, benzimidamido, C₆-C₁₄ arylcarbonyloxy, C₁-C₆ alkyloxycarbonyloxy, 5-12 membered heterocyclylcarbonyloxy, N-methyl-hydroxylpyrrolidinylcarbonyl, carboxyl, amino, C₁-C₆ alkylcarbonylthio, C₆-C₁₄ aryl-C₁-C₆ alkylcarbonyloxy, C₆-C₁₄ aryloxycarbonyloxy, C₃-C₆ cycloalkyloxycarbonyloxy; more preferably, R³ represents H, methyl, heptylcarbonyloxy, methyloxycarbonyl, ethyloxycarbonyl, propyloxycarbonyl, butyloxycarbonyl, hexyloxycarbonyl, propyloxycarbonyloxy, isopropyloxycarbonyloxy, benzoyl, nicotinoyloxy, carboxyl, amino, tert-butylcarbonylthio, phenylpropylcarbonyloxy, phenoxycarbonyloxy, cyclobutyloxycarbonyloxy, cyclopentyloxycarbonyloxy; most preferably, R³ is selected from the following atoms or groups: H, phenyl, methyl, carboxyl, amino,

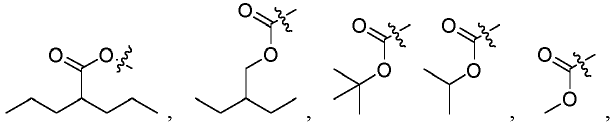

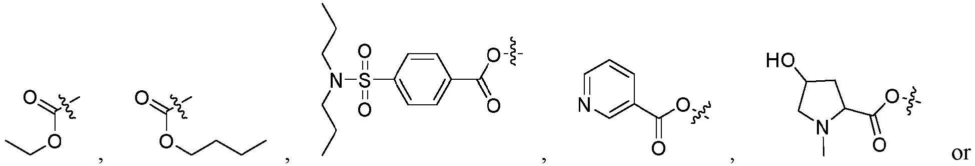

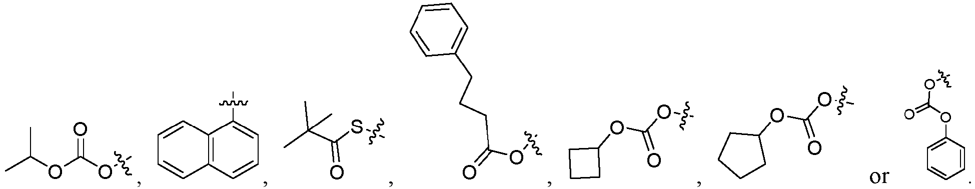

In some preferred embodiments in the disclosure, R¹-L¹-X- and R²-L²-Y-, independently of each other, represents the following groups:

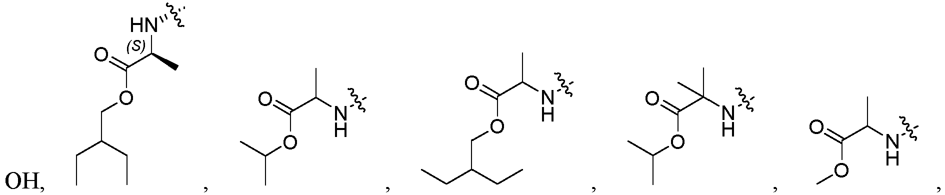

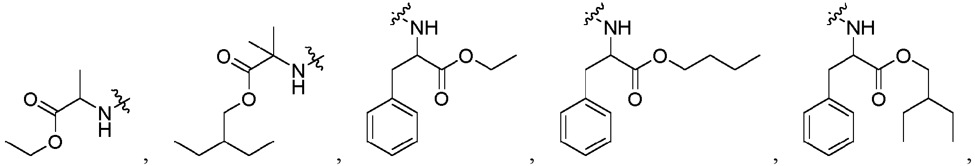

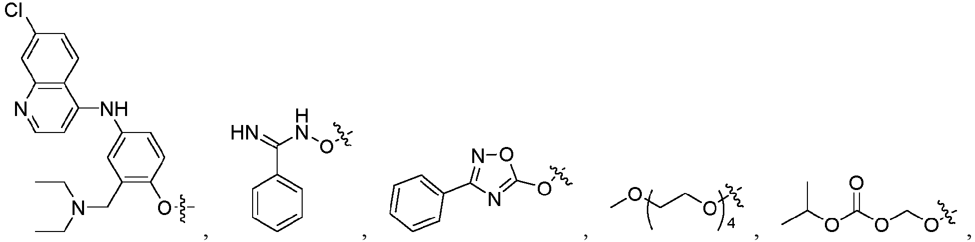

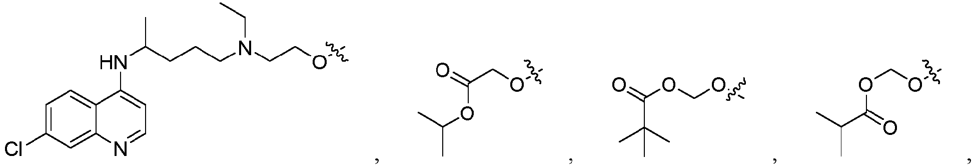

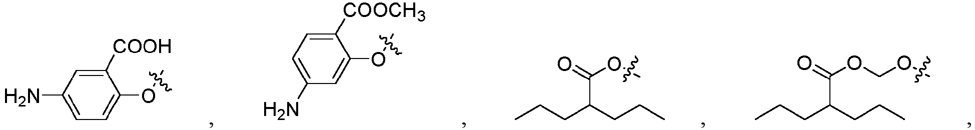

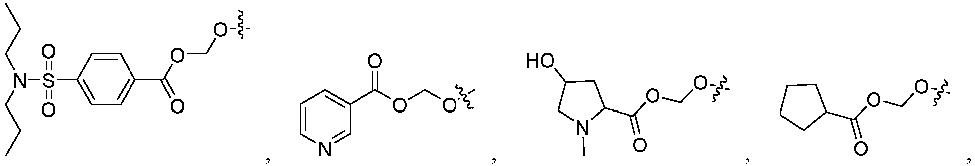

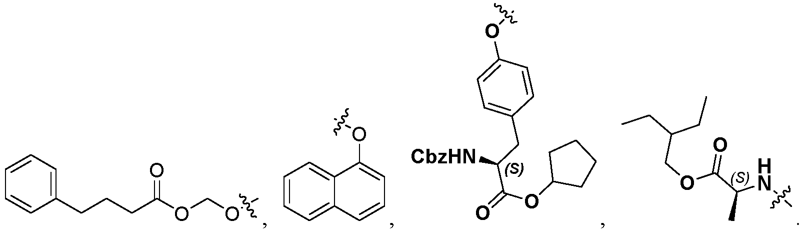

In some preferred embodiments in the disclosure, R³-L³-Q- represents the following groups:

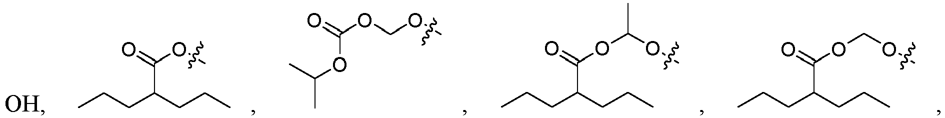

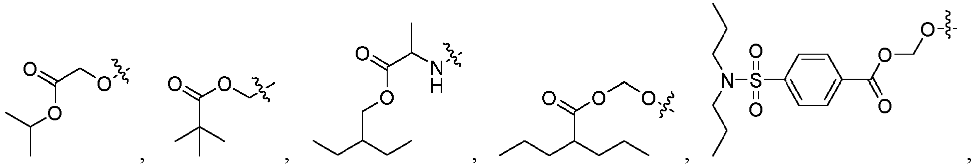

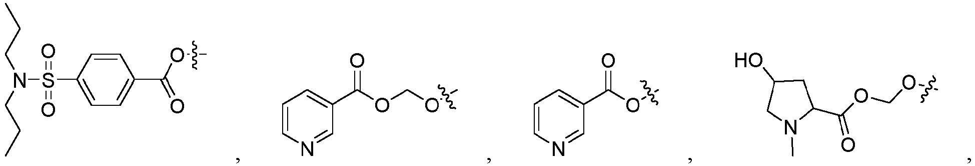

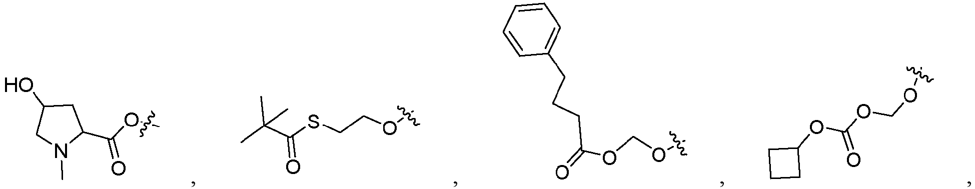

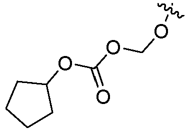

In some preferred embodiments in the disclosure, the compound of formula (X) or (XI), formula (I) or (II), formula (I-I) or (II-I), formula (IA) or (IIA), formula (IB) or (IIB), formula (IC) or (IIC) is selected from those in Tables 1~9 below.

In the third aspect, it is disclosed herein a pharmaceutical composition comprising a compound of formula (X) or (XI), formula (I) or (II), formula (I-I) or (II-I), formula (IA) or (IIA), formula (IB) or (IIB), formula (IC) or (IIC) as defined herein, or an isotopically-labeled compound, optical isomer, geometric isomer, tautomer isomer or a mixture of isomers, or a pharmaceutically acceptable salt, prodrug, metabolite, polymorph or solvate thereof.

In some preferred embodiments in the disclosure, the pharmaceutical composition disclosed herein may also optionally contain at least one physiologically/pharmaceutically acceptable excipient.

In some preferred embodiments in the disclosure, the pharmaceutical composition disclosed herein may also optionally comprise additional active ingredients. The additional active ingredient is, for example, a fusion inhibitor, a viral entry inhibitor, a protease inhibitor, a polymerase inhibitor, an antiviral nucleoside drug and derivates thereof other than remdesivir, a viral maturation inhibitor, JAK inhibitor, angiotensin converting enzyme 2 (ACE2) inhibitor, a SARS-CoV specific human monoclonal antibody, a compound for inhibiting cytokine storm, preferably ribavirin, sofosbuvir, GS-441524, palivizumab, motavizumab and the like.

In some preferred embodiments in the disclosure, the pharmaceutical composition disclosed herein comprises a therapeutically effective amount of a compound of formula (X) or (XI), formula (I) or (II), formula (I-I) or (II-I), formula (IA) or (IIA), formula (IB) or (IIB), formula (IC) or (IIC) or an isotopically-labeled compound, optical isomer, geometric isomer, tautomer isomer or a mixture of isomers, or a pharmaceutically acceptable salt, prodrug, metabolite, polymorph or solvate thereof.

In some preferred embodiments in the disclosure, the pharmaceutical composition disclosed herein may inhibit the replication of RNA virus, preferably orthomyxovirus (for example, influenza virus) or paramyxovirus (for example, coronavirus or respiratory syncytial virus).

In some preferred embodiments in the disclosure, the pharmaceutical composition disclosed herein is used for the prevention or treatment of coronavirus infection or a disease or symptom caused by coronavirus. Preferably, the coronavirus is selected from severe acute respiratory syndrome coronavirus (SARS-CoV), novel coronavirus (SARS-CoV-2), Middle East respiratory syndrome coronavirus (MERS-CoV), coronavirus 229E (HCoV-229E), coronavirus OC43 (HCoV-OC43), murine hepatitis coronavirus (MHV), human coronavirus NL63, human coronavirus HKUl, and coronaviruses with more than 85% homology and viral activity with any of the above coronaviruses. More preferably, the coronavirus is the novel coronavirus (SARS-CoV-2). Alternatively, the coronavirus is selected from porcine epidemic diarrhea virus (PEDV) or feline infectious peritonitis virus.

In some preferred embodiments in the disclosure, a disease or symptom caused by coronavirus comprise respiratory infections caused by coronavirus, acute respiratory syndrome (SARS), pneumonia (including severe pneumonia), gastroenteritis (including acute gastroenteritis), cough, fever, chills, emesis, headache, cold intolerance, tachypnea, cytokine storm and the like.

In the disclosure, the pharmaceutical composition disclosed herein may be formulated into a dosage form suitable for administration by methods known in the art.

In the fourth aspect, it is disclosed herein the use of the compound of formula (X) or (XI), formula (I) or (II), formula (I-I) or (II-I), formula (IA) or (IIA), formula (IB) or (IIB), formula (IC) or (IIC) as disclosed herein, or an isotopically-labeled compound, optical isomer, geometric isomer, tautomer isomer or a mixture of isomers, or a pharmaceutically acceptable salt, prodrug, metabolite, polymorph or solvate thereof in the preparation of a medicine.

In some preferred embodiments in the disclosure, the medicine may also optionally comprise additional active ingredients. The additional active ingredient is, for example, a fusion inhibitor, a viral entry inhibitor, a protease inhibitor, a polymerase inhibitor, an antiviral nucleoside drug and derivates thereof other than remdesivir, a viral maturation inhibitor, JAK inhibitor, angiotensin converting enzyme 2 (ACE2) inhibitor, a SARS-CoV specific human monoclonal antibody, a compound for inhibiting cytokine storm, preferably ribavirin, sofosbuvir, GS-441524, palivizumab, motavizumab and the like.

In some preferred embodiments in the disclosure, the medicine is used for inhibiting the replication of RNA virus, preferably orthomyxovirus (for example, influenza virus) or paramyxovirus (for example, coronavirus or respiratory syncytial virus).

In some preferred embodiments in the disclosure, the medicine is used for the prevention or treatment of coronavirus infection or a disease or symptom caused by coronavirus. Preferably, the coronavirus is selected from severe acute respiratory syndrome coronavirus (SARS-CoV), novel coronavirus (SARS-CoV-2), Middle East respiratory syndrome coronavirus (MERS-CoV), coronavirus 229E (HCoV-229E), coronavirus OC43 (HCoV-OC43), murine hepatitis coronavirus (MHV), human coronavirus NL63, human coronavirus HKUl, and coronaviruses with more than 85% homology and viral activity with any of the above coronaviruses. More preferably, the coronavirus is the novel coronavirus (SARS-CoV-2). Alternatively, the coronavirus is selected from porcine epidemic diarrhea virus (PEDV) or feline infectious peritonitis virus.

In the fifth aspect, it is disclosed herein a method for the treatment or prevention of a disease or symptom caused by RNA virus, preferably orthomyxovirus (for example, influenza virus) or paramyxovirus (for example, coronavirus or respiratory syncytial virus), the method comprising administering to a subject in need thereof the compound of formula (X) or (XI), formula (I) or (II), formula (I-I) or (II-I), formula (IA) or (IIA), formula (IB) or (IIB), formula (IC) or (IIC) as disclosed herein, or an isotopically-labeled compound, optical isomer, geometric isomer, tautomer isomer or a mixture of isomers, or a pharmaceutically acceptable salt, prodrug, metabolite, polymorph or solvate thereof.

In some preferred embodiments in the disclosure, the compound disclosed herein may be administered orally, parenterally, via intravenous injection, via intramuscular injection, cia subcutaneous injection, nasally, via oral mucosa, ocularly, pulmonal, via respiratory tract, vaginal, rectal, intraperitoneal, intralesional, perilesional routes and the like.

In some preferred embodiments in the disclosure, the compound of formula (X) or (XI), formula (I) or (II), formula (I-I) or (II-I), formula (IA) or (IIA), formula (IB) or (IIB), formula (IC) or (IIC) as disclosed herein, or an isotopically-labeled compound, optical isomer, geometric isomer, tautomer isomer or a mixture of isomers, or a pharmaceutically acceptable salt, prodrug, metabolite, polymorph or solvate thereof may administered to a subject in need thereof in combination with the following active ingredients: a fusion inhibitor, a viral entry inhibitor, a protease inhibitor, a polymerase inhibitor, an antiviral nucleoside drug and derivates thereof other than remdesivir, a viral maturation inhibitor, JAK inhibitor, angiotensin converting enzyme 2 (ACE2) inhibitor, a SARS-CoV specific human monoclonal antibody, a compound for inhibiting cytokine storm, preferably ribavirin, sofosbuvir, GS-441524, palivizumab, motavizumab and the like.

The dosage specifically administered will depend on the route of administration, the severity of the disease, age and weight of the patient and other factors normally considered by the attending physician, when determining the subject regimen and dosage level as the most appropriate for a particular patient. For example, the daily dosage of the compound of formula (X) or (XI), formula (I) or (II), formula (I-I) or (II-I), formula (IA) or (IIA), formula (IB) or (IIB), formula (IC) or (IIC) as disclosed herein may be particularly 0.001-150mg/kg body weight (0.1mg/kg body weight, 1mg/kg body weight, 10mg/kg body weight or 100mg/kg body weight etc.).

The specific administration frequency may be determined by those skilled in the relevant art, for example, once every day, once every 2 days, once every 3 days, once every 4 days, once every 5 days, once every 6 days, twice every day, three times every day etc.

In the sixth aspect, it is disclosed herein the compound of formula (X) or (XI), formula (I) or (II), formula (I-I) or (II-I), formula (IA) or (IIA), formula (IB) or (IIB), formula (IC) or (IIC) as disclosed herein, or an isotopically-labeled compound, optical isomer, geometric isomer, tautomer isomer or a mixture of isomers, or a pharmaceutically acceptable salt, prodrug, metabolite, polymorph or solvate thereof for inhibiting the replication of RNA virus, preferably orthomyxovirus (for example, influenza virus) or paramyxovirus (for example, coronavirus or respiratory syncytial virus) or for the treatment or prevention of a disease or symptom caused by RNA virus, preferably orthomyxovirus (for example, influenza virus) or paramyxovirus (for example, coronavirus or respiratory syncytial virus).

Preferably, the coronavirus is selected from severe acute respiratory syndrome coronavirus (SARS-CoV), novel coronavirus (SARS-CoV-2), Middle East respiratory syndrome coronavirus (MERS-CoV), coronavirus 229E (HCoV-229E), coronavirus OC43 (HCoV-OC43), murine hepatitis coronavirus (MHV), human coronavirus NL63, human coronavirus HKUl, and coronaviruses with more than 85% homology and viral activity with any of the above coronaviruses. More preferably, the coronavirus is the novel coronavirus (SARS-CoV-2). Alternatively, the coronavirus is selected from porcine epidemic diarrhea virus (PEDV) or feline infectious peritonitis virus.

In the seventh aspect, it is disclosed herein a method of forming a prodrug of a nucleoside drug, optionally comprising the steps of:
(S-A) reacting the p-nitrophenyl phosphate compound (V) with a (deoxy)nucleoside drug (VI) having free hydroxyl groups at positions C5 to obtain a prodrug compound of formula (VII), 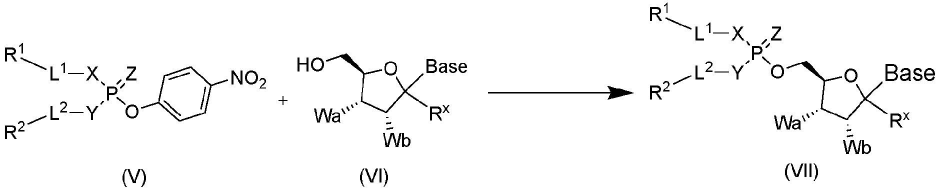
   wherein, R¹, R², R^{x}, L¹, L², Wa, Wb, X, Y, Z are as defined in formula (I) or (II) or any preferred embodiment thereof, the base may be selected from purine or pyrimidine bases or any basic (hetero)aryl group, preferably selected from guanine, adenine, thymine, uracil, cytosine bases or 4-aminopyrrolo[2,1-f][1,2,4]triazin-7-yl;
   preferably, the compound of formula (VI) is GS-441524; preferably, step (S-A) is performed under basic conditions; more preferably, step (S-A) is performed under basic conditions and in the presence of a Grignard reagent; most preferably, step (S-A) is performed in the presence of tert-butylmagnesium bromide;
   or,
(S-B) reacting the (deoxy)nucleoside drug phosphate (IX) with a phenolic compound (VIII) to obtain a prodrug compound of formula (X), 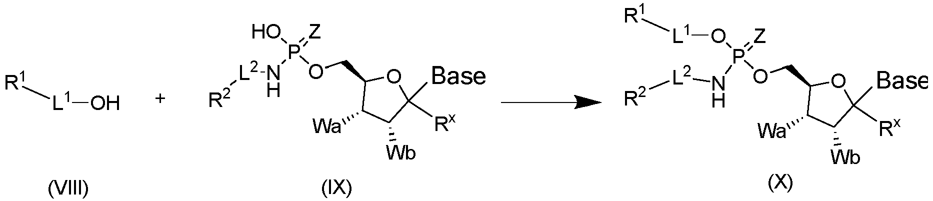 wherein, R¹, R², R^{x}, L¹, L², Wa, Wb, Z are as defined in formula (I) or (II) or any preferred embodiment thereof, L¹ represents C₆-C₁₄ arylene or 5-12 membered heteroarylene, preferably C₆-C₁₀ arylene or 5-8 membered heteroarylene, more preferably 1,4-phenylene or 1,2,4-oxadiazol-3,5-diyl, the base may be selected from purine or pyrimidine bases or any basic (hetero)aryl group, preferably selected from guanine, adenine, thymine, uracil, cytosine bases or 4-aminopyrrolo[2,1-f][1,2,4]triazin-7-yl,
   or,
(S-C) converting the (deoxy)nucleoside drug phosphate (XI) into a silver salt (XII) firstly and then reacting the silver salt (XII) with the halides R¹-L¹-Hal and/or R²-L²-Hal, to obtain a prodrug compound of formula (XIII), 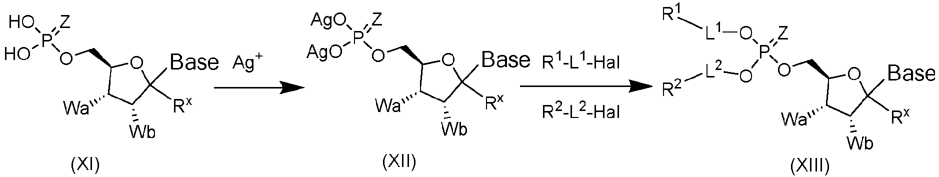 wherein, R¹, R², R^{x}, L¹, L², Wa, Wb, X, Y, Z are as defined in formula (I) or any preferred embodiment thereof, the base may be selected from purine or pyrimidine bases or any basic (hetero)aryl group, preferably selected from guanine, adenine, thymine, uracil, cytosine bases or 4-aminopyrrolo[2,1-f][1,2,4]triazin-7-yl; Hal represents halogen, preferably Br or I, more preferably I; preferably, the silver salt is silver carbonate;
   or,
(S-D) reacting cyclic (deoxy)nucleoside drug phosphate (XIV) with halide R³-L³-Hal in the presence of a basic compound, to obtain a prodrug compound of formula (XV), 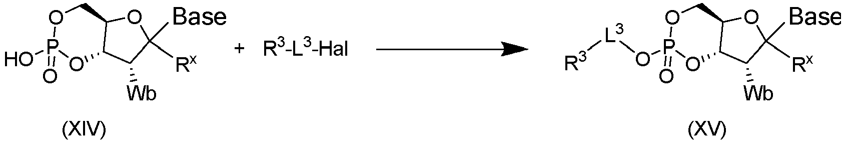 wherein, R^{x}, R³, L³ and Wb are as defined in formula (II) or any preferred embodiment thereof, the base may be selected from purine or pyrimidine bases or any basic (hetero)aryl group, preferably selected from guanine, adenine, thymine, uracil, cytosine bases or 4-aminopyrrolo[2,1-f][1,2,4]triazin-7-yl; Hal represents halogen, preferably Br or Cl, more preferably Cl; the basic compound is preferably an alkali metal carbonate, more preferably cesium carbonate;
   or,
(S-E) converting the (deoxy)nucleoside drug phosphate (XVI) into a silver salt (XVII) firstly and then reacting the silver salt (XVII) with the halide R³-L³-Hal, to obtain a prodrug compound of formula (XV), 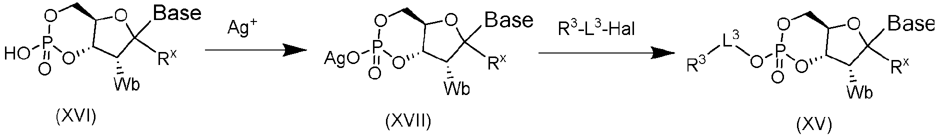 wherein, R^{x}, R³, L³ and Wb are as defined in formula (II) or any preferred embodiment thereof, the base may be selected from purine or pyrimidine bases or any basic (hetero)aryl group, preferably selected from guanine, adenine, thymine, uracil, cytosine bases or 4-aminopyrrolo[2,1-f][1,2,4]triazin-7-yl; Hal represents halogen, preferably Br or I, more preferably I;
   or,
(S-F) reacting the phosphoramidite compound (XVIII) with a (deoxy)nucleoside drug (VI) having free hydroxyl groups at positions C3 and C5 to obtain a prodrug compound of formula (XIX), 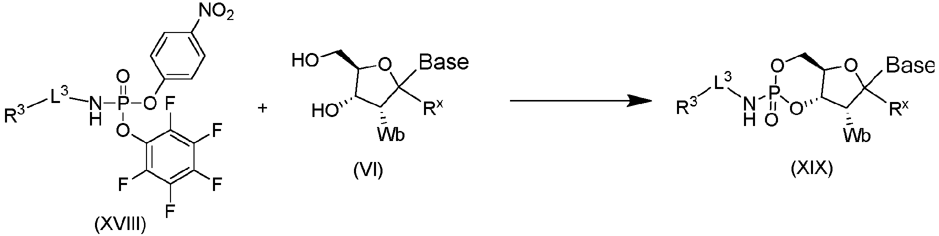 wherein, R^{x}, R³, L³ and Wb are as defined in formula (II) or any preferred embodiment thereof, the base may be selected from purine or pyrimidine bases or any basic (hetero)aryl group, preferably selected from guanine, adenine, thymine, uracil, cytosine bases or 4-aminopyrrolo[2,1-f][1,2,4]triazin-7-yl; preferably, step (S-F) is performed under basic conditions; more preferably, step (S-F) is performed under basic conditions and in the presence of a Grignard reagent; most preferably, step (S-F) is performed in the presence of tert-butylmagnesium bromide.

In some preferred embodiments of the methods of forming the prodrugs of a nucleoside drug herein, W represents OH, R^{x} represents cyano and the base is 4-aminopyrrolo[2,1-f][1,2,4]triazin-7-yl. Therefore, the nucleoside drug is preferably GS-441524.

Unexpectedly, the inventors found that a nucleoside drug can be converted into its prodrug form by phosphorylating the hydroxyl group at C5 or the hydroxyl groups at C3, C5 of the nucleoside drug, so that it is more suitable for oral administration. Especially when the base of the nucleoside moiety is 4-aminopyrrolo[2,1-f][1,2,4]triazin-7-yl, a higher concentration of GS-443902 can be detected in lung tissue.

Herein, when referring to the position of the carbon atom in the molecule of the nucleoside drug compound, the numbering of the carbon atom usually used in the art is used. Taking the ribofuranose used to form the ribonucleoside sugar group as an example, the carbon atom in the form of an acetal is designated as the C1 position, and the carbon atom with a primary hydroxyl group is designated as the C5 position, and then the remaining carbon atoms are numbered sequentially.

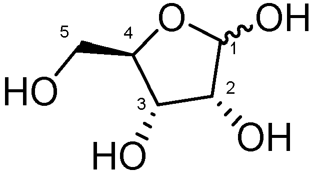

If necessary, a protecting group may be used to protect any group of the reactant or intermediate in the above method. After the corresponding reaction is completed, a suitable method is selected to remove the protecting group.

The starting materials of the reactants may be synthesized by the method in the art or are available from commercial sources, e.g., the starting materials are generally available from Aldrich, or can be readily prepared using methods well known to those skilled in the art (obtained by SciFinder and Reaxys online databases).

The method for forming a prodrug of a nucleoside drug herein may comprise suitable reaction conditions and raw materials that can be selected according to each situation, for example, only one substituent can be replaced with another substituent as defined herein in a reaction, or more substituents are replaced in one reaction step with other substituents as defined herein.

In the disclosure, as for the method for forming a prodrug of a nucleoside drug, after the reaction is completed, the reaction product can be processed by a conventional after-treatment method; conventional technical means e.g. preparative high performance liquid chromatography, preparative thin layer chromatography or recrystallization may be used for separation and purification to obtain the prodrug compounds in a desired purity.

It will be understood by those skilled in the art that the features recited in the various aspects of the disclosure and in the various embodiments can be freely combined as long as they do not conflict with each other.

### Benificial effects of the invention

A class of modified nucleoside drugs (prodrugs) are provided in the disclosure, which are more suitable for oral administration to subjects in need as compared to other nucleoside drugs, such as remdesivir for injection. It is found that, in the compound of formula (X) or (XI), formula (I) or (II), formula (I-I) or (II-I), formula (IA) or (IIA), formula (IB) or (IIB), formula (IC) or (IIC) in the disclosure, when the base of the nucleoside moiety is 4-aminopyrrolo[2,1-f][1,2,4]triazine-7-yl, higher concentrations of GS-443902 (a triphosphate metabolite of remdesivir known in the art, it is a potent viral RNA-dependent RNA polymerase inhibitor) can be detected in the tissues of lung.

### Embodiments

The present invention is further described in detail below.

Unless otherwise specified, the following terms used herein have the meanings explained below, and their definitions as embodiments, exemplary definitions, preferred definitions, definitions in the tables, and definitions of specific compounds in the examples, etc. may be in combination with and incorporation into each other; terms not explained in detail shall have the same meaning as commonly understood by those skilled in the art; patent and non-patent literatures or otherwise disclosed materials cited herein in whole or in part are all incorporated herein by reference.

### Terms

Herein, the terms "include", "comprise" and/or "contain" are open-ended expressions, i.e., comprising the contents specified herein, but not excluding other aspects. When describing one, two or more, "more" shall refer to the instance of greater than 2, such as the instance of an integer greater than or equal to 3, such as 3, 4, 5, 6 , 7, 8, 9 or 10. The term "optional/optionally" denotes the presence or absence of the recited feature, which means that the event subsequently described may, but need not, occur, and thus includes both instances in which the event occurs or does not. For example, "heterocyclic group optionally substituted with an alkyl group" means that the alkyl group may, but not necessarily, be present, thus including the instance of a heterocyclic group substituted with an alkyl group and a heterocyclic group not substituted with an alkyl group. The expressions "X is selected from A, B or C", "X is selected from A, B and C", "X is A, B and/or C", "X is A, B and/or C", etc. all express the same meaning, that is, X can be any one, two or more of A, B and C.

As used herein, the term "unsubstituted" means that a certain hydrogen atom or some of the hydrogen atoms on an atom, radical, group or moiety with which the term is used in combination is not replaced by other atoms or groups of atoms other than hydrogen atoms (i.e., substituent), so that the atom, radical, group or moiety retains its original structure. The term "substituted" means that one, two or more hydrogen atoms in a group, preferably up to 5 hydrogen atoms, more preferably 1-3 hydrogen atoms are each independently substituted by the corresponding number of substituents. When substituted with more than one or more substituents, these substituents are independent of each other, that is, the one or more substituents can be the same with each other, but it is not excluded the possibility that they may be the same. Unless specifically indicated, a substituent group may be substituted at any substitutable position on the substituted group. When more than one position in a given formula can be substituted by one, two or more substituents, then these substituents can be independently substituted at those positions. It goes without saying that the substituents are only in their possible chemical positions, and those skilled in the art can determine possible or impossible substitutions without undue labor, either experimentally or theoretically. The phrase "independently of each other" should be understood to mean that the described subjects are independent of each other and may be independently selected from the same or different options. For example, "be, independently of each other, selected from" can either mean that in different groups, the specific options expressed by the same symbol do not affect each other; it can also mean that in the same group, the specific options expressed by the same symbol do not affect each other.

Herein, linkers are sometimes described which are in the middle of a compound structure and are attached to the rest of the compound through at least two sites of attachment. A Markush variable listed for a linker should be understood to mean the divalent group of that variable, i.e., a "-ene" group. For example, if a linking group is defined in a compound structure and an "alkyl" or "aryl" group is listed for a Markush group definition for that linking group, it should be understood that the "alkyl" or "aryl" represents an alkylene or arylene group, respectively.

Herein, the notation "Cₓ-C_{y}", when used in combination with a group, represents a rang with an upper and a lower limit of the number of carbon atoms contained in the group. For example, "C₁-C₁₂" alkyl refers to an alkyl group containing at least one carbon atom and up to twelve carbon atoms. It is understood by those of skill in the art that such number does not comprise the number of carbon atoms contained in the substituents to which these groups are attached when these groups are further substituted. The expression "X-Y membered", when used in combination with a cyclic group, represents a rang with an upper and lower limit of the number of ring atoms contained in the cyclic group. For example, a "3-20 membered" heterocyclic group refers to a heterocyclic group containing at least three ring atoms up to a maximum of twenty ring atoms. It is understood by those skilled in the art that such number does not comprise the number of atoms contained in the substituents to which these groups are attached when these groups are further substituted.

Herein, the term "halogen" represents fluorine, chlorine, bromine, and/or iodine. Accordingly, the term "halo-" refers to fluoro-, chloro-, bromo- and/or iodo-. Herein, when an atom, a radical, a group or a moiety is halogenated, the atom at the halogenated position(s) can be mono-, di- or multiple-substituted with halogen atoms. Herein, when an atom, a radical, a group or a moiety is halogenated, the atom at the halogenated position may be mono-substituted, di-substituted, or multiple-substituted with halogen atom(s) or even all substituted.

The term "alkyl" represents a linear or branched, saturated, monovalent aliphatic hydrocarbyl group. Non-limiting examples of alkyl comprise methyl, ethyl, n-propyl, n-propyl, n-butyl, iso-butyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and branched isomers thereof etc.

The term "alkenyl" refers to a linear or branched, unsaturated, monovalent aliphatic hydrocarbyl group containing one, two or more double bonds. It should be understood that in the case that the alkenyl comprises more than one double bond, the double bonds can be separated from one another or conjugated. Non-limiting examples of alkyl comprise vinyl, allyl, (*E*)-2-methylvinyl, (*Z*)-2-methylvinyl, (*E*)-but-2-enyl, (*Z*)-but-2-enyl, (*E*)-but-1-enyl, (*Z*)-but-1-enyl, pent-4-enyl, (*E*)-pent-3-enyl, (*Z*)-pent-3-enyl, (*E*)-pent-2-enyl, (*Z*)-pent-2-enyl, (*E*)-pent-1-enyl, (*Z*)-pent-1-enyl, hex-5-enyl, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethylprop-1-enyl, 1-propylvinyl or 1-isopropylvinyl.

The term "alkylene/alkylidene" refers to a divalent group obtained by removing an additional hydrogen atom from the "alkyl" group. Similarly, " alkenylene/alkenylidene" and " alkynylene/alkynylidene" refer to divalent groups obtained by removing one additional hydrogen atom from "alkenyl" and "alkynyl", respectively.

The term "alkyloxy/alkoxy" refers to -O-alkyl, wherein alkyl is defined as described herein. Non-limiting examples of alkoxy groups comprise, for example, methoxy, ethoxy, propoxy, butoxy, cyclopropoxy, cyclobutoxy, cyclopentyloxy, cyclohexyloxy, etc. The alkoxy group may be unsubstituted or optionally substituted.

The term "heterocyclic/heterocyclyl/heterocycle" refers to a saturated or partially unsaturated monocyclic or polycyclic hydrocarbyl group containing 3 to 20 atoms, in which one or more ring atom is a heteroatom or moiety selected from N, O, NH, S, S(O) or S(O)₂, but excluding -O-O-, -O-S- or -S-S- and the remaining ring atom(s) are carbon atom(s). Preferably it contains 3 to 12 ring atoms, of which 1-4 are heteroatoms (e.g., 1, 2, 3 and 4); more preferably 3 to 6 ring atoms (e.g., 3, 4, 5, 6). A heterocyclyl group can be attached to the remaining mioety of the molecule through any of the carbon atoms or nitrogen atom(s) (if present) or oxygen or sulfur atom(s) (especially in the case of an onium salt is formed). The heterocyclyl group may include fused or bridged rings and/or spiro rings. Non-limiting examples of monocyclic heterocyclyl groups include azetidinyl, oxetanyl, pyrrolidinyl, imidazolidinyl, tetrahydrofuranyl, tetrahydrothienyl, dihydroimidazolyl, dihydrofuranyl, dihydropyrazolyl, dihydropyrrolyl, dioxolyl, tetrahydropyranyl, pyrrolinyl, piperidinyl, piperazinyl, morpholinyl, thiomorpholinyl, dithianyl, trithianyl, homopiperazinyl, a diazepanyl, etc., preferably a piperidinyl group and a pyrrolidinyl group. Polycyclic heterocyclyls include spiro, fused and bridged heterocyclic groups, and may also be benzo-fused heterocyclic groups such as dihydroisoquinolinyl. The heterocyclyl may be bicyclic, non-limiting examples of which include hexahydrocyclopenta[*c*]pyrrol-2(1*H*)-yl or hexahydropyrrolo[1,2-*a*]pyrazin-2(1*H*)-yl ring. The heterocyclyl may be partially unsaturated, i.e., it may comprise one or more double bonds, non-limiting examples of which include dihydrofuranyl, dihydropyranyl, 2,5-dihydro-1*H*-pyrrolyl, 4*H*-[1,3,4]thiadiazinyl, 4,5-dihydrooxazolyl or *4H-[1,4]thiazinyl.*

The term "aryl/aryl ring" refers to a 6 to 14-membered all-carbon monocyclic or fused polycyclic (i.e., rings sharing adjacent carbon atom pairs) group having a conjugated electron system, preferably a 6 to 10-membered ring, such as phenyl and naphthyl.

Herein, the term "heteroaryl/heteroaryl ring" refers to a heteroaromatic ring system containing 1 to 4 heteroatoms and 5-20 ring atoms, wherein the heteroatoms are selected from oxygen, sulfur and nitrogen. The heteroaryl is preferably 5 to 10 membered (for example, 5, 6, 7, 8, 9 or 10 membered), more preferably 5 membered or 6 membered. Non-limiting examples of the heteroaryl include, but not limited to, thienyl, furyl, pyrrolyl, oxazolyl, thiazolyl, imidazolyl, pyrazolyl, isoxazolyl, isothiazolyl, oxadiazolyl, triazolyl, thiadiazolyl, thia-4H-pyrazolyl and the like and benzo derivatives thereof, such as benzofuranyl, benzothienyl, benzoxazolyl, benzoisoxazolyl, benzimidazolyl, benzotriazolyl, indazolyl, indolyl, isoindolyl, and the like; or pyridinyl, pyridazinyl, pyrimidinyl, pyrazinyl, triazinyl and the like and benzo derivatives thereof, such as quinolyl, quinazolinyl, isoquinolyl, and the like; or azocinyl, indolizinyl, purinyl and the like and benzo derivatives thereof; or cinnolinyl, phthalazinyl, quinazolinyl, quinoxalinyl, naphthyridinyl, pteridinyl, carbazolyl, acridinyl, phenazinyl, phenothiazinyl, phenoxazinyl, and the like.

Unless otherwise stated, the heterocyclyl, heteroaryl or heteroarylene includes all possible isomeric forms thereof, e.g., positional isomers thereof. Accordingly, for some illustrative non-limiting examples, this may include the form of substitution or bonding to other groups at one, two or more of its 1-, 2-, 3-, 4-, 5-, 6-, 7-, 8-, 9-, 10-, 11-, 12-positions, etc. (if present), including pyridin-2-yl, pyridinylene-2-yl, pyridin-3-yl, pyridinylene-3-yl, pyridin-4-yl, and pyridinylene-4-yl; thienyl or thienylene including thien-2-yl, thien-2-ylene, thien-3-yl, and thien-3-ylene; pyrazol-1-yl, pyrazol-3-yl, pyrazol-4-yl, pyrazol-5-yl.

Aryl/aromatic ring/heteroaryl/heteroaromatic ring may be optionally substituted or unsubstituted; when being substituted, the substituents may preferably be one, two or more groups independently selected from the group consisting of: alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboxylate ester group.

Unless otherwise indicated, the definition of a term herein apply equally to expressions comprising the term, e.g., the definition of C₁-C₆ alkyl also applies to C₁-C₆ alkoxy (alkyloxy), -N(C₁₋₆ alkyl)₂, -NHC₁₋₆ alkyl, -SO-C₁₋₆ alkyl, or -S(O)₂-C₁₋₆ alkyl, etc.

Herein, "physiologically/pharmacologically acceptable salt" means a salt of a compound provided in the disclosure that is safe and effective when administered in mammals and has the desired biological activity.

Physiologically/pharmacologically acceptable salts comprise acid addition salts of the compounds disclosed herein that having a nitrogen atom in the chain or ring with sufficient basicity. In addition, the basic nitrogen-containing groups may be quaternized with the following agents: lower alkyl halides such as methyl, ethyl, propyl and butyl chlorides, bromides and iodides; dialkyl sulfates such as dimethyl sulfate, diethyl sulfate, dibutyl sulfate, and dipentyl sulfate; long chain halides such as decyl, lauryl, myristyl and stearyl chlorides, bromides and iodides; and aralkyl halides such as benzyl and phenethyl bromides. As an example, physiologically/pharmaceutically acceptable salts comprise hydrochloride, sulfate, nitrate, bisulfate, hydrobromide, acetate, oxalate, citrate, mesylate, formate, meglumine, and the like.

Since the compounds disclosed herein may have a plurality of salt-forming sites, the "physiologically/pharmaceutically acceptable salt" includes not only a salt formed at 1 salt-forming site of the compounds disclosed herein but also salts formed at 2, 3 or all of the salt-forming sites thereof. For this purpose, as for physiologically/pharmaceutically acceptable salt, the molar ratio of the compound of formula (X) or (XI), formula (I) or (II), formula (I-I) or (II-I), formula (IA) or (IIA), formula (IB) or (IIB), formula (IC) or (IIC) to a radical ion (anion) of an acid or a cation of a base required for salt formation may vary within a wide range, and may be, for example, 4:1 to 1:4, such as 3:1, 2:1, 1:1, 1:2 or 1:3.

Herein, the term "Nitrogen oxide" disclosed herein refers to an N-oxide formed by oxidizing one or more nitrogen atoms when the compound contains several nitrogen-containing functional groups. Specific examples of N-oxides are N-oxides of tertiary amines or N-oxides of a nitrogen atom of a nitrogen-containing heterocycle. The corresponding nitrogen-containing compound can be treated with an oxidizing agent such as hydrogen peroxide or peracid (e.g., peroxycarboxylic acid) to form N-oxides (see Advanced Organic Chemistry, Wiley Interscience, 4th edition, Jerry March). In particular, N-oxides may be prepared by the method of L. W. Deady (Syn. Comm. 1977, 7, 509-514) in which a nitrogen-containing compound is reacted with m-chloroperoxybenzoic acid (MCPBA), for example, in an inert solvent such as dichloromethane.

Herein, the term "Ester" refers to an ester that is hydrolyzable in vivo and formed by a compound containing hydroxyl or carboxyl. Such esters are, for example, physiologically/pharmaceutically acceptable esters that are hydrolyzed in human or animal to produce parent alcohols or acids. The compound of formula (X) or (XI), formula (I) or (II), formula (I-I) or (II-I), formula (IA) or (IIA), formula (IB) or (IIB), formula (IC) or (IIC) disclosed herein contains carboxyl, which can form an ester that is hydrolyzed in vivo with appropriate groups including, but not limited to, alkyl, arylalkyl and the like.

In terms of the location and nature of different substituents, the compounds disclosed herein may further contain one or more asymmetric centers. The asymmetric carbon atom may has (R) or (S) configuration. A racemic mixture is obtained with one asymmetric center; and a mixture of diastereomers is obtained with multiple asymmetric centers. In some cases, asymmetry is also produced due to hindrance of rotation around a particular bond, for example, when the particular bond is connected with two substituted aromatic rings in the particular compound. In addition, the substituents may exist in a cis- or trans-isomer form.

The compounds disclosed herein also include all possible stereoisomers thereof, which are single stereoisomers or any mixtures of stereoisomers (for example R-isomers or S-isomers, or E-isomers or Z-isomer) in any ratio. The separation of single stereoisomers (e.g., single enantiomers or single diastereomers) of the compounds disclosed herein can be achieved by a method according to any suitable prior art (for example, chromatography, particularly, chiral chromatography).

The term "optical isomer" means that when a compound has one or more chiral centers, each of which can be in the R or S configuration, the resulting various isomers are optical isomers. Optical isomers include all diastereoisomers, enantiomers, mesoisomers, racemates or mixtures thereof. For example, optical isomers can be separated by a chiral column or by chiral synthesis.

The term "tautomer" refers to functional isomers resulting from the rapid movement of an atom in a molecule between two positions. The compounds disclosed herein may exhibit the tautomerism. Tautomeric compounds may exist in two or more interconvertible forms. Prototropic tautomers result from the migration of a covalently bonded hydrogen atom between two atoms. Tautomers generally exist in an equilibrium form. Trying to separate a single tautomer usually lead to a mixture, the physicochemical properties of which are consistent with the mixture of the compound. The position of the equilibrium depends on the chemical properties of the molecule. For example, in many aliphatic aldehydes and ketones such as acetaldehyde, the keto form predominates; whereas in phenol, the enol form predominates. The present disclosure comprises all tautomeric forms of the compound.

The term "geometric isomer" refers to the presence of cis-isomer, trans-isomer, E-isomer and Z-isomer when a double bond is present in the compound. Geometric isomers include cis-isomers, trans-isomers, E-isomers, Z-isomers or mixtures thereof.

In the present disclosure, the compounds disclosed herein also include isotopically-labeled compounds. The isotopically-labeled compounds are the same as those shown in formula (X) or (XI), formula (I) or (II), formula (I-I) or (II-I), formula (IA) or (IIA), formula (IB) or (IIB), formula (IC) or (IIC), except that one or more are replaced by atoms having an atomic mass or mass number different from the usually naturally-occurring atomic mass or mass number. Examples of isotopes that can be incorporated into the compounds of the present disclosure include isotopes of H, C, N, O, S, F and Cl, such as ²H , ³H, ¹³C, ¹¹C, ¹⁴C, ¹⁵N, ¹⁸O, ¹⁷O, ³²P, ³⁵S, ¹⁸F and ³⁶Cl. The compounds of the present disclosure containing the above-mentioned isotopes and/or other isotopes of other atoms, prodrugs thereof, or physiologically/pharmaceutically acceptable salts of the compounds or prodrugs are within the scope of the present disclosure. The isotopically labelled compounds of the present disclosure can generally be prepared according to the methods described herein by replacing non-isotopically labelled reagents with isotopically labelled reagents. Certain isotopically-labeled compounds of the present disclosure, such as compounds incorporating radioisotopes (such as ³H and ¹⁴C), can be used for drug and/or substrate tissue distribution determination. Tritium (i.e., ³H) and carbon 14 (i.e., ¹⁴C) isotopes are particularly preferred due to their ease of preparation and detectability. Furthermore, the replacement with heavier isotopes (such as deuterium (i.e., ²H)) can provide certain therapeutic advantages derived from higher metabolic stability (for example, increased in vivo half-life or reduced dosage requirements), and therefore are preferred in certain cases. The compounds of the present disclosure as claimed in the claims can be specifically defined to be substituted with deuterium or tritium. In addition, the hydrogen existing in the substituent where deuterium or tritium is not listed separately does not mean that deuterium or tritium is excluded, but deuterium or tritium can also be included.

The term "prodrug" used herein represents a compound that is converted *in vivo* to a compound of formula (X) or (XI), formula (I) or (II), formula (I-I) or (II-I), formula (IA) or (IIA), formula (IB) or (IIB), formula (IC) or (IIC) or specific compounds. Such conversion is affected by hydrolysis of the prodrug in the blood or by enzymatic conversion of the prodrug into the parent structure in the blood or tissue. The prodrugs disclosed herein can be esters, and in the prior art, the esters that can be used as prodrugs include phenyl esters, aliphatic esters, acyloxymethyl esters, carbonates, carbamates and amino acid esters. For example, a compound disclosed herein containing hydroxyl/carboxyl can be acylated to give a compound as a prodrug. Other prodrugs include phosphate esters, and those phosphate esters are obtained by phosphorylating via the hydroxyl on the parent structure.

The term "metabolite" used herein refers to a product obtained by the metabolism of a particular compound or salt thereof *in vivo.* Metabolites of a compound can be identified by techniques well known in the art, and their activities can be characterized by assays as described herein. Such products may be obtained by the oxidation, reduction, hydrolysis, amidation, deamidation, esterification, defatting, enzymatic cleavage, and the like of the administered compound. Accordingly, it is disclosed herein metabolites of the compound, including metabolites produced by bringing the compound disclosed herein into contact with a mammal for a sufficient period of time.

Unless otherwise indicated, abbreviations for any of the protecting groups, amino acids and other compounds used herein are provided based on their commonly used and accepted abbreviations, or by referring to IUPAC-IUB Commission on Biochemical Nomenclature.

The term "solvate" disclosed herein refers to an association compound of one or more solvent molecules with the compound disclosed herein. Solvents that form the solvate include, but are not limited to, water, isopropanol, ethanol, methanol, dimethyl sulfoxide, ethyl acetate, acetic acid and aminoethanol. The term "hydrate" refers to an association compound in which the solvent molecules are water molecules.

The term "pharmaceutical composition" disclosed herein refers to a mixture comprising one or more of the compounds disclosed herein or physiologically/pharmaceutically acceptable salts or prodrugs thereof and other chemical components such as physiologically/pharmaceutically acceptable carriers and excipients. The purpose of a pharmaceutical composition is to facilitate administration of a compound to an organism, which is conducive to the absorption of the active ingredient, thus displaying biological activity.

The term "physiologically/pharmaceutically acceptable" disclosed herein refers to molecular entities and compositions that are physiologically tolerable and generally do not produce an allergic or similar untoward reaction, such as gastrointestinal distress and dizziness, when administered to a human. The term "carrier" refers to a diluent, adjuvant, excipient, or matrix with which the compound is administered. Such pharmaceutical carriers can be sterile liquid, such as water and oil, including those derived from petroleum, animals, plants or synthesis, such as peanut oil, soybean oil, mineral oil and sesame oil. Water or aqueous saline solutions and aqueous dextrose and glycerol solutions are preferably used as carriers, particularly injectable solutions.

The term "treat", "treating" or "treatment" used herein, in some embofiments, refers to ameliorating a disease or disorder (i.e., slowing or arresting or reducing the progression of the disease or at least one clinical symptom thereof). In other embodiments, "treat", "treating" or "treatment" refers to mitigating or improving at least one physical parameter, including physical parameters that may not be perceived by a patient. In other embodiments, "treat", "treating" or "treatment" refers to modulating a disease or disorder, either physically (e.g., stabilizing a perceptible symptom) or physiologically (e.g., stabilizing a physical parameter), or both. In other embodiments, "treat", "treating" or "treatment" refers to preventing or delaying the onset, occurrence, or deterioration of a disease or disorder.

The term "effective amount" or "therapeutically effective amount" refers to an amount of the compounds disclosed herein sufficient to effect the intended use, including but not limited to the treatment of a disease as defined below. The therapeutically effective amount may vary depending on the following factors: the intended use (in vitro or in vivo), or the subject and diseases or disorders being treated, such as weight and age of the subject, severity of the diseases or disorders, and mode of administration, and it can be readily determined by one of ordinary skill in the art. The specific dosage will vary depending on the following factors: the selected particular compound, the dosage regimen to be followed, whether to administer in combination with other compounds, the schedule of administration, the tissue to be administered and the physical delivery system carried.

In some preferred embodiments in the disclosure, pharmaceutical excipients may be those widely used in the field of drug production. The excipients are primarily used to provide a safe, stable and functional pharmaceutical composition and may also provide a method for dissolving the active ingredients at a desired rate or for promoting effective absorption of the active ingredients after administration of the composition to a subject. The pharmaceutically acceptable excipients may be inert fillers or provide a function such as stabilizing the overall pH of the composition or preventing degradation of the active ingredients of the composition. The pharmaceutically acceptable excipients may include one or more of the following excipients: binders, suspending agents, emulsifiers, diluents, fillers, granulating agents, gluing agents, disintegrating agents, lubricants, anti-adherents, glidants, wetting agents, gelling agents, absorption retardants, dissolution inhibitors, reinforcing agents, adsorbents, buffering agents, chelating agents, preservatives, colorants, flavoring agents and sweeteners.

Substances which may serve as physiologically/pharmaceutically acceptable excipients include, but are not limited to, ion exchangers; aluminum; aluminum stearate; lecithin; serum proteins such as human serum protein; buffer substances such as phosphate; glycine; sorbic acid; potassium sorbate; partial glyceride mixture of saturated vegetable fatty acid; water; salts or electrolytes such as protamine sulfate, disodium hydrogen phosphate, potassium hydrogen phosphate, sodium chloride, zinc salt, colloidal silica, magnesium trisilicate; polyvinylpyrrolidone, polyacrylate; waxes; polyethylene-polyoxypropylene-blocking polymer; wool fat; sugars such as lactose, glucose and sucrose; starches such as corn starch and potato starch; cellulose and its derivatives such as sodium carboxymethyl cellulose, ethyl cellulose and cellulose acetate; gum powder; malt; gelatin; talc powder; adjuvants such as cocoa butter and suppository waxes; oils such as peanut oil, cottonseed oil, safflower oil, sesame oil, olive oil, corn oil and soybean oil; glycols such as propylene glycol and polyethylene glycol; esters such as ethyl oleate and ethyl laurate; agar; buffers such as magnesium hydroxide and aluminum hydroxide; alginic acid; pyrogen-free water; isotonic salts; Ringer's solution; ethanol, phosphate buffered solution, and other non-toxic suitable lubricants such as sodium lauryl sulfate and magnesium stearate, coloring agents, releasing agents, coating agents, sweeteners, flavoring agents and perfumes, preservatives and antioxidants.

The pharmaceutical composition disclosed herein may be prepared in accordance with the disclosure using any method known to those skilled in the art, for example, conventional mixing, dissolving, granulating, emulsifying, levigating, encapsulating, embedding or lyophilizing processes.

The dosage form of the drug disclosed herein can be selected according to specific conditions. Pharmaceutical dosage forms often consist of drugs, excipients, and containers/closure systems. One or more excipients (also known as inactive ingredients) may be added to the compounds disclosed herein to improve or facilitate the manufacture, stability, administration and safety of drugs, and may provide a means to obtain a desired drug release profile. Thus, the type of excipient added to a drug may depend on various factors, such as physical and chemical properties of the drug, route of administration and preparation steps. There are pharmaceutical excipients in the art, including those listed in various pharmacopoeias.

The pharmaceutical composition disclosed herein may include one or more physiologically acceptable inactive ingredients that facilitate processing of active molecules into preparations for pharmaceutical use.

Appropriate preparations will be determined according to the desired route of administration. The route of administration includes intravenous injection, transmucosal or nasal administration, oral administration and the like. For oral administration, the compound may be formulated into liquid or solid dosage forms and used as immediate release or controlled/sustained release preparations. Suitable dosage forms for oral ingestion by an subject include tablets, pills, dragees, hard and soft shell capsules, liquids, gels, syrups, ointments, suspensions and emulsions.

Oral solid dosage forms can be obtained using excipients including fillers, disintegrants, binders (dry and wet), dissolution retardants, lubricants, glidants, anti-adherents, cationic exchange resins, wetting agents, antioxidants, preservatives, colorants and flavoring agents. These excipients may be of synthetic or natural sources. Examples of such excipients include cellulose derivatives, citric acid, dicalcium phosphate, gelatin, magnesium carbonate, magnesium/sodium lauryl sulfate, mannitol, polyethylene glycol, polyvinylpyrrolidone, silicates, silica, sodium benzoate, sorbitol, starches, stearic acid or salts thereof, sugars (i.e., dextrose, sucrose, lactose, etc.), talc, tragacanth mucilage, vegetable oils (hydrogenated) and waxes. Ethanol and water may be used as adjuvants for granulation. In some cases, it is desirable to coat tablets with, for example, a taste-masking film, a gastric acid-resistant film or a sustained release film. Natural and synthetic polymers are often used to coat tablets in combination with colorants, sugars, and organic solvents or water to produce dragees. When capsules are superior to tablets, the pharmaceutical powders, suspensions or solutions may be delivered in the form of compatible hard or soft shell capsules.

The therapeutically effective dosage can first be estimated using various methods well known in the art. Initial dosage for animal studies can be based on established effective concentrations in cell culture assays. Dosage ranges suitable for humans can be determined, for example, using data obtained from animal studies and cell culture assays. In certain embodiments, the compounds disclosed herein may be prepared as medicaments for oral administration.

The correct preparation, route of administration, dosage and time interval between administrations can be determined based on methods known in the art while taking the specificity of the subject into account.

### Examples

The preparation method in the disclosure will be illustrated further in detail below with reference to specific examples. It should be understood that the following examples are only to illustrate and explain the invention in an exemplary way, and should not be construed as limiting the protection scope of the invention. All technical solutions achieved based on the content of the invention are comprised within the intended protection scope of the invention.

Unless otherwise specified, the experimental methods used in the following examples are conventional methods in the art; the reagents, raw materials, instruments, equipments, etc. used in the following examples may be obtained commercially.

### Abbreviation or acronym

DCM: dichloromethane
TEA: triethanolamine
Et₂O: diethyl ether
*n*-Bu₄NOH: tetra-n-butylammonium hydroxide
DMF: N,N-dimethylformamide
EA: ethyl acetate
PE: petroleum ether
THF: tetrahydrofuran
NMP: N-methylpyrrolidone
DIEA: N,N-diisopropylethylamine
ACN: acetonitrile
MeOH: methanol
Bu: butyl
TEAB: triethylamine-carbonate buffer
TBHP: *tert*-butyl hydroperoxide
RT: retention time
TLC: thin layer chromatography

### Preparation Example

### Preparation Example 1 Preparation of compound 001, compound 070 and compound 071

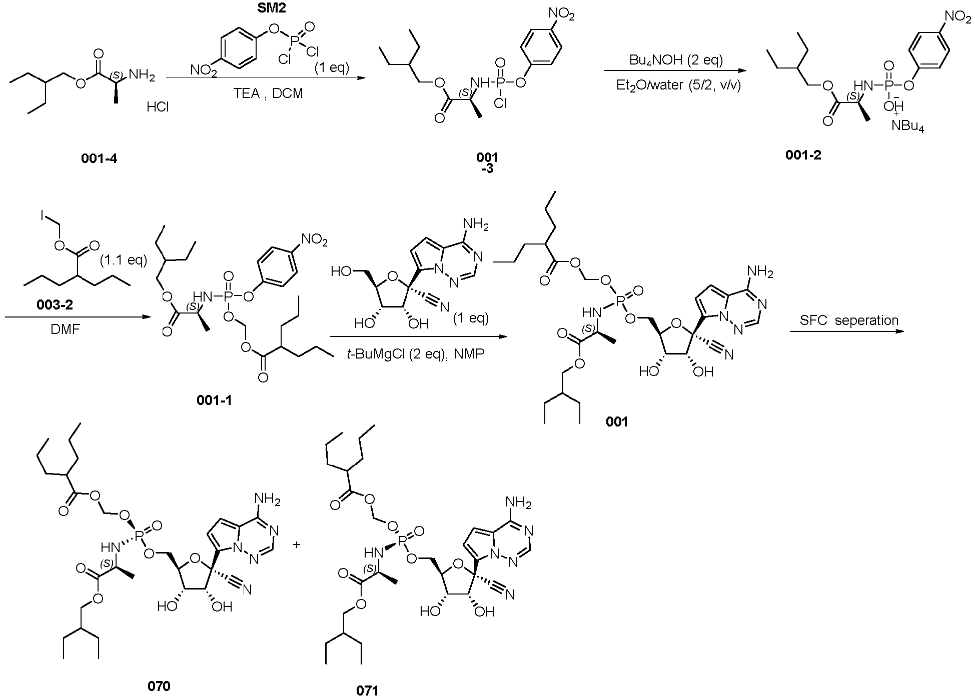

### Preparation of compound 001-3:

To a mixture of compound 001-4 (15 g, 71.527 mmol, 1 equiv.) and 4-nitrophenyl phosphorodichloridate (18.31 g, 71.527 mmol, 1 equiv.) in DCM (300 mL) was added TEA (14.48 g, 143.054 mmol, 2 equiv.) in DCM at -78 °C under Ar atmosphere. The resulting mixture was stirred for 1 h at -78 °C and then warmed to room temperature in 2 h. The reaction mixture was concentrated, the residue was diluted with Et₂O (150 mL) and stirred for 1 h at room temperature. The solid formed was removed by filtration, the filtrate was concentrated under reduced pressure to afford compound 001-3 (30 g) as crude product which was used through. Confirmed by LC-MS.

### Preparation of compound 001-2:

To a mixture of compound 001-3 (10 g, 25.460 mmol, 1 equiv.) in Et₂O (60 mL) was added *aq. n*-Bu₄NOH (15 mL, 30.552 mmol, 1.2 equiv, 40% wt%) dropwise at 0 °C under Ar atmosphere. The resulting mixture was stirred for 2 h at room temperature. The reaction mixture was concentrated under reduced pressure and the residue was purified by RP-flash chromatography, eluted with 0-20% MeCN in 10 mM *aq.* NH₄HCO₃ to afford compound 001-2 (4.7 g, 29.98% yield) as a yellow oil. Confirmed by LC-MS, H/P NMR.

### Preparation of compound 001-1:

To a solution of compound 001-2 (1 g, 1.621 mmol, 1 equiv.) in DMF (10 mL) was added a solution of compound 003-2 (506.73 mg, 1.783 mmol, 1.1 equiv.) in DMF (1 mL) at 0 °C under Ar atmosphere, the resulting mixture was stirred for 1 h at 20 °C. The reaction was quenched with *aq.* HCl (1% wt%, 20 mL), extracted with EA (200 mL), the organic layer was washed with water (30 mL) and brine *(sat.,* 20 mL), dried over Na2SO4, concentrated under reduced pressure. The residue was purified by silica gel column chromatography, eluted with 23% EA in PE to afford compound 001-1 (500 mg, 58.14% yield) as a white solid. Confirmed by LC-MS, H/P NMR.

### Preparation of compound 001, compound 070 and compound 071:

To a solution of compound 3 (2.91 g, 9.990 mmol, 1 equiv.) in NMP (50 mL) was added tert-butyl(chloro)magnesium (1 M in THF, 20 mL, 19.980 mmol, 2 equiv.) at 0 °C under argon atmosphere, the resulting mixture was stirred for 30 min. To the mixture was added a solution of compound 001-1 (5.3 g, 9.990 mmol, 1 equiv.) in NMP (10 mL), the resulting mixture was stirred for 2 h at 20 °C. The reaction was quenched with *aq.* NH₄Cl *(sat.,* 50 mL), extracted with EA (500 mL), the organic layer was washed with brine *(sat.,* 3 x 50 mL), dried over anhydrous Na₂SO₄. After filtration, the filtrate was concentrated under reduced pressure to afford 001 (5 g) as a racemic mixture which was separated by prep-chiral-SFC with the following condition: Column: CHIRALPAK IG, 5*25 cm, 10 µm; Mobile Phase A: CO₂, Mobile Phase B: MEOH (0.1% 2M NH₃-MEOH); Flow rate: 200 mL/min; Gradient: isocratic 40% B; Column Temperature (°C): 35; Back Pressure (bar): 100; Wave Length: 220 nm; RT1: 7.66 min to afford compound 070 (2.61 g) as a white solid. And RT2: 10.22 min to afford compound 071 (890 mg) as a white solid. Confirmed by LCMS, H NMR, P NMR.

Compound 070: ¹H NMR: (400 MHz, CD₃OD) δ 7.91 (s, 1H), 6.95-6.89 (m, 2H), 5.57-5.53 (m, 2H), 4.88-4.83 (m, 1H), 4.37-4.34 (m, 2H), 4.25-4.20 (m, 2H), 4.09-1.08(m, 1H), 4.07-4.05 (m, 1H), 4.05-3.99 (m, 1H), 2.40-2.38 (m, 1H), 1.61-1.56 (m, 5H), 1.58-1.29 (m, 12H), 0.91-0.85 (m, 12H).

Compound 071: ¹H NMR: (400 MHz, CD₃OD) δ 7.88 (s, 1H), 6.94-6.88 (m, 2H), 5.64-5.55 (m, 2H), 4.35-4.21 (m, 2H), 4.07-4.03 (m, 2H), 3.96-3.94 (m, 1H), 3.93-3.92 (m, 2H), 2.42-2.40 (m, 1H), 1.60-1.59 (m, 2H), 1.58-1.57 (m, 3H), 1.36-1.27 (m, 11H), 0.90-0.85 (m, 12H).

According to the invention, additional exemplary compounds were synthesized with reference to the methods in Preparation Example 1 above and are shown in Table 1 below:

**Table 1**

| No. | Structural Formula | Structural Characterization |
|---|---|---|
| 034 | 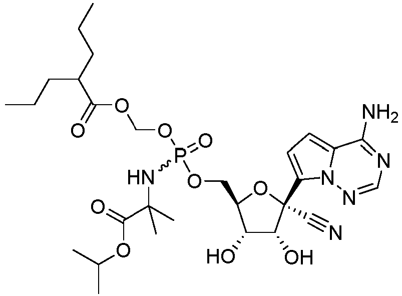 | ¹H NMR (400 MHz, DMSO-d6) δ 7.99-7.84 (m, 3H), 6.90 (d, J = 4.5 Hz, 1H), 6.84 (t, J = 4.5 Hz, 1H), 6.36-6.31 (m, J = 5.6 Hz, 1H), 5.60-5.55 (m, J = 9.6, 2.4 Hz, 1H), 5.50 (d, J = 12.3 Hz, 2H), 5.41-5.34 (m, 1H), 4.89-4.82 (m, 1H), 4.69-4.64 (m, 1H), 4.24-4.12 (m, 2H), 4.07-3.98 (m, J = 11.4, 5.8 Hz, 1H), 3.96-3.89 (m, 1H), 2.34-2.31 (m, J = 2.0 Hz, 1H), 1.54-1.44 (m, 2H), 1.39-1.30 (m, 7H), 1.24-1.15 (m, 9H), 1.07-1.01 (m, 2H), 0.85-0.77 (m, 6H). |
| 074 | 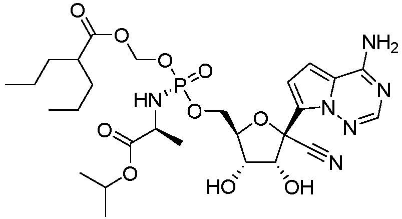 | ¹H NMR (400 MHz, DMSO-d6) |
| | | δ 8.07-7.81 (m, 3H), 6.88 (d, J = 4.5 Hz, 1H), 6.80 (d, J = 4.5 Hz, 1H), 6.37-6.19 (m, 1H), 5.74 (dd, J = 12.4, 10.0 Hz, 1H), 5.53-5.43 (m, 2H), 5.39-5.23 (m, 1H), 4.88-4.76 (m, 1H), 4.67-4.58 (m, J = 4.2 Hz, 1H), 4.21-4.06 (m, 2H), 4.00-3.91 (m, 1H), 3.90-3.84 (m, 1H), 3.67-3.58 (m,1H), 2.36-2.26 (m, 1H), 1.54-1.40 (m, 2H), 1.40-1.30 (m, 2H), 1.25-1.15 (m, 7H), 1.14-1.09 (m, 6H), 0.83-0.75 (m, 6H). |
| 075 | 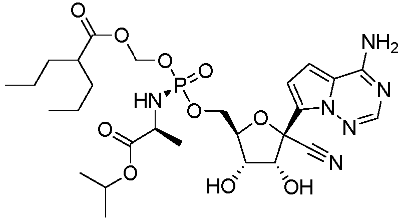 | ¹H NMR (400 MHz, DMSO-d6) |
| | | δ 8.02-7.75 (m, 3H), 6.87 (d, J = 4.5 Hz, 1H), 6.79 (d, J = 4.5 Hz, 1H), 6.28 (d, J = 6.1 Hz, 1H), 5.75 (dd, J = 12.7, 9.9 Hz, 1H), 5.49-5.39 (m, 2H), 5.35 (d, J = 5.7 Hz, 1H), 4.90-4.79 (m, 1H), 4.63 (t, J = 5.5 Hz, 1H), 4.24-4.09 (m, 2H), 4.06-3.86 (m, 2H), 3.60 (ddd, 1H), 2.34-2.25 (m, 1H),1.54-1.40 (m, 2H), 1.38-1.29 (m, 2H), 1.21-1.12 (m, 13H), 0.82-0.74 (m, 6H). |
| 122 | 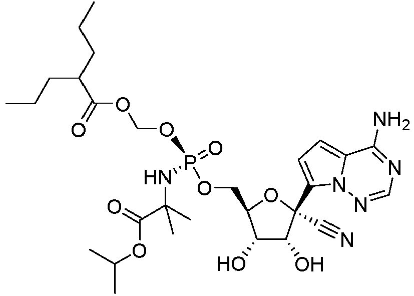 | ¹H NMR: (300 MHz, CD₃OD) |
| | | δ 7.87 (s, 1H), 6.95 (d, J = 4.8 Hz, 1H), 6.89 (d, J = 4.5 Hz, 1H), 5.63-5.53 (m, 2H), 4.97-4.86 (m, 2H), 4.36-4.17 (m, 4H), 2.41-2.39 (m, 1H), 1.62-1.55 (m, 10H), 1.46-1.21 (m, 10H), 0.90-0.85 (m, 6H). |
| 123 | 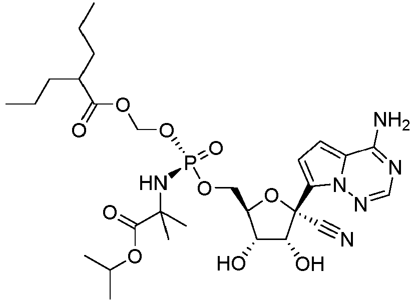 | ¹H NMR: (400 MHz, CD₃OD) |
| | | δ 7.87 (s, 1H), 6.95 (d, J = 4.5 Hz, 1H), 6.90 (d, J = 4.5 Hz, 1H), 5.62-5.56 (m, 2H), 4.98-4.86 (m, 2H), 4.40-4.34 (m, 2H), 4.26-4.18 (m, 2H), 2.42-2.37 (m, 1H), 1.64-1.51 (m, 10H), 1.47-1.22 (m, 1 0H), 0.89-0.84 (m, 6H). |
| 036 | 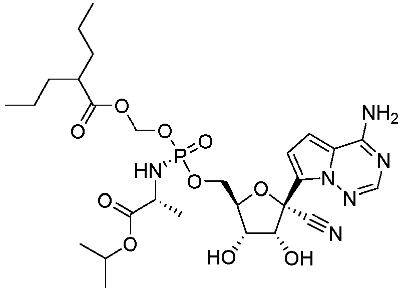 | ¹H NMR (400 MHz, CDCl₃) |
| | | δ 7.97(s, 1H), 6.90-6.89 (d, J = 15.2 Hz, 2H), 6.83-6.27 (m, 1H), 5.64-5.56 (m, 2H), 4.99-4.98 (m, 1H), 4.74-4.54 (m,2H), 4.50-4.39 (m, 1H), 4.22-4.15 (m, 2H), 3.82-3.81 (d, J = 7.3 Hz, 1H), 2.39(s, 1H), 1.58-1.50 (m, 2H), 1.48 -1.42 (m, 2H), 1.25-1.15 (m, 13H), 0.87-0.85 (m, 6H). |
| 095 | 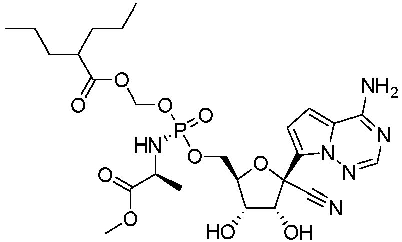 | ¹H NMR (400 MHz, DMSO-d6) |
| | | δ 7.98-7.84 (m, 3H), 6.90 (d, J = 4.5 Hz, 1H), 6.83 (d, J = 4.5 Hz, 1H), 6.32 (d, J = 6.1 Hz, 1H), 5.84 (dd, J = 12.6, 9.9 Hz, 1H), 5.50-5.44 (m, 2H), 5.38 (d, J = 5.7 Hz, 1H), 4.68-4.64 (m, 1H), 4.24-4.15 (m, 2H), 4.04-3.94 (m, 2H), 3.74-3.67 (m, 1H), 3.63 (s, 3H), 2.38-2.31 (m, 1H), 1.54-1.45 (m, 2H), 1.42-1.33 (m, 2H), 1.27-1.18 (m, 7H), 0.85-0.79 (m, 6H). |
| 096 | 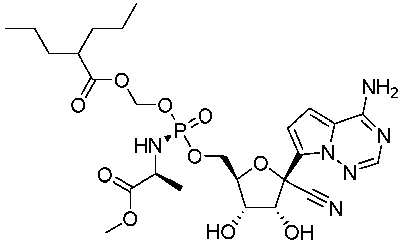 | ¹H NMR (400 MHz, DMSO-d6) δ 8.00-7.85 (m, 3H), 6.90 (d, J = 4.5 Hz, 1H), 6.83 (d, J = 4.5 Hz, 1H), 6.32 (d, J = 6.1 Hz, 1H), 5.83 (dd, J = 12.5, 9.9 Hz, 1H), 5.56-5.47 (m, 2H), 5.36 (d, J = 5.8 Hz, 1H), 4.68-4.64 (m, 1H), 4.23-4.18 (m, 1H), 4.16-4.11 (m, 1H), 4.01-3.90 (m, 2H), 3.77-3.70 (m, 1H), 3.61 (s, 3H), 2.40-2.32 (m, 1H), 1.54-1.45 (m, 2H), 1.43-1.34 (m, 2H), 1.28-1.19 (m, 7H), 0.86-0.80 (m, 6H). |
| 097 | 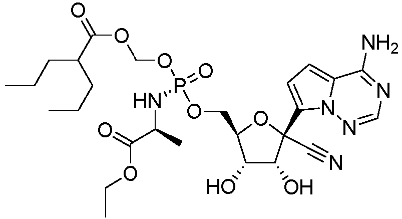 | ¹H NMR (400 MHz, DMSO-d6) δ 7.90 (m, 3H), 6.90 (d, J = 4.6 Hz, 1H), 6.83 (d, J = 4.5 Hz, 1H), 6.31 (d, J = 6.1 Hz, 1H), 5.81 (s, 1H), 5.49-5.46 (m, 2H), 5.38 (d, J = 5.7 Hz, 1H), 4.68-4.65 (m,1H), 4.24-4.14 (m, 3H), 4.07-4.01 (m, 3H), 3.70-3.66 (m, 1H), 2.34-2.32 (m, 1H), 1.51-1.46 (m, 2H), 1.40-1.35 (m, 2H), 1.25-1.16(m, 10H), 0.86-0.78 (m, 6H). |
| 098 | 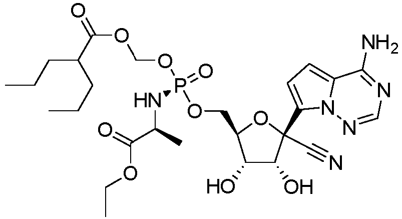 | ¹H NMR (400 MHz, DMSO-d6) δ 7.99-7.84 (m, 3H), 6.90 (d, J = 4.5 Hz, 1H), 6.83 (d, J = 4.5 Hz, 1H), 6.31 (d, J = 6.1 Hz, 1H), 5.83-5.77(m, 1H), 5.54-5.48 (m,2H), 5.35 (d, J = 5.8 Hz, 1H), 4.67-4.65 (m, 1H), 4.22-4.18 (m, 1H), 4.17-4.11 (m, 1H), 4.10-3.97 (m, 3H), 3.94-3.90 (m, 1H), 3.74-7.65 (m, 1H), 2.37-2.33 (m, 1H), 1.56-1.46 (m, 2H), 1.43-1.35 (m, 2H), 1.28-1.20 (m, 7H), 1.20-1.14 (m, 3H), 0.85-0.81 (m, 6H). |
| 120 | 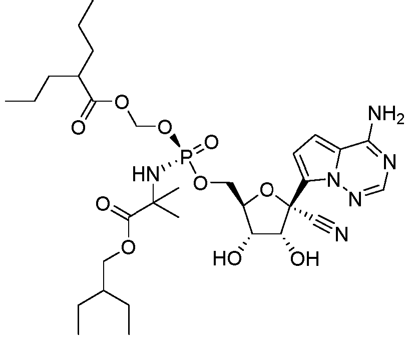 | ¹H NMR (400 MHz, CD₃OD) δ 7.87 (s, 1H), 6.95 (d, J = 4.8 Hz, 1H), 6.89 (d, J = 4.8 Hz, 1H), 5.64-5.52 (m, 2H), 4.88-4.87 (m, 1H), 4.36-4.34 (m, 2H), 4.23-4.19 (m, 2H), 4.04-4.02 (m, 2H), 2.42-2.40 (m, 1H), 1.65-1.23 (m, 19H), 0.90-0.85 (m, 12H). |
| 121 | 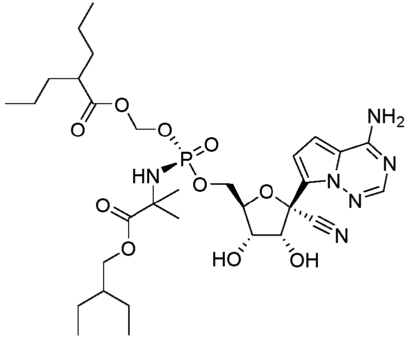 | ¹H NMR: (400 MHz, CD₃OD) δ 7.87 (s, 1H), 6.95 (d, J = 4.4 Hz, 1H), 6.89 (d, J = 4.4 Hz, 1H), 5.64-5.52 (m, 2H), 4.88-4.87 (m, 1H), 4.36-4.34 (m, 2H), 4.23-4.19 (m, 2H), 4.04-4.02 (m, 2H), 2.42-2.40 (m, 1H), 1.65-1.23 (m, 19H), 0.90-0.85 (m, 12H). |
| 137 | 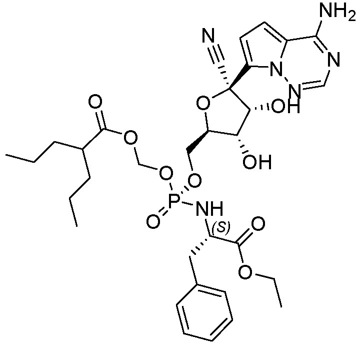 | ¹H NMR:(400 MHz, DMSO-d6) |
| | | δ 8.00-7.78 (m, 3H), 7.26-7.15 (m, 5H), 6.92 (d, J = 4.5 Hz, 1H), 6.82 (d, J = 4.5 Hz, 1H), 6.30 (d, J = 6.1 Hz, 1H), 5.87 (dd, J = 12.3, 10.5 Hz, 1H), 5.30 (m, 3H), 4.66-4.59 (m, 1H), 4.10 (td, J = 6.2, 3.2 Hz, 1H), 4.02 (qd, J = 7.1, 3.2 Hz, 2H), 3.84 (ddd, J = 17.4, 10.6, 6.3 Hz, 3H), 3.69 (dd, J = 11.5, 5.8 Hz, 1H), 3.01-2.94 (m, 1H), 2.81 (dd, J = 13.5, 8.8 Hz, 1H), 2.36-2.30 (m, 1H), 1.52-1.43 (m, 2H), 1.41-1.33 (m, 2H), 1.23 (s, 2H), 1.19 (d, J = 7.7 Hz, 2H), 1.08 (t, J = 7.1 Hz, 3H), 0.81 (td, J = 7.3, 2.3 Hz, 6H). |
| 138 | 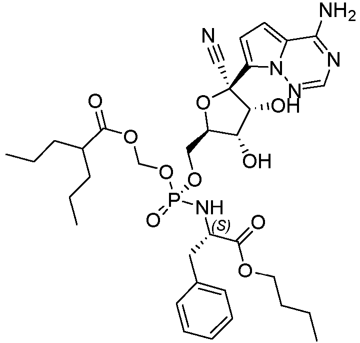 | ¹H NMR: (400 MHz, DMSO-d6) |
| | | δ 7.903 (m, 3H), 7.22-7.15 (m, 5H), 6.917 (d, J = 8.54 Hz, 1H), 6.818 (d, J = 8.54 Hz, 1H), 6.312 (s, 1H), 5.889 (t, J = 11.2 Hz, 1H), 5.32-5.27 (m, 3H), 4.619 (s, 1H), 4.101 (d, J = 6.83 Hz, 1H), 3.950-3.800 (m, 5H), 3.676 (dd, J = 12.13 Hz, 1H), 2.949 (d, J = 7.84 Hz, 1H), 2.790 (t, J = 15.85 Hz, 1H), 2.346-2.311 (d, J = 8.9 Hz, 1H), 1.438-1.399 (m, 6H), 1.217-1.142 (m, 6H), 0.848-0.786 (m, 9H). |
| 139 | 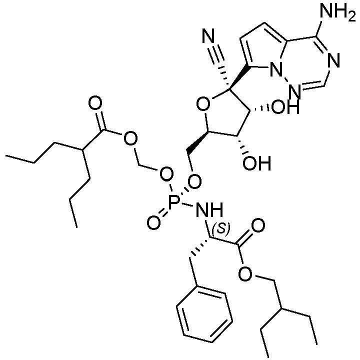 | ¹H NMR: (400 MHz, DMSO-d6) |
| | | δ 8.00-7.76 (m, J = 9.2 Hz, 3H), 7.27-7.16 (m, 5H), 6.92 (d, J = 4.5 Hz, 1H), 6.82 (d, J = 4.5 Hz, 1H), 6.31 (d, J = 5.8 Hz, 1H), 5.90 (dd, J = 12.4, 10.5 Hz, 1H), 5.34-5.27 (m, 3H), 4.62 (t, J = 5.1 Hz, 1H), 4.10 (td, J = 6.1, 3.0 Hz, 1H), 3.92-3.85 (m, 3H), 3.81 (dd, J = 10.4, 4.8 Hz, 1H), 3.70 (dt, J = 11.5, 5.8 Hz, 1H), 3.01-2.92 (m, 1H), 2.83 (dd, J = 13.5, 8.6 Hz, 1H), 2.33 (ddd, J = 8.6, 5.3, 3.2 Hz, 1H), 1.52-1.45 (m, 2H), 1.41-1.29 (m, 4H), 1.25-1.21 (m, 4H), 1.20-1.17 (m, 4H), 0.83-0.74 (m, 12H). |
| 028 | 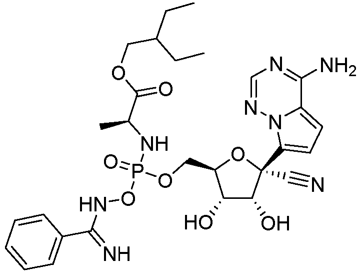 | ¹H NMR (400 MHz, DMSO-d6) δ 7.97-7.74 (m, 3H), 7.67-7.58 (m, 2H), 7.48-7.34 (m, 3H), 6.85-6.78 (m, 2H), 6.72-6.52 (m, 2H), 6.28 (t, J = 6.1 Hz, 1H), 5.47 (dd, J =21.3, 9.8 Hz, 1H), 5.33 (dd, J = 10.4, 5.9 Hz, 1H), 4.60 (dd, J = 11.0, 5.6 Hz, 1H), 4.25-4.15 (m, 2H), 4.10-3.98 (m, 1H), 3.99-3.81 (m, 3H), 3.81-3.64 (m, 1H),1.40-1.32 (m, 1H), 1.22-1.15 (m, 7H), 0.73 (t, J = 7.4 Hz, 6H). |
| 060 | 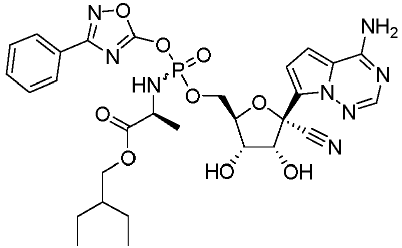 | ¹H NMR (400 MHz, DMSO-d6) δ 8.03-7.84 (m, 5H), 7.62-7.52 (m, 3H), 7.05-6.97 (m, 1H), 6.96-6.89 (m,2H), 5.66-5.59 (m, 1H), 5.31-5.23 (m, 1H), 4.67-4.62 (m, 1H), 3.97-3.69 (m, 5H), 3.59-3.50(m, 1H), 3.21-3.07 (m, 1H), 1.41-1.33 (m, 1H), 1.26-1.17 (m, 4H), 1.03-0.93 (m,3H), 0.77 (t, J = 7.4 Hz, 6H). |
| 065 | 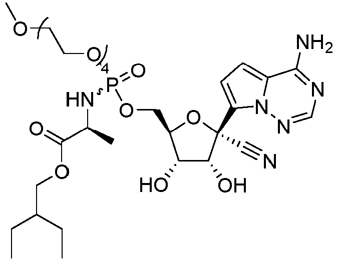 | ¹H NMR: (300 MHz, CD₃CN) |
| | | δ 7.97 (s, 1H), 6.93-6.91 (m, 1H), 6.82-6.80 (m, 1H), 6.41 (s, 2H), 4.79-4.76 (m, 2H), 4.37-4.36 (m, 1H), 4.21-4.17 (m, 3H), 4.06-3.90 (m, 7H), 3.60-3.54 (m, 11H), 3.50-3.46 (m, 2H), 1.37-1.35 (m, 1H), 1.33-1.25 (m, 7H), 0.92-0.86 (m, 6H). |
| 076 | 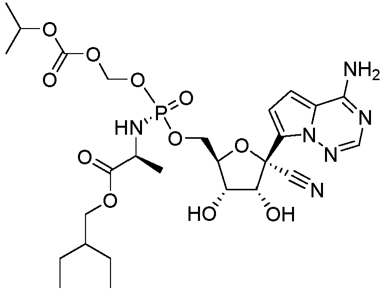 | ¹H NMR (400 MHz, DMSO-d6) |
| | | δ 8.06-7.78 (m, 3H), 6.90 (d, J = 4.5 Hz, 1H), 6.83 (dd, J = 4.5, 1.9 Hz, 1H), 6.31 (t, J = 6.4 Hz, 1H), 5.90-5.81 (m, 1H), 5.56-5.42 (m, 2H), 5.41-5.33 (m, 1H), 4.85-4.76 (m, 1H), 4.68-4.62 (m, 1H), 4.25-4.12 (m, 2H), 4.08-3.85 (m, 4H), 3.78-3.66 (m, 1H), 1.51-1.40 (m, 1H), 1.32-1.19 (m, 13H), 0.86-0.77 (m, 6H). |
| 068 | 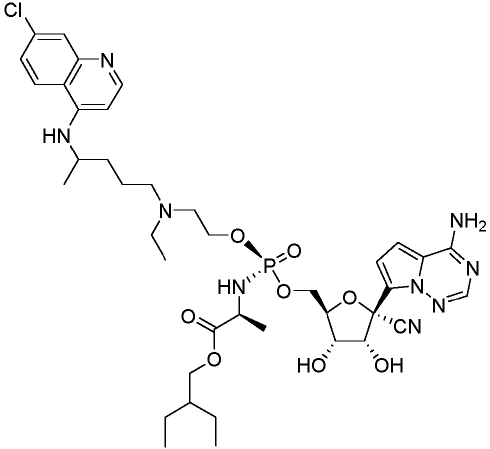 | ¹H NMR: (400 MHz, DMSO-d6) |
| | | δ 8.37-8.34 (m, 2H), 7.98-7.81 (m, 3H), 7.75 (d, J = 1.6 Hz, 1H), 7.42-7.39 (m, 1H), 6.90-6.83 (m, 3H), 6.48-6.46 (m, 1H), 6.30 (d, J = 6.0 Hz, 1H), 5.61-5.50 (m, 1H), 5.35 (d, J = 6.0 Hz, 1H), 4.65-4.62 (m, 1H), 4.23-4.19 (m, 1H), 4.15-4.08 (m, 1H), 3.95-3.85 (m, 6H), 3.82-3.64 (m, 2H), 2.55-2.50 (m, 2H), 2.45-2.38 (m, 4H), 1.46-1.45 (m, 1H), 1.43-1.41 (m, 4H), 1.27-1.19 (m, 10H), 0.90-0.86 (m, 3H), 0.82-0.78 (m, 6H). |
| 069 | 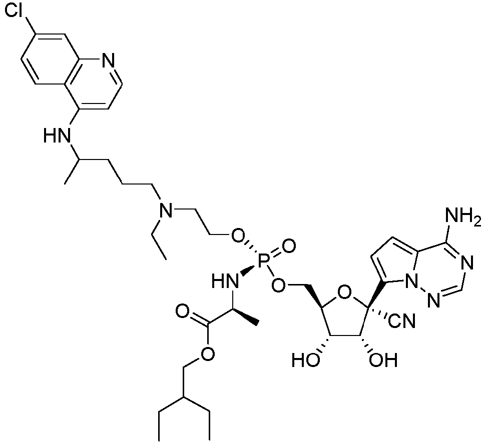 | ¹H NMR: (400 MHz, DMSO-d6) |
| | | δ 8.37-8.34 (m, 2H), 7.98-7.81 (m, 3H), 7.75 (d, J = 1.6 Hz, 1H), 7.42-7.39 (m, 1H), 6.90-6.83 (m, 3H), 6.48-6.46 (m, 1H), 6.30 (d, J = 6.0 Hz, 1H), 5.61-5.50 (m, 1H), 5.35 (d, J = 6.0 Hz, 1H), 4.65-4.62 (m, 1H), 4.23-4.19 (m, 1H), 4.15-4.08 (m, 1H), 3.95-3.85 (m, 6H), 3.82-3.64 (m, 2H), 2.55-2.50 (m, 2H), 2.45-2.38 (m, 4H), 1.46-1.45 (m, 1H), 1.43-1.41 (m, 4H), 1.27-1.19 (m, 10H), 0.90-0.86 (m, 3H), 0.82-0.78 (m, 6H). |
| 103 | 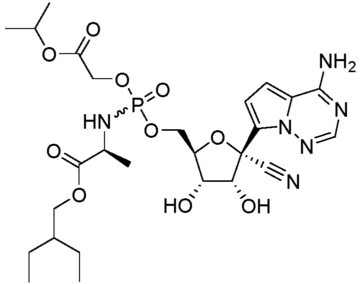 | ¹H NMR: (400 MHz, DMSO-d6) |
| | | δ 7.93 (s, 3H), 6.90 (d, J = 4.4 Hz, 1H), 6.84 (d, J = 4.4 Hz, 1H), 6.30 (d, J = 6.0 Hz, 1H), 5.70 (dd, J = 12.8, 10.0 Hz, 1H), 5.36 (t, J = 6.8 Hz, 1H), 4.95 (dt, J = 12.4, 6.4 Hz, 1H), 4.66 (dd, J = 11.2, 5.2 Hz, 1H), 4.46-4.36 (m, 2H), 4.25-4.15 (m, 2H), 4.12-4.02 (m, 1H), 4.00-3.89 (m, 3H), 3.75 (ddd, J = 19.6, 10.0, 7.2 Hz, 1H), 1.46 (dd, J = 12.4, 6.0 Hz, 1H), 1.33-1.26 (m, 4H), 1.21 (dd, J = 11.2, 6.4 Hz, 9H), 0.83 (dd, J = 10.4, 4.8 Hz, 6H). |
| 104 | 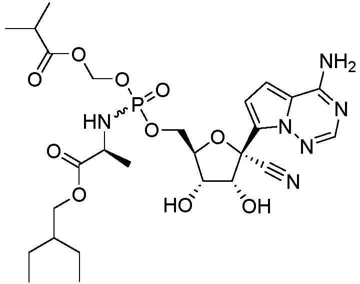 | ¹H NMR: (400 MHz, DMSO-d6) |
| | | δ 8.00-7.79 (m, 3H), 6.90 (d, J = 4.5 Hz, 1H), 6.83 (dd, J = 4.5, 1.6 Hz, 1H), 6.36-6.27 (m, 1H), 5.85-5.76 (m, 1H), 5.53-5.42 (m, 2H), 5.36 (t, J = 6.3 Hz, 1H), 4.66 (dd, J = 11.0, 5.5 Hz, 1H), 4.25-4.11 (m, 2H), 4.04-3.93 (m, 3H), 3.92-3.88 (m, 1H), 3.78-3.68 (m, 1H), 2.59-2.52 (m, 1H), 1.49-1.41 (m, 1H), 1.31-1.26 (m, 4H), 1.24-1.21 (m, 3H), 1.10-1.05 (m, 6H), 0.86-0.80 (m, 6H). |
| 107 | 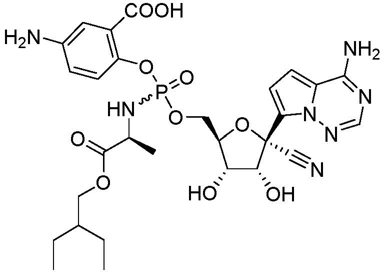 | ¹H NMR: (400 MHz, DMSO-d6) |
| | | δ 9.732 (s, 1H), 8.133 (s, 0.19H), 7.921 (m, 3H), 7.127-7.031 (m, 2H), 6.897-6.851 (m, 2H), 6.789-6.754 (m, 1H), 6.205 (m, 1H), 5.414 (s, 1H), 4.603 (m, 1H), 4.317-4.284 (m, 1H), 4.175-4.161 (m, 1H), 4.121-4.066 (m, 1H), 4.012-3.876 (m, 4H), 1.492-1.431 (m, 1H), 1.355-1.240 (m, 7H), 0.841-0.804 (m, 6H). |
| 128 | 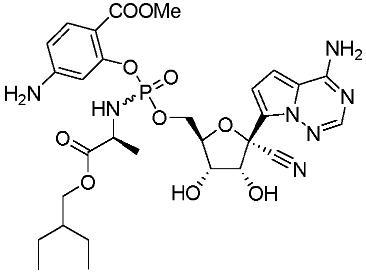 | ¹H NMR: (400 MHz, DMSO-d6) |
| | | δ 8.242 (s, 0.35H), 7.920-7.914 (m, 3H), 7.573-7.540 (m, 1H), 6.897-6.796 (m, 2H), 6.608-6.592 (m, 1H), 6.385-6.340 (m, 2H), 6.115-6.104 (m, 2H), 5.758-5.641 (m, 1H), 5.389 (s, 1H), 4.662-4.619 (m, 1H), 4.304-4.112 (m, 3H), 3.956-3.801 (m, 4H), 1.431-1.383 (m, 1H), 1.266-1.141 (m, 7H), 0.817-0.761 (m, 6H). |
| 160 | 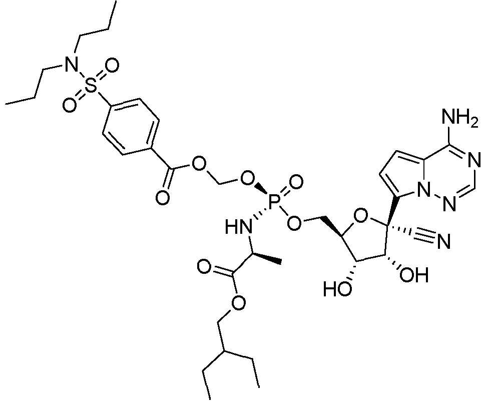 | ¹H NMR: (400 MHz, CD₃OD) |
| | | δ 8.20-8.19 (m, 2H), 7.90-7.84 (m, 3H), 6.90-6.85 (m, 1H), 5.90-5.86 (m, 2H), 4.77 (d, J = 5.2 Hz, 1H), 4.43-4.29 (m, 2H), 4.22-4.19 (m, 1H), 4.13-4.10 (m, 1H), 4.05-4.01 (m, 1H), 3.93-3.87 (m, 2H), 3.09-3.06 (m, 4H), 1.56-1.44 (m, 5H), 1.34-1.27 (m, 7H), 0.87-0.83 (m, 12H). |
| 161 | 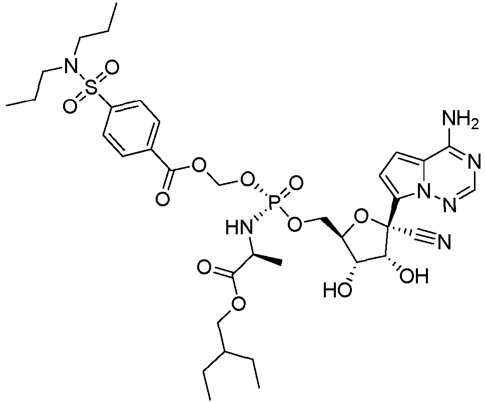 | ¹H NMR: (400 MHz, CD₃OD) |
| | | δ 8.23-8.18 (m, 2H), 7.88-7.84 (m, 3H), 6.90-6.85 (m, 2H), 5.86-5.82 (m, 2H), 4.77 (d, J = 5.6 Hz, 1H), 4.35-4.28 (m, 3H), 4.17-4.14 (m, 1H), 4.06-4.02 (m, 1H), 3.97-3.93 (m, 1H), 3.84-3.82 (m, 1H), 3.09-3.05 (m, 4H), 1.55-1.46 (m, 5H), 1.37-1.27 (m, 7H), 0.89-0.83 (m, 12H). |
| 162 | 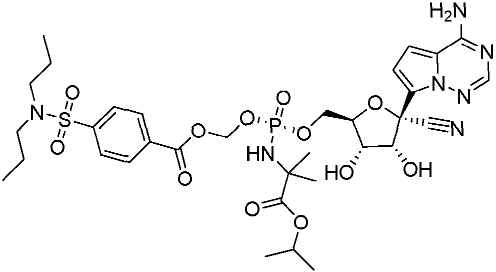 | ¹H NMR: (300 MHz, CD₃OD) |
| | | δ 8.19-8.17 (m, 2H), 7.88-7.84 (m, 3H), 6.90-6.84 (m, 2H), 5.91-5.80 (m, 2H), 4.96-4.90 (m, 1H), 4.88-4.74 (m, 1H), 4.39-4.26 (m, 3H), 4.15-4.11 (m, 1H), 3.08-3.03 (m, 4H), 1.56-1.48 (m, 4H), 1.41 (s, 6H), 1.23-1.21 (m, 6H), 0.87-0.82 (m, 6H). |
| 163 | 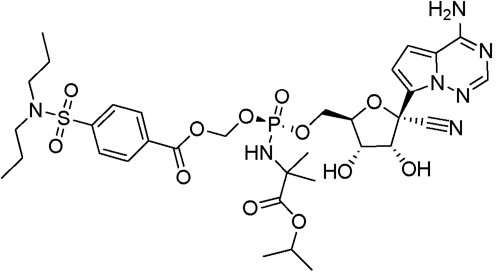 | ¹H NMR: (300 MHz, CD₃OD) |
| | | δ 8.19-8.17 (m, 2H), 7.89-7.84 (m, 3H), 6.91-6.84 (m, 2H), 5.88-5.83 (m, 2H), 4.96-4.90 (m, 1H), 4.85-4.78 (m, 1H), 4.39-4.12 (m, 4H), 3.10-3.05 (m, 4H), 1.59-1.46 (m, 4H), 1.41 (s, 6H), 1.23-1.20 (m, 6H), 0.89-0.83 (m, 6H). |
| 174 | 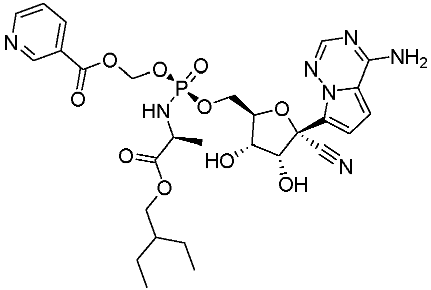 | ¹H NMR: (400MHz, CD₃CN) |
| | | δ 9.03-9.02 (m, 1H), 8.67-8.66 (m, 1H), 8.19-8.17 (m, 1H), 7.82 (s, 1H), 7.37-7.34 (m, 1H), 6.76 (d, J = 4.4 Hz, 1H), 6.65 (d, J = 4.4 Hz, 1H), 6.28 (s, 2H), 5.69-5.65 (m, 2H), 4.68 (s, 1H), 4.60 (d, J = 5.6 Hz, 1H), 4.24-4.11 (m, 4H), 3.91-3.88 (m, 1H), 3.83-3.79 (m, 4H), 1.40-1.32 (m, 1H), 1.23-1.20 (m, 4H), 1.13-1.12 (m, 3H), 0.78-0.74 (m, 6H). |
| 175 | 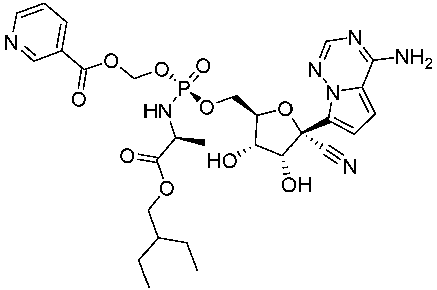 | ¹H NMR: (400MHz, CD₃CN) |
| | | δ 9.16 (s, 1H), 8.80-8.79 (m, 1H), 8.31-7.29 (m, 1H), 7.94 (s, 1H), 7.50-7.46 (m, 1H), 6.89 (d, J = 4.4 Hz, 1H), 6.77 (d, J = 4.4 Hz, 1H), 6.40 (s, 2H), 5.81-5.77 (m, 2H), 4.95 (s, 1H), 4.73 (d, J = 5.6 Hz, 1H), 4.36-4.35 (m, 1H), 4.23-4.17 (m, 4H), 4.05-3.89 (m, 4H), 1.47 (m, 1H), 1.35-1.25 (m, 7H), 0.88-0.84 (m, 6H). |
| 176 | 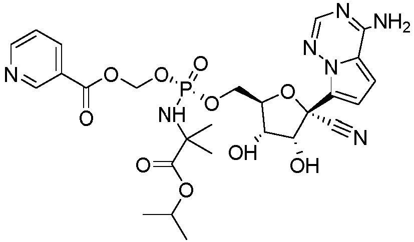 | ¹H NMR: (400 MHz, CD₃CN) |
| | | δ 9.14 (s, 1H), 8.79-8.77 (m, 1H), 8.30-8.27 (m, 1H), 7.94 (s, 1H), 7.47-7.44 (m, 1H), 6.88 (d, J = 4.8 Hz, 1H), 6.75 (d, J = 4.0 Hz, 1H), 6.36 (s, 2H), 5.86-5.75 (m, 2H), 4.94-4.91 (m, 1H), 4.71-4.70 (m, 2H), 4.36-4.25 (m, 2H), 4.25-4.11 (m, 3H), 3.82 (s, 1H), 1.39 (d, J = 6.4 Hz, 6H), 1.22 (s, 6H). |
| 177 | 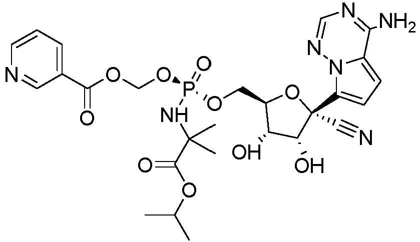 | ¹H NMR: (400 MHz, CD₃CN) |
| | | δ 9.15 (s, 1H), 8.80-8.79 (m, 1H), 8.31-8.28 (m, 1H), 7.94 (s, 1H), 7.49-7.46 (m, 1H), 6.88 (s, 1H), 6.77 (d, J = 4.4 Hz, 1H), 6.34 (s, 2H), 5.79-5.75 (m, 2H), 4.94-4.91 (m, 1H), 4.78-4.74 (m, 2H), 4.36-4.35 (m, 1H), 4.26-4.16 (m, 4H), 3.82-3.80 (m, 1H), 1.39 (s, 6H), 1.22-1.20 (m, 6H). |
| 192 | 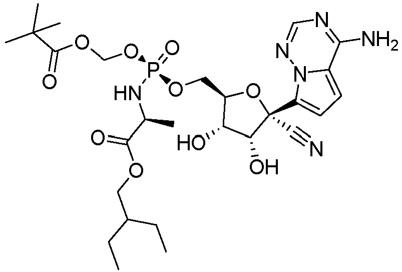 | ¹H NMR: (300MHz, CD₃OD) |
| | | δ 7.87 (s, 1H), 6.95-6.89 (m, 2H), 5.64-5.54 (m, 2H), 4.87-4.82 (m, 1H), 4.35-4.30 (m, 2H), 4.22-4.14 (m, 2H), 4.07-4.02 (m, 1H), 3.96-3.80 (m, 2H), 1.47-1.45 (m, 1H), 1.37-1.27 (m, 7H), 1.20 (s, 9H), 0.92-0.84 (m, 6H). |
| 193 | 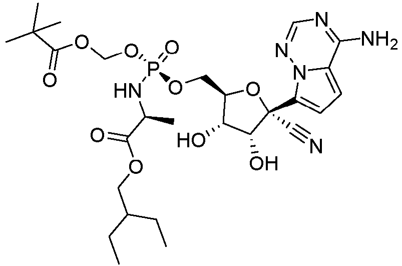 | ¹H NMR: (300MHz, CD₃OD) |
| | | δ 7.87 (s, 1H), 6.95-6.89 (m, 2H), 5.57-5.52 (m, 2H), 4.87-4.85 (m, 1H), 4.39-4.34 (m, 2H), 4.25-4.20 (m, 2H), 4.08-4.04 (m, 1H), 4.01-3.98 (m, 1H), 3.83-3.78 (m, 1H), 1.52-1.48 (m, 1H), 1.40-1.27 (m, 7H), 1.19 (s, 9H), 0.89-0.87 (m, 6H). |
| 194 | 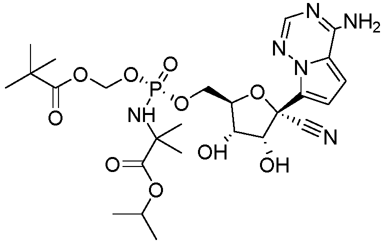 | ¹H NMR: (300 MHz, CD₃CN) |
| | | δ 7.96 (s, 1H), 6.92 (d, J = 4.5 Hz, 1H), 6.81 (d, J = 4.5 Hz, 1H), 6.39 (s, 2H), 5.54-5.49 (m, 2H), 4.98-4.89 (m, 1H), 4.79-4.77 (m, 1H), 4.39-4.35 (m, 1H), 4.30-4.20 (m, 2H), 4.18-4.03 (m, 2H) 1.37 (m, 6H), 1.33 - 1.19 (m, 15H). |
| 195 | 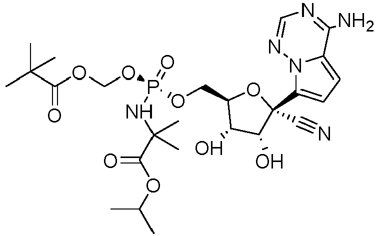 | ¹H NMR: (300 MHz, CD₃CN) |
| | | δ 7.96 (s, 1H), 6.92 (d, J = 4.5 Hz, 1H), 6.80 (d, J = 4.5 Hz, 1H), 6.39 (s, 2H), 5.54-5.44 (m, 2H), 4.98-4.90 (m, 1H), 4.78-4.76 (m, 2H), 4.38 - 4.35 (m, 1H), 4.24-4.13 (m, 3H), 4.09-4.07 (m, 1H), 3.81-3.80 (m, 1H), 1.42 - 1.36 (m, 6H), 1.20 (s, 15H). |
| 267 | 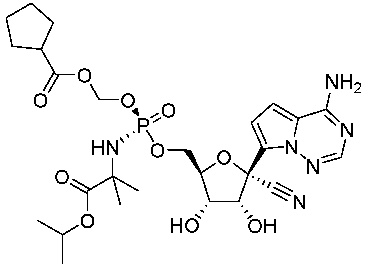 | ¹H NMR: (300 MHz, CD₃OD) |
| | | δ 7.88 (s, 1H), 6.96 - 6.88 (m, 2H), 5.59 - 5.54 (m, 2H), 4.98 - 4.91 (m, 1H), 4.89 - 4.85 (m, 1H), 4.39 - 4.34 (m, 2H), 4.27 - 4.20 (m, 2H), 2.78 - 2.73 (m, 1H), 1.89 - 1.76 (m, 4H), 1.71 - 1.53 (m, 4H), 1.40 - 1.39 (m, 6H), 1.32 - 1.23 (m, 6H). |
| 268 | 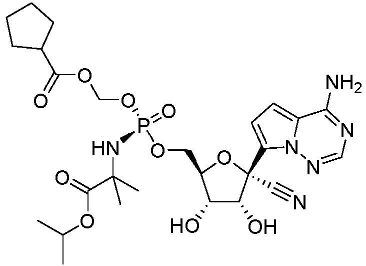 | ¹H NMR: (300 MHz, CD₃OD) |
| | | δ 7.88 (s, 1H), 6.95 - 6.88 (m, 2H), 5.61 - 5.51 (m, 2H), 4.98 - 4.93 (m, 1H), 4.88-4.80 (m, 1H), 4.37 - 4.32 (m, 2H), 4.25 - 4.18 (m, 2H), 2.78 - 2.75 (m, 1H), 1.88 - 1.77 (m, 4H), 1.69 - 1.56 (m, 4H), 1.40 (s, 6H), 1.24 - 1.22 (m, 6H). |
| 269 | 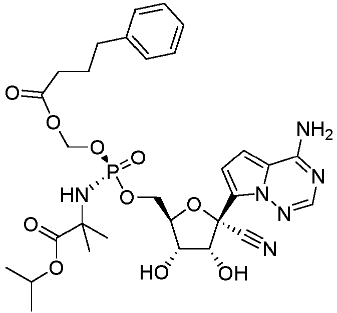 | ¹H NMR: (300 MHz, CD₃OD) |
| | | δ 7.87 (s, 1H), 7.25 - 7.20 (m, 2H), 7.14 - 7.12 (m, 3H), 6.95 - 6.87 (m, 2H), 5.61 - 5.54 (m, 2H), 4.99 - 4.90 (m, 1H), 4.87-4.85 (m, 1H), 4.39 - 4.33 (m, 2H), 4.25 - 4.18 (m, 2H), 2.62 - 2.57 (m, 2H), 2.36 - 2.31 (m, 2H), 1.93 - 1.85 (m, 2H), 1.39 (d, J = 3.0 Hz, 6H), 1.24 - 1.22 (m, 6H). |
| 270 | 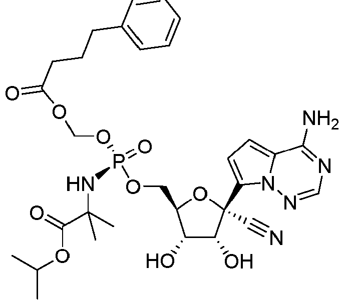 | ¹H NMR: (300 MHz, CD₃OD) |
| | | δ 7.87 (s, 1H), 7.26 - 7.21 (m, 2H), 7.16 - 7.13 (m, 3H), 6.95 - 6.87 (m, 2H), 5.61 - 5.51 (m, 2H), 4.97 - 4.92 (m, 1H), 4.87 - 4.85 (m, 1H), 4.37 - 4.32 (m, 2H), 4.26 - 4.17 (m, 2H), 2.64 - 2.59 (m, 2H), 2.38 - 2.34 (m, 2H), 1.92 - 1.87 (m, 2H), 1.40 (s, 6H), 1.23 - 1.21 (m, 6H). |

### Preparation Example 2: preparation of compound 016

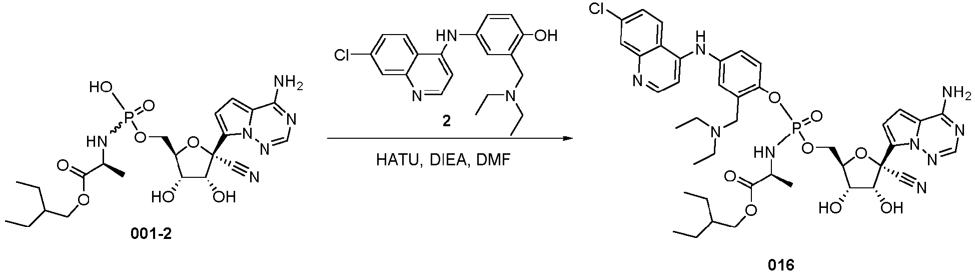

To a stirred solution of compound 001-2 (20 mg, 0.038 mmol, 1.0 equiv.), HATU (21.67 mg, 0.057 mmol, 1.5 equiv.) and compound 2 (13.52 mg, 0.038 mmol, 1.0 equiv.) in DMF (0.5 mL, 25.00 equiv.) was added DIEA (14.73 mg, 0.114 mmol, 3.0 equiv.) dropwise at 0 °C under Ar atmosphere. The resulting mixture was stirred for 1 h at room temperature. The reaction mixture was purified by Prep-HPLC with the following conditions (Column: XBridge Prep C18 OBD Column, 19 x 150 mm, 5 µm; Mobile Phase A: Water (10 mmol/L NH₄HCO₃), Mobile Phase B: ACN; Flow rate: 20 mL/min; Gradient: 60% B to 65% B in 4.5 min, Detector: UV 254/210 nm; RT (min): 4.35 to afford compound 016 (11 mg, 33.50%) as a light yellow solid. Confirmed by LCMS, H NMR P NMR.

¹H NMR:(400 MHz, DMSO-d6) δ 9.08 (s, 1H), 8.44-8.42 (m, 2H), 7.93-7.89 (m, 4H), 7.58-7.55 (m, 1H), 7.45-7.44 (m, 1H), 7.36-7.31 (m, 1H), 7.22-7.18 (m, 1H), 6.91-6.79 (m, 3H), 6.34-6.31 (m, 1H), 6.20-6.14 (m, 1H), 5.40-5.36 (m, 1H), 4.68-4.66 (m, 1H), 4.29-4.26 (m, 2H), 4.18-4.12 (m, 1H), 4.02-3.77 (m, 4H), 3.66-3.46 (m, 2H), 2.53-2.51 (m, 4H), 1.50-1.40 (m, 1H), 1.32-1.22 (m, 7H), 0.98-0.94 (m, 6H), 0.84-0.78 (m, 6H).

Racemic mixture of compound 016 (75 mg, 0.087 mmol) was separated by prep-chiral-HPLC with the following condition: Column: CHIRAL ART Cellulose-SC, 3*25 cm, 5 µm; Mobile Phase A: MtBE (0.5% 2M NH₃-MeOH)--HPLC, Mobile Phase B: EtOH--HPLC; Flow rate: 20 mL/min; Gradient: 15% B to 15% B in 13 min; Wave Length: 254/220 nm; Sample Solvent: EtOH--HPLC; Injection Volume: 0.8 mL; Number Of Runs: 5. RT1: 5.81 min to afford compound 062 (39.5 mg, 50.33% yield) as a light yellow solid. And RT2: 10.64 min to afford compound 063 (25.8 mg, 31.89% yield) as a light yellow solid. Confirmed by ¹H NMR.

According to the invention, additional exemplary compounds were synthesized with reference to the methods in Preparation Example 2 above and are shown in Table 2 below:

**Table 2**

| No. | Structural Formula | Structural Characterization |
|---|---|---|
| 062 | 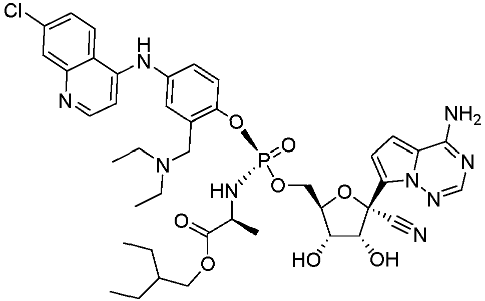 | ¹H NMR: (300 MHz, CD₃OD) δ 8.55-8.44 (m, 2H), 8.02-8.01 (m, 1H), 7.89-7.83 (m, 2H), 7.74-7.66 (m, 2H), 7.61-7.57 (m, 1H), 6.97-6.95 (m, 3H), 4.94-4.85 (m, 3H), 4.52-4.48 (m, 3H), 4.41-4.34 (m, 2H), 4.19-3.97 (m, 4H), 3.34-3.32 (m, 2H), 1.53-1.33 (m, 14H), 0.93-0.88 (m, 6H). |
| 063 | 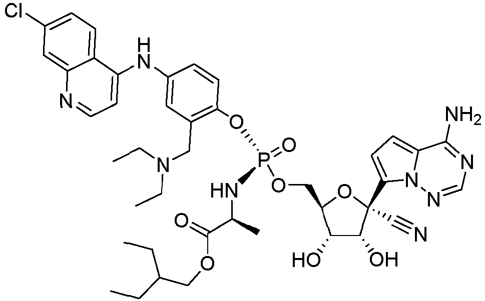 | ¹H NMR: (300 MHz, CD₃OD) δ 8.37-8.28 (m, 2H), 7.90-7.85 (m, 2H), 7.55-7.51 (m, 1H), 7.43-7.40 (m, 2H), 7.28-7.25 (m, 1H), 6.95-6.86 (m, 3H), 4.89-4.85 (m, 2H), 4.43-4.42 (m, 3H), 4.25-4.23 (m, 1H), 4.10-3.95 (m, 3H), 3.89-3.82 (m, 1H), 3.69-3.61 (m, 1H), 2.71-2.68 (m, 3H), 1.53-1.51 (m, 1H), 1.42-1.30 (m, 7H), 1.14-1.09 (m, 6H), 0.93-0.88 (m, 6H). |
| 086 | 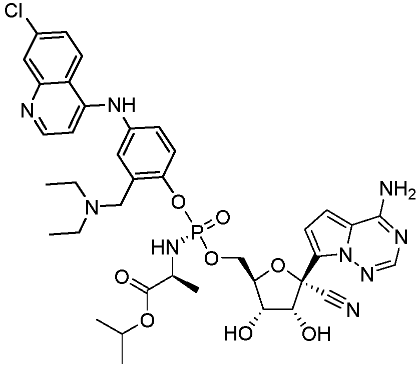 | ¹H NMR (400 MHz, DMSO-d6) δ 9.14-9.06 (m, 1H), 8.50-8.36 (m, 2H), 8.05-7.77 (m, 4H), 7.61-7.52 (m, 1H), 7.46-7.40 (m, 1H), 7.37-7.29 (m, 1H), 7.23- 7.16 (m, 1H), 6.90 (d, J = 4.5 Hz, 1H), 6.84 (t, J = 5.0 Hz, 1H), 6.80 (dd, J = 8.2, 5.4 Hz, 1H), 6.48-6.31 (m, 1H), 6.20-6.11 (m, 1H), 5.55-5.40 (m, 1H),4.90-4.82 (m, 1H), 4.69-4.62 (m, 1H), 4.34-4.20 (m, 2H), 4.18-4.10 (m, 1H), 4.01-3.94 (m, 1H), 3.85-3.74 (m, 1H), 3.66-3.57 (m, 1H), 3.52-3.44 (m,1H), 2.87 (s, 1H), 2.47-2.40 (m, 4H), 1.29-1.07 (m, 9H), 0.99-0.89 (m, 6H). |
| 087 | 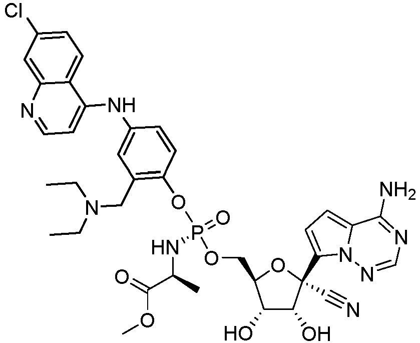 | M+H=794.32 |
| 088 | 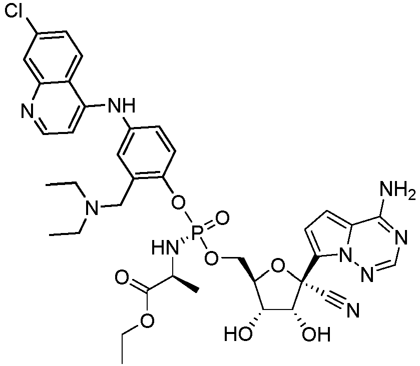 | ¹H NMR (400 MHz, DMSO-d6) δ 9.14-9.06 (m, 1H), 8.47-8.38 (m, 2H), 8.00-7.79 (m, 4H), 7.59-7.54 (m, 1H), 7.45-7.41 (m, 1H), 7.32 (t, J = 8.2 Hz, 1H), 7.23-7.17 (m, 1H), 6.90 (d, J = 4.5 Hz, 1H), 6.87-6.78 (m, 2H), 6.40-6.34 (m, J = 5.4 Hz, 1H), 6.24-6.14 (m, 1H), 5.46-5.38 (m, 1H), 4.69-4.63 (m, J = 4.0 Hz, 1H), 4.31-4.12 (m, 3H), 4.08-3.93 (m, 3H), 3.87-3.75 (m, 1H), 3.64-3.44 (m, 2H), 2.48-2.39 (m, 4H), 1.26-1.10 (m, 6H), 0.97-0.92 (m, 6H). |

### Preparation Example 3: preparation of compound 003

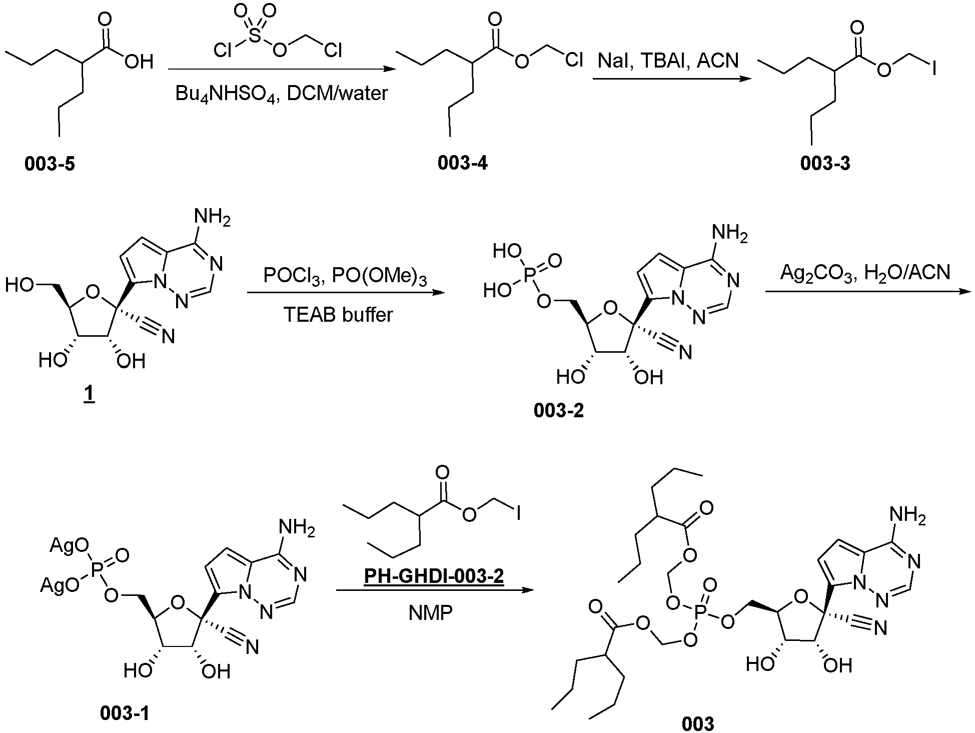

### Preparation of compound 003-4:

To a stirred solution of NaHCO₃ (1165.03 mg, 13.867 mmol, 2.5 equiv.) and Bu₄NHSO₄ (376.70 mg, 1.109 mmol, 0.2 equiv.) in DCM (0.80 mL) and water (4.00 mL) was added a solution of valproic acid (800 mg, 5.547 mmol, 1.00 equiv.) in DCM (1.20 mL) at room temperature under Ar atmosphere. Then a solution of chloromethyl sulfurochloridate (1098.23 mg, 6.656 mmol, 1.2 equiv.) in DCM (6.00 mL) was added dropwise at 0 °C. The resulting mixture was stirred for additional 15 h at room temperature. The reaction mixture was diluted with DCM (100 mL), washed with sat. aq. NaHCO₃ (50 mL) and sat. aq. NaCl (2 x 50 mL), dried over anhydrous MgSO₄, after filtration, the filtrate was concentrated under reduced pressure. The residue was purified by silica gel column chromatography, eluted with EA in PE (0%~50%) to afford compound 003-4 (663 mg, 3.45 mmol, 62.03%) as a colorless oil. Confirmed by H NMR.

### Preparation of compound 003-3:

A mixture of compound 003-4 (420 mg, 2.180 mmol, 1.00 equiv.), NaI (1.31 g, 8.720 mmol, 4 equiv.) and TBAI (1 mg) in ACN (5 mL) was stirred for 15 h at 40 °C under Ar atmosphere. The resulting mixture was concentrated under vacuum. The residue was dissolved with DCM (50 mL) and washed with sat. NaHCO₃ (50 mL), sat. aq. Na₂S₂O₃ (50 mL) and sat. aq. NaCl (2 x 50 mL), dried over anhydrous MgSO₄. After filtration, the filtrate was concentrated under reduced pressure to afford compound 003-3 (470 mg, 75.89%) as a light-yellow oil. Confirmed by H NMR.

### Preparation of compound 003-2:

To a mixture of compound 1 (300 mg, 0.716 mmol, 1.00 equiv.) in trimethyl phosphate (5 mL) was added POCl₃ (219.47 mg, 1.432 mmol, 2 equiv.) at 0 °C. The resulting mixture was stirred for 4 h at 0 °C. The reaction was quenched with TEAB buffer to pH = 7, the resulting mixture was stirred for an additional 30 min. The resulting mixture was diluted with CHCl₃ (10 mL) and water (10 mL). The water phase was extracted with CHCl₃ (2 x 10 mL) then concentrated under reduced pressure. The residue was purified by prep-HPLC with the following condition: Column: XBridge Shield RP18 OBD Column, 30 x 150 mm, 5 µm; Mobile Phase A: 10 mM aq. NH₄HCO₃, Mobile Phase B: ACN; Flow rate: 35 mL / min; Gradient: 0 to 20% B in 11 min, Detector: 254 / 210 nm; RT: 10.2 min to afford compound 003-2 (200 mg, 0.539 mmol, 52.2%) as an off-white solid. Confirmed by LC-MS, H NMR and P NMR.

### Preparation of compound 003-1:

A mixture of compound 003-2 (100 mg, 0.269 mmol, 1.00 equiv.) and Ag₂CO₃ (148.49 mg, 0.538 mmol, 2 equiv.) in ACN (1 mL) and water (1 mL) was stirred for 2 h at 25 °C. The solid was filtered out, the filtrate was concentrated under reduced pressure to afford compound 003-1 (100 mg, 0.171 mmol, 63.46%) as a light brown solid.

### Preparation of compound 003:

To a mixture of compound 003-1 (100 mg, 0.17 mmol, 1.00 equiv.) in NMP (1.5 mL) was added a solution of compound PH-GHDI-003-2 (485.15 mg, 1.7 mmol, 10 equiv.) in NMP (0.5 mL) at 0 °C. The resulting mixture was stirred for 2 h at 25 °C. The mixture was purified by prep-HPLC with the following condition: Column: XBridge Prep C18 OBD Column, 19 x 150 mm, 5 µm; Mobile Phase A: 10 mM aq. NH4HCO3, Mobile Phase B: ACN; Flow rate: 20 mL / min; Gradient: 59% B to 59% B in 11 min, Detector: 254 / 210 nm; RT1: 8.5 min to afford compound 003 (10 mg, 0.014 mmol, 8.61% yield) as an off-white solid. Confirmed by LC-MS, H NMR and P NMR.

¹H NMR: (300 MHz, CD₃OD) δ 7.89 (s, 1H), 6.94-6.88 (m, 2H), 5.64-5.59 (m, 4H), 4.88-4.83 (m, 1H), 4.43-4.34 (m, 3H), 4.19-4.15 (m, 1H), 2.43-2.40 (m, 2H), 1.64-1.54 (m, 4H), 1.48-1.38 (m, 4H), 1.34-1.24 (m, 8H), 0.91-0.84 (m, 12H).

### Preparation of compound 251:

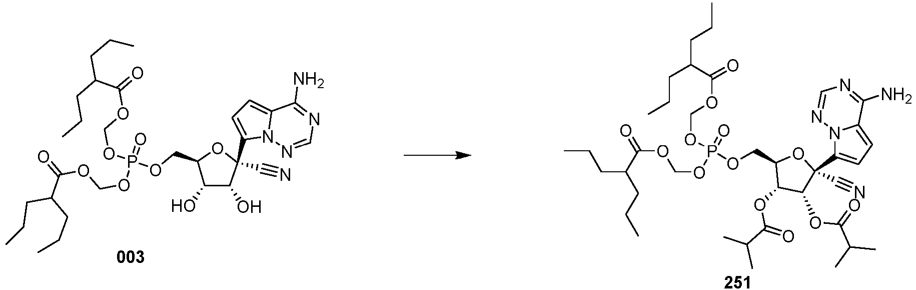

To a mixture of compound 003 (148 mg, 0.216 mmol, 1 equiv.) in DMF (2.5 mL) was added with isobutyric acid (57.07 mg, 0.648 mmol, 3 equiv.) and DIC (81.75 mg, 0.648 mmol, 3 equiv.) at 0 °C under argon. The resulting mixture was stirred for 15 min at room temperature, added with DMAP (13.19 mg, 0.108 mmol, 0.5 equiv.). After the resulting mixture was stirred for 15 h at room temperature, a major amount of desired compound was detected by LCMS. The mixture was purified by RP-flash chromatography under the following conditions: Column: C18 silica gel; Mobile Phase: MeCN in water, Gradient: 0% B to 70% B in 25 min, Detector: 254 / 210 nm, to afford compound 251.

Compound 212 (deuterated compound) was synthesised by reference to compounds 208 and 209.

According to the invention, additional exemplary compounds were synthesized with reference to the methods in Preparation Example 3 above and are shown in Table 3 below:

**Table 3**

| No. | Structural Formula | Structural Characterization |
|---|---|---|
| 164 | 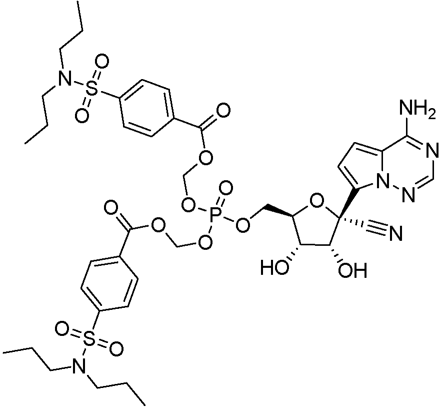 | ¹H NMR: (400 MHz, CD₃CN) δ 8.12-8.09 (m, 4H), 7.92-7.87 (m, 5H), 6.85-6.74 (m, 2H), 6.33 (s, 2H), 5.87-5.81 (m, 4H), 4.70-4.69 (m, 1H), 4.39-4.31 (m, 3H), 4.17-4.16 (m, 1H), 3.11-3.07 (m, 8H), 1.58-1.48 (m, 8H), 0.87-0.83 (m, 12H). |
| 196 | 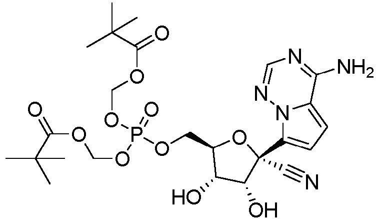 | ¹H NMR: (300 MHz, CD₃CN) δ 7.96 (s, 1H), 6.91 (d, J = 6.0 Hz, 1H), 6.81 (d, J = 6.0 Hz, 1H), 6.38 (s, 2H), 5.59-5.49 (m, 4H), 4.78 (d, J = 6.0 Hz, 1H), 4.40-4.39 (m, 1H), 4.38-4.37 (m, 1H), 4.36-4.30 (m, 2H), 1.20 (s, 18H). |
| 212 | 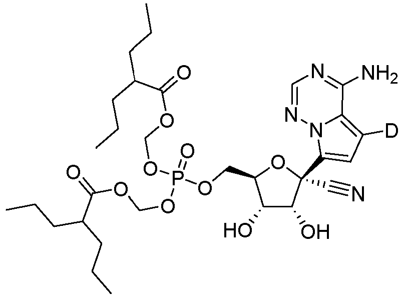 | ¹H NMR: (300MHz, CD₃OD) δ 7.87 (s, 1H), 6.93 (s, 1H), 5.64 - 5.59 (m, 4H), 4.37 - 4.34 (m, 3H), 4.19 - 4.17 (m, 1H), 2.43 - 2.41 (m, 2H), 1.58 - 1.33 (m, 17H), 0.91 - 0.85 (m, 12H). |
| 251 | 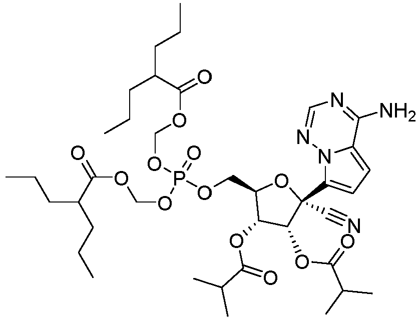 | ¹H NMR: (300MHz, CD₃CN) δ 7.96 (s, 1H), 6.92 (d, J = 4.8 Hz, 1H), 6.80 (d, J = 4.5 Hz, 1H), 6.42 (s, 2H), 6.19 (d, J = 6.0 Hz, 1H), 5.57 - 5.48 (m, 5H), 4.64 - 4.59 (m, 1H), 4.40 - 4.38 (m, 2H), 2.67 - 2.63 (m, 2H), 2.50 - 2.34 (m, 2H), 1.59 - 1.15 (m, 28H), 0.91 - 0.86 (m, 12H). |
| 252 | 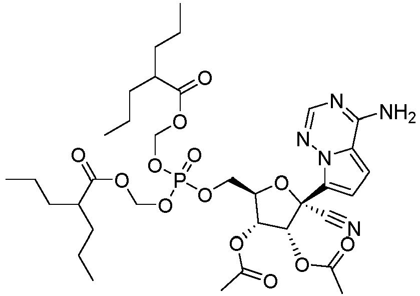 | ¹H NMR: (300MHz, CD₃CN) δ 7.96 (s, 1H), 6.94 (d, J = 4.5 Hz, 1H), 6.81 (d, J = 4.8 Hz, 1H), 6.41 (s, 2H), 6.18 (d, J = 6.0 Hz, 1H), 5.57 - 5.53 (m, 2H), 5.52 - 5.48 (m, 2H), 5.46 - 5.44 (m, 1H) 4.69 - 4.57 (m, 1H), 4.38 - 4.36 (m, 2H), 2.42 - 2.41 (m, 2H), 2.17 - 2.12 (m, 6H), 1.58 - 1.25 (m, 16H), 0.91 - 0.86 (m, 12H). |
| 253 | 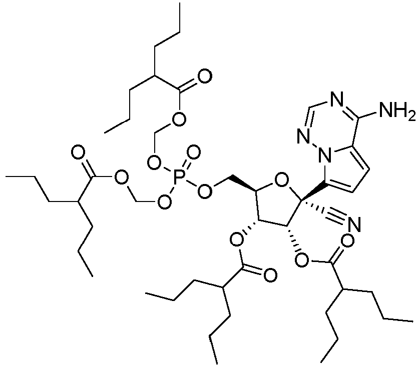 | ¹H NMR: (300 MHz, CD₃CN) δ 7.84 (s, 1H), 6.83 (d, J = 4.8 Hz, 1H), 6.67 (d, J = 4.8 Hz, 1H), 6.34 - 6.32 (m, 3H), 5.46 (d, J = 1.2 Hz, 2H), 5.42 (d, J = 1.2 Hz, 2H), 5.36 - 5.32 (m, 1H), 4.45 - 4.42 (m, 1H), 4.29 - 4.25 (m, 2H), 2.38 - 2.29 (m, 4H), 1.86 - 1.12 (m, 32H), 0.84 - 0.70 (m, 24H). |
| 258 | 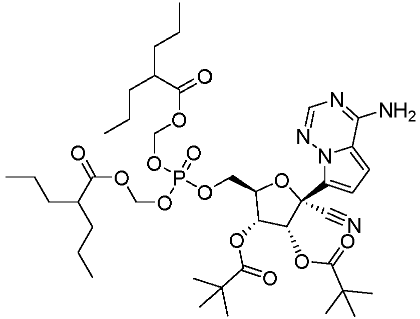 | ¹H NMR: (300MHz, CD₃CN) δ 7.96 (s, 1H), 6.91 (d, J = 4.5 Hz, 1H), 6.80 (d, J = 4.8 Hz, 1H), 6.39 (s, 2H), 6.22 (d, J = 5.7 Hz, 1H), 5.58 (s, 2H), 5.53 (s, 2H), 5.48 - 5.44 (m, 1H), 4.62 - 4.58 (m, 1H), 4.41 - 4.37 (m, 2H), 2.43 - 2.42 (m, 2H), 1.61 - 1.41 (m, 9H), 1.35 - 1.26 (m, 16H), 1.26 - 1.21 (m, 9H), 0.91 - 0.87 (m, 12H). |
| 265 | 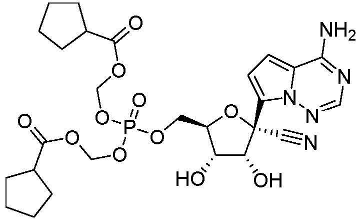 | ¹H NMR: (300MHz, CD₃OD) δ 7.87 (s, 1H), 6.94 - 6.88 (m, 2H), 5.63 - 5.58 (m, 4H), 4.86 (s, 1H), 4.43 - 4.15 (m, 4H), 2.83 - 2.74 (m, 2H), 1.91 - 1.56 (m, 16H). |
| 266 | 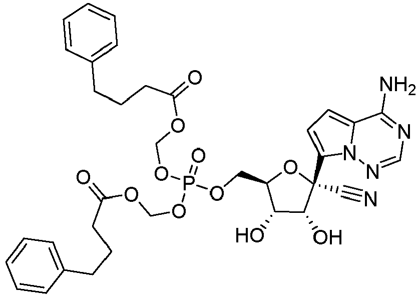 | ¹H NMR: (300MHz, CD₃OD) δ 7.88 (s, 1H), 7.27 - 7.21 (m, 4H), 7.16 - 7.12 (m, 6H), 6.95 - 6.89 (m, 2H), 5.65 - 5.59 (m, 4H), 4.89 - 4.88 (m, 1H), 4.47 - 4.31 (m, 3H), 4.19 - 4.16 (m, 1H), 2.64 - 2.57 (m, 4H), 2.40 - 2.32 (m, 4H), 1.95 - 1.85 (m, 4H). |
| 276 | 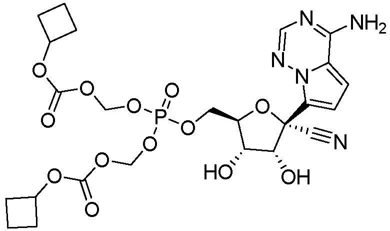 | ¹H NMR: (300 MHz, DMSO-d6) δ 7.92 - 7.89 (m, 3H), 6.91 (d, J = 4.8 Hz, 1H), 6.81 (d, J = 4.5 Hz, 1H), 6.32 (d, J = 6.0 Hz, 1H), 5.59 (s, 2H), 5.54 (s, 2H), 5.39 (d, J = 5.7 Hz, 1H), 4.92 - 4.85 (m, 2H), 4.70 - 4.67 (m, 1H), 4.34 - 4.13 (m, 3H), 3.95 - 3.93 (m, 1H), 2.31 - 2.23 (m, 4H), 2.09 - 2.00 (m, 4H), 1.76 - 1.71 (m, 2H), 1.61 - 1.54 (m, 2H). |
| 277 | 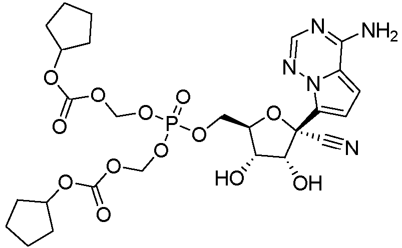 | ¹H NMR: (300 MHz, DMSO-d6) δ 7.92 - 7.89 (m, 3H), 6.90 (d, J = 4.5 Hz, 1H), 6.81 (d, J = 4.5 Hz, 1H), 6.32 (d, J = 6.0 Hz, 1H), 5.59 - 5.54 (m, 4H), 5.47 (d, J = 5.7 Hz, 1H), 5.07 - 5.02 (m, 2H), 4.70 - 4.67 (m, 1H), 4.24 - 4.17 (m, 3H), 3.94 - 3.92 (m, 1H), 1.87 - 1.80 (m, 4H), 1.78 - 1.55 (m, 12H). |

### Preparation Example 4: preparation of compound 057

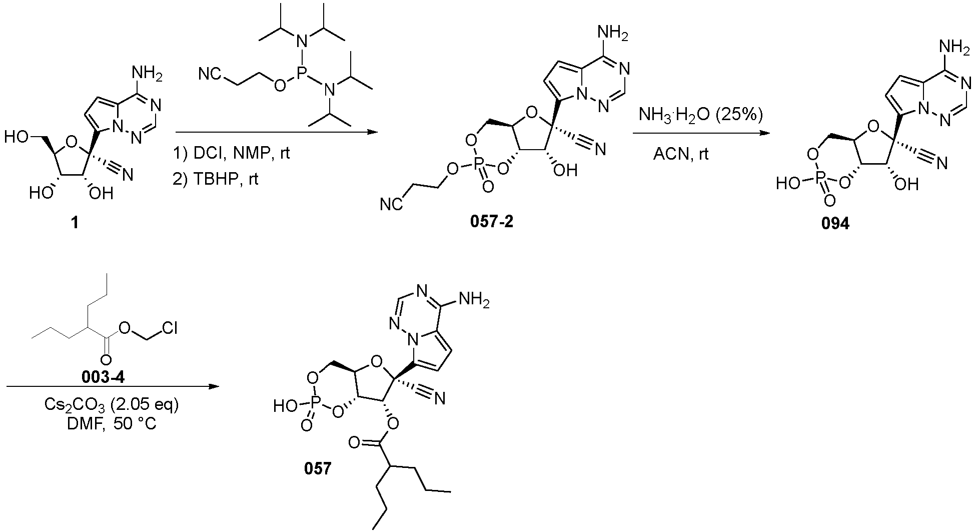

### Preparation of compound 057-2:

To a stirred mixture of compound 1 (1 g, 3.433 mmol, 1.00 equiv.) in NMP (40 mL) were added 1H-imidazole-4,5-dicarbonitrile (810.93 mg, 6.866 mmol, 2 equiv.) and a solution of 3-{[bis(diisopropylamino)phosphanyl]oxy}propanenitrile (1552.26 mg, 5.149 mmol, 1.5 equiv.) in ACN (5 mL) at room temperature under Ar atmosphere. The mixture was stirred at room temperature for 1 h. Then TBHP (3094.10 mg, 34.330 mmol, 10 equiv.) was added to the above mixture. The mixture was stirred at the same temperature for 30 min. 55% product was detected by LCMS. The solvent was removed under reduced pressure, the residue was purified by RP-flash chromatography, eluted with 0-50% MeCN in 5 mM aq. NH₄HCO₃ to give compound 057-2 (560 mg, 40.15% yield) as a white solid.

### Preparation of compound 094:

To a stirred mixture of compound 057-2 (560 mg, 1.378 mmol, 1 equiv, 70%) in ACN (8 mL) was added NH₃.H₂O (8 mL) at room temperature. The mixture was stirred at room temperature for 1 h. Major desired product was detected by LCMS. The solvent was removed under reduced pressure, the residue was purified by prep-HPLC with the following condition: Column: XBridge Prep C18 OBD Column, 19 x 150 mm, 5 µm; Mobile Phase A: 10 mM aq. NH₄HCO₃, Mobile Phase B: ACN; Flow rate: 20 mL / min; Gradient: 10% B to 30% B in 7.5 min, Detector: UV 254 / 210 nm; RT: 7.2 min. The fractions contained the product was concentrated under reduced pressure to give compound 094 (320 mg, 0.91 mmol, 65.73% yield) as a white solid. Confirmed by LCMS H NMR, P NMR.

### Preparation of compound 057:

To a stirred mixture of compound 094 (40 mg, 0.113 mmol, 1 equiv.) and Cs₂CO₃ (75.64 mg, 0.232 mmol, 2.05 equiv.) in DMF (0.4 mL) was added compound 003-4 (44.73 mg, 0.232 mmol, 2.05 equiv.) at room temperature under Ar atmosphere. The mixture was stirred at 50 °C for 15 h. 38% desired mass was detected by LCMS. The mixture was purified by *prep-*HPLC used the following condition: Column: XBridge Prep C18 OBD Column, 19 x 150 mm, 5 µm; Mobile Phase A: 10 mM *aq.* NH₄HCO₃, Mobile Phase B: ACN; Flow rate: 20 mL / min; Gradient: 30% B to 50% B in 6.5 min, Detector: UV 254 / 210 nm; RT: 5.8 min. The fractions contained product was lyophilized directly to give compound 057 (8 mg, 0.017 mmol, 14.74% yield) as a white solid. Confirmed by LCMS, H NMR, P NMR.

Compound 094: ¹H NMR: (400 MHz, CD₃OD) δ 7.801 (s, 1H), 6.804-6.792 (d, J = 4.8 Hz, 1H), 6.707-6.696 (d, J = 4.4 Hz, 1H), 4.688-4.679 (m, 1H), 4.390-4.285 (m, 3H), 4.199-4.130 (m, 1H).

Compound 057: ¹H NMR: (300 MHz, CD₃OD) δ 7.84 (s, 1H), 6.91-6.85 (m, 2H), 5.96 (d, J = 6.0 Hz, 1H), 4.71-4.67 (m, 1H), 4.50-4.27 (m, 3H), 2.59-2.55 (m, 1H), 1.78-1.72 (m, 2H), 1.58-1.37 (m, 6H), 0.97-0.91 (m, 6H).

**Table 4**

| No. | Structural Formula | Structural Characterization |
|---|---|---|
| 158 | 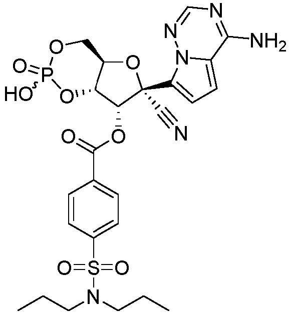 | ¹H NMR: (300 MHz, CD₃OD) δ 8.38 - 8.35 (m, 2H), 8.00 - 7.92 (m, 3H), 6.98 - 6.93 (m, 2H), 6.26 - 6.24 (m, 1H), 4.86- 4.78 (m, 1H), 4.55 - 4.41 (m, 2H), 4.35 - 4.28 (m, 1H), 3.16 - 3.07 (m, 4H), 1.63 - 1.51 (m, 4H), 0.92 - 0.84 (m, 6H). |
| 181 | 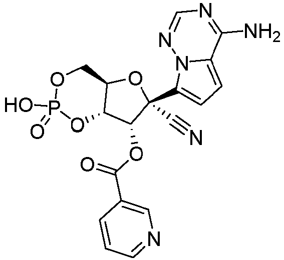 | ¹H NMR: (400 MHz, DMSO-d6) δ 9.31-9.30 (m, 1H), 8.94-8.92 (m, 1H), 8.50-8.48 (m, 1H), 8.37-8.23 (m, 2H, NH2), 8.01 (s, 1H), 7.71-7.68 (m, 1H), 7.04-6.97 (m, 2H), 6.26-6.25 (m, 1H), 4.75-4.71 (m, 1H), 4.67-4.54 (m, 3H), 4.27-4.22 (m, 1H). |
| 199 | 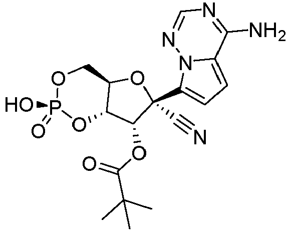 | ¹H NMR: (300 MHz, D₂O) δ 7.77 (d, J = 3.0 Hz, 1H), 6.78-6.70 (m, 2H), 5.87 (s, 1H), 4.70-4.57 (m, 1H), 4.44-4.40 (m, 2H), 4.17-4.14 (m, 1H), 1.23 (s, 9H). |
| 200 | 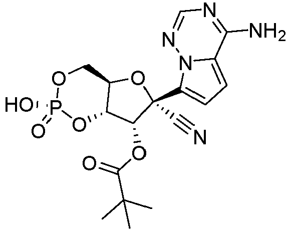 | ¹H NMR: (300 MHz, D₂O) δ 7.90 (d, J = 3.0 Hz, 1H), 6.88 (s, 1H), 6.80 (s, 1H), 5.80 (d, J = 6.0 Hz, 1H), 5.66-5.58 (m, 1H), 4.45-4.36 (m, 3H), 0.57 (s, 9H). |
| 260 | 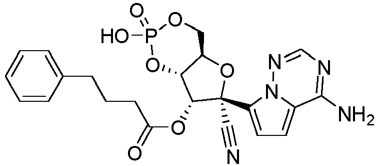 | ¹H NMR: (400 MHz, CD₃OD) δ 7.89 (s, 1H), 7.28 - 7.22 (m, 4H), 7.17 - 7.13 (m, 1H), 6.96 (d, J = 4.8 Hz, 1H), 6.91 (d, J = 4.4 Hz, 1H), 6.01 (s, 1H), 4.81 - 4.77 (m, 1H), 4.48 - 4.41 (m, 1H), 4.34 - 4.27 (m, 2H), 2.73 - 2.70 (m, 2H), 2.56 - 2.50 (m, 2H), 2.06 - 1.99 (m, 2H). |
| 280 | 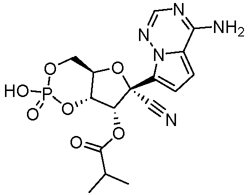 | ¹H NMR: (400MHz, DMSO-d6) δ 8.08 - 7.95 (m, 3H), 6.96 (d, J = 4.4 Hz, 1H), 6.86 (d, J = 4.4 Hz, 1H), 5.84 (d, J = 4.8 Hz, 1H), 4.44 (s, 1H), 4.26 - 4.19 (m, 1H), 4.12 - 3.97 (m, 2H), 2.78 - 2.68 (m, 1H), 1.25 - 1.07 (m, 6H). |
| 281 | 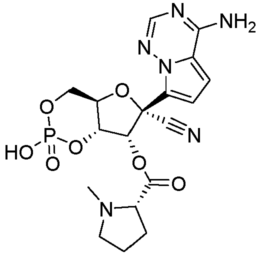 | ¹H NMR: (400 MHz, DMSO-d6) δ 8.11 - 7.95 (m, 3H), 6.96 (d, J = 4.4 Hz, 1H), 6.87 (d, J = 4.8 Hz, 1H), 5.97 (d, J = 4.8 Hz, 1H), 4.51 (s, 1H), 4.49 - 4.48 (m, 1H), 4.26 - 4.18 (m, 1H), 4.14 - 4.07 (m, 1H), 4.02 - 3.97 (m, 1H), 13.57 - 3.49 (m, 2H), 2.83 (d, J = 4.4 Hz, 3H), 2.23 - 1.95 (m, 4H). |
| 288 | 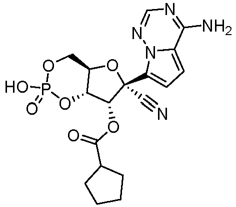 | ¹H NMR: (300 MHz, DMSO-d6) δ 8.54 (s, 1H), 8.06 - 7.93 (m, 3H), 6.95 (d, J = 4.8 Hz, 1H), 6.86 (d, J = 4.5 Hz, 1H), 5.85 (d, J = 5.1 Hz, 1H), 4.46 - 4.41 (m, 1H), 4.29 - 4.18 (m, 1H), 4.08 - 4.04 (m, 1H), 4.04 - 3.98 (m, 1H), 2.97 - 2.94 (m, 1H), 2.92 - 2.88 (m, 4H), 1.95 - 1.85 (m, 4H), 1.68 - 1.57 (m, 4H), 1.27 - 1.13 (m, 6H). |

### Preparation Example 5: preparation of compounds 077 and 145

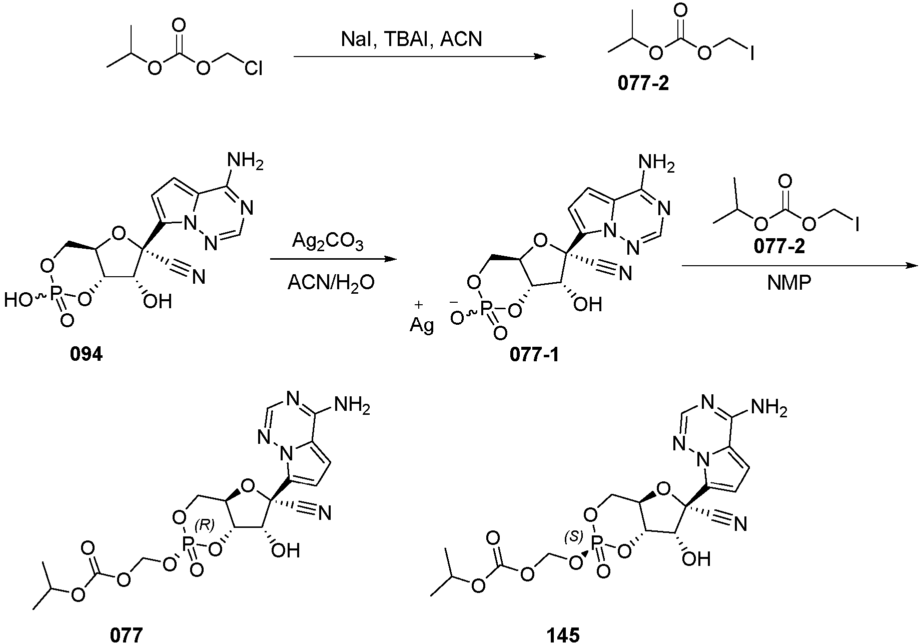

### Preparation of compound 077-2:

To a stirred solution of chloromethyl isopropyl carbonate (5 g, 32.772 mmol, 1 equiv.) in ACN (70 mL) were added NaI (19.7 g, 131.426 mmol, 4.01 equiv.) and TBAI (5 mg) at room temperature under Ar atmosphere. The resulting mixture was stirred at 40 °C for 16 h and then cooled down to room temperature. The resulting mixture was diluted with DCM (200 mL), washed with *sat. aq.* NaHCO₃ (50 mL), *sat. aq.* Na₂S₂O₃ (50 mL) and *sat. aq.* NaCl (2 x 50 mL), dried over anhydrous MgSO₄. After filtration, the filtrate was concentrated under reduced pressure to afford compound 077-2 (7.3 g, 0.030 mol, 91.28%) as a lightyellow oil. Confirmed by H NMR.

### Preparation of compound 077-1:

To a stirred solution of compound 094 (1 g, 2.831 mmol, 1 equiv.) in ACN (10 mL) and H2O (10 mL) was added Ag2CO3 (858.70 mg, 3.114 mmol, 1.1 equiv.) at room temperature. The resulting mixture was stirred at room temperature for 2 h then filtered. The filtrate was lyophilized directly to give compound 077-1 (1.3 g, 2.830 mmol, 99.81%) as a light yellow solid.

### Preparation of compound 077 and compound 145:

To a stirred solution of compound 077-1 (90 mg, 0.196 mmol, 1 equiv.) in NMP (1.5 mL) was added a solution of compound 077-2 (143.21 mg, 0.588 mmol, 3 equiv.) in NMP (0.3 mL) at 0 °C under Ar atmosphere. The resulting mixture was stirred at room temperature for 1.5 h. 40% desired mass was detected by LCMS. The mixture was purified by prep-HPLC used the following condition: Column: XBridge Prep C18 OBD Column, 19 x 150 mm, 5 µm; Mobile Phase A: 10 mM aq. NH₄HCO₃, Mobile Phase B: ACN; Flow rate: 20 mL / min; Gradient: 35% B to 35% B in 5.8 min, Detector: 254 / 210 nm; RT1: 4.8 min to give compound 077 (4.3 mg, 0.009 mmol, 4.68%) as a white solid and RT2: 5.5 min to give compound 145 (6.6 mg, 0.014 mmol, 7.18%) as a white solid. Confirmed by LC-MS, H NMR, P NMR.

Compound 077: ¹H NMR: (300 MHz, CD₃OD) δ 7.90 (s, 1H), 6.91-6.87 (m, 2H), 5.76-5.65 (m, 2H), 4.83-4.52 (m, 6H), 1.34-1.32 (m, 6H).

Compound 145: ¹H NMR: (300 MHz, CD₃OD) δ 7.91 (s, 1H), 6.91-6.85 (m, 2H), 5.69-5.64 (m, 2H), 4.72-4.43 (m, 6H), 1.26-1.20 (m, 3H), 1.16-1.14 (m, 3H).

### Preparation of compound 245:

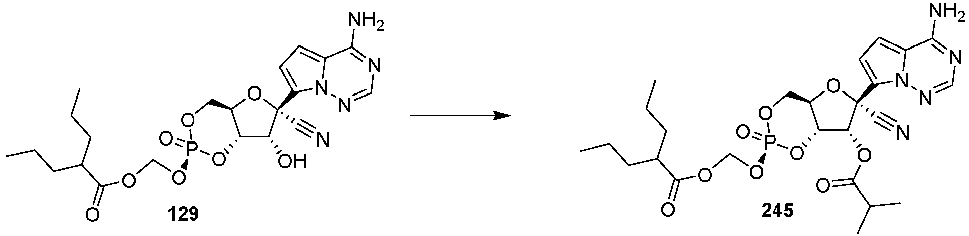

To a mixture of compound 129 (110 mg, 0.216 mmol, 1 equiv.) in DMF (2.5 mL) was added with isobutyric acid (57.07 mg, 0.648 mmol, 3 equiv.) and DIC (81.75 mg, 0.648 mmol, 3 equiv.) at 0 °C under argon. The resulting mixture was stirred for 15 min at room temperature, added with DMAP (13.19 mg, 0.108 mmol, 0.5 equiv.). After the resulting mixture was stirred for 15 h at room temperature, a major amount of desired compound was detected by LCMS. The mixture was purified by RP-flash chromatography under the following conditions: Column: C18 silica gel; Mobile Phase: MeCN in water, Gradient: 0% B to 70% B in 25 min, Detector: 254 / 210 nm, to afford compound 245.

Compounds 210 and 211 were synthesised by reference to compounds 208 and 209.

According to the invention, additional exemplary compounds were synthesized with reference to the methods in Preparation Example 5 above and are shown in Table 4 below:

**Table 4**

| No. | Structural Formula | Structural Characterization |
|---|---|---|
| 141 | 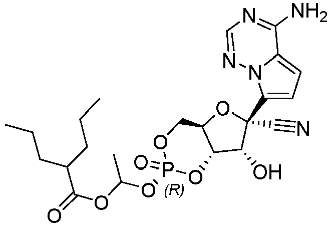 | ¹H NMR: (300MHz, DMSO-d6) |
| | | δ 8.10-7.89 (m, 3H), 7.38 (d, J = 6.0 Hz, 1H), 6.94 (d, J = 4.5 Hz, 1H), 6.78 (d, J = 4.5 Hz, 1H), 6.37-6.34 (m, 1H), 4.91-4.75 (m, 2H), 4.60-4.42 (m, 1H), 4.39-4.22 (m, 2H), 2.26-2.13 (m, 1H), 1.50 (d, J = 5.4 Hz, 3H), 1.36-1.18 (m, 6H), 1.16-1.03 (m, 2H), 0.78-0.66 (m, 6H). |
| 142 | 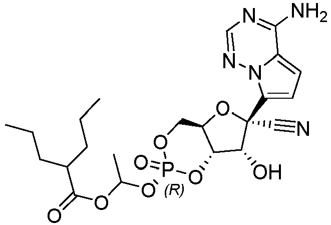 | ¹H NMR: (300MHz, DMSO-d6) |
| | | δ 8.02-7.92 (m, 3H), 7.28 (d, J = 5.7 Hz, 1H), 6.93 (d, J = 4.5 Hz, 1H), 6.78 (d, J = 4.5 Hz, 1H), 6.45-6.41 (m, 1H), 4.89-4.75 (m, 2H), 4.44-4.29 (m, 3H), 2.36-2.30 (m, 1H), 1.51-1.41 (m, 7H), 1.27-1.15 (m, 4H), 0.84-0.76 (m, 6H). |
| 129 | 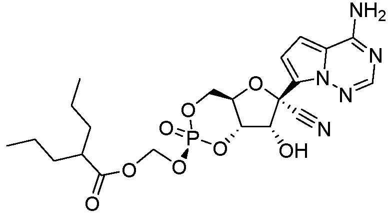 | ¹H NMR: (400MHz, CD₃OD) |
| | | δ 7.91 (s, 1H), 6.91-6.85 (m, 2H), 5.69 (d, J = 12.8 Hz, 2H), 4.89-4.88 (m, 1H), 4.84-4.68 (m, 2H), 4.59-4.53 (m, 1H), 4.50-4.40 (m, 1H), 2.42-2.35 (m, 1H), 1.58-1.48 (m, 2H), 1.44-1.34 (m, 2H), 1.32-1.15 (m, 4H), 0.84-0.78 (m, 6H). |
| 130 | 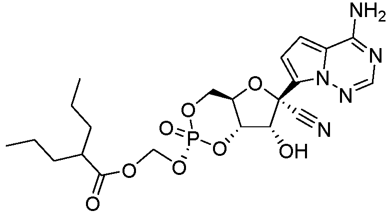 | ¹H NMR: (400MHz, CD₃OD) |
| | | δ 7.90 (s, 1H), 6.94-6.85 (m, 2H), 5.74 (d, J = 13.6 Hz, 2H), 4.93-4.75 (m, 3H), 4.72-4.66 (m, 1H), 4.57-4.50 (m, 1H), 2.53-2.46 (m, 1H), 1.70-1.61 (m, 2H), 1.56-1.43 (m, 2H), 1.43-1.26 (m, 4H), 0.95-0.86 (m, 6H). |
| 146 | 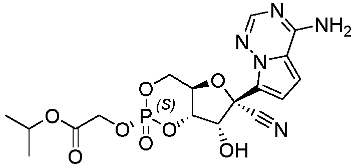 | ¹H NMR: (400 MHz, CD₃OD) |
| | | δ 7.91 (s, 1H), 6.94-6.81 (m, 2H), 5.10-5.01 (m, 1H), 4.95-4.89 (m, 2H), 4.76-4.74 (m, 1H), 4.67-4.61 (m, 2H), 4.58-4.52 (m, 2H), 1.28-1.19 (m, 6H). |
| 147 | 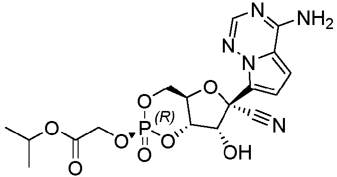 | ¹H NMR: (400 MHz, CD₃OD) |
| | | δ 7.90 (s, 1H), 6.93-6.85 (m, 2H), 5.15-5.10 (m, 1H), 4.95-4.82 (m, 4H), 4.76-4.74 (m, 2H), 4.54-4.50 (m, 1H), 1.30-1.26 (m, 6H). |
| 197 | 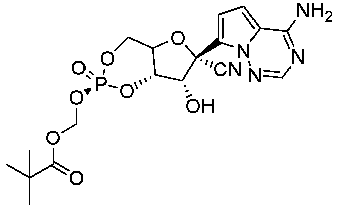 | ¹H NMR: (400 MHz, CD₃CN) δ 7.98 (s, 1H), 6.74 - 6.69 (m, 2H), 6.35 (brs, 2H), 5.56 - 5.47 (m, 2H), 4.80-4.79 (m, 1H), 4.66-4.58 (m, 1H), 4.43-4.41 (m, 2H), 4.29-4.25 (m, 1H), 1.02 (s, 9H). |
| 198 | 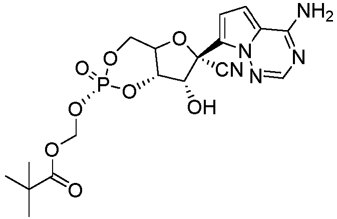 | ¹H NMR: (400 MHz, CD₃CN) δ 7.97 (s, 1H), 6.85 - 6.80 (m, 2H), 6.45 (brs, 2H), 5.71 - 5.63 (m, 2H), 4.93-4.92 (m, 1H), 4.77-4.63 (m, 3H), 4.51-4.47 (m, 1H), 1.25 (s, 9H). |
| 179 | 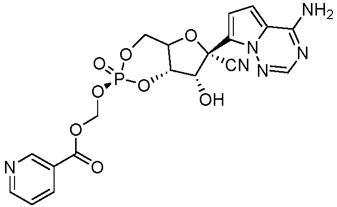 | ¹H NMR: (300 MHz, CD₃OD) δ 9.05-9.02 (m, 1H), 8.72-8.70 (m, 1H), 8.31-8.27 (m, 1H), 7.84 (s, 1H), 7.49-7.45 (m, 1H), 6.84-6.76 (m, 2H), 6.05-5.90 (m, 2H), 4.82-4.75 (m, 1H), 4.66-4.61 (m, 2H), 4.59-4.51 (m, 2H). |
| 180 | 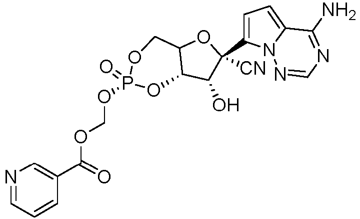 | ¹H NMR: (300 MHz, CD₃OD) |
| | | δ 9.21-9.17 (m, 1H), 8.81-8.79 (m, 1H), 8.53-8.50 (m, 1H), 7.88 (s,1H), 7.63-7.59 (m, 1H), 6.90-6.85 (m, 2H), 6.05-5.96 (m, 2H), 4.90-4.89 (m, 2H), 4.84-4.80 (m, 2H), 4.78-4.70 (m, 1H). |
| 210 | 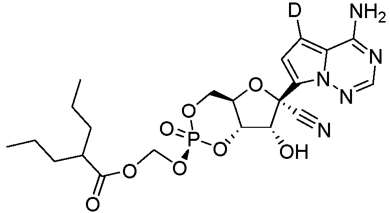 | ¹H NMR: (300 MHz, CD₃CN) |
| | | δ 7.98 (s, 1H), 6.84 (s, 1H), 6.40 (s, 2H), 5.64 (d, J = 13.2 Hz, 2H), 4.93-4.89 (m, 2H), 4.77-4.68 (m, 1H), 4.56-4.52 (m, 2H), 4.41-4.38 (m, 1H), 2.43-2.40 (m, 1H), 1.56-1.39 (m, 4H), 1.30-1.20 (m, 4H), 0.86-0.79 (m, 6H). |
| 211 | 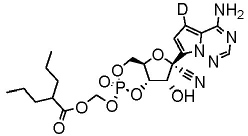 | ¹H NMR: (300 MHz, CD₃CN) |
| | | δ 7.86 (s, 1H), 6.85 (s, 1H), 6.38 (s, 2H), 5.70-5.65 (m, 2H), 4.93-4.77 (m, 2H), 4.74-4.65 (m, 3H), 4.53-4.47 (m, 1H), 2.52-2.47 (m, 1H), 1.67-1.62 (m, 2H), 1.57-1.45 (m, 2H), 1.41-1.28 (m, 4H), 0.95-0.82 (m, 6H). |
| 228 | 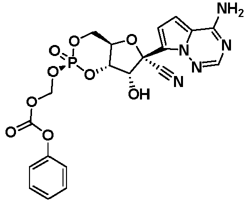 | ¹H NMR: (300 MHz, CD₃OD) |
| | | δ 7.95 (s, 1H), 7.40 - 7.35 (m, 2H), 7.29 - 7.27 (m, 1H), 7.24 - 7.11 (m, 3H), 6.97 (d, J = 4.5 Hz, 1H), 5.84 - 5.77 (m, 2H), 4.91 - 4.90 (m, 1H), 4.81 - 4.80 (m, 1H), 4.66 - 4.50 (m, 3H). |
| 229 | 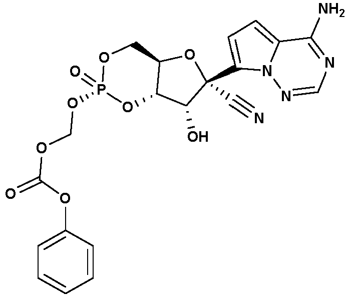 | ¹H NMR: (300 MHz, CD₃OD) |
| | | δ 8.07 (s, 1H), 7.46 - 7.41 (m, 2H), 7.32 - 7.25 (m, 4H), 7.01 (d, J = 4.5 Hz, 1H), 5.89 - 5.77 (m, 2H), 4.93 - 4.92 (m, 2H), 4.78 - 4.74 (m, 2H), 4.64 - 4.58 (m, 1H). |
| 245 | 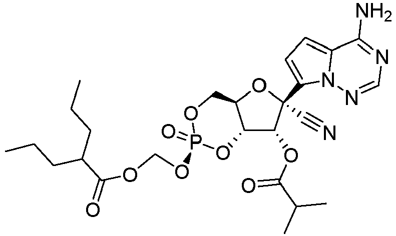 | ¹H NMR: (300 MHz, CD₃CN) |
| | | δ 7.94 (s, 1H), 6.91 - 6.80 (m, 2H), 6.46 (s, 2H), 5.99 (d, J = 5.4 Hz, 1H), 5.67 - 5.62 (m, 2H), 4.95 - 4.93 (m, 1H), 4.53 - 4.50 (m, 1H), 4.42 - 4.38 (m, 2H), 2.81 - 2.76 (m, 1H), 2.41 - 2.35 (m, 1H), 1.55 - 1.39 (m, 4H),1.29 - 1.20 (m, 10H), 0.84 - 0.77 (m, 6H). |
| 246 | 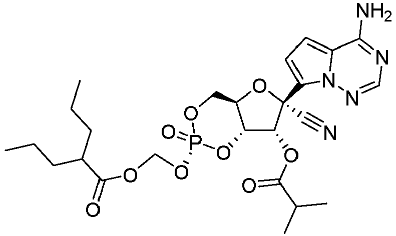 | ¹H NMR: (300MHz, CD₃CN) |
| | | δ 7.93 (s, 1H), 6.91 (d, J = 4.5 Hz, 1H), 6.80 (d, J = 4.8 Hz, 1H), 6.40 (s, 2H), 6.04 (d, J = 5.4 Hz, 1H), 5.63 (d, J = 12.9 Hz, 2H), 5.12 - 5.09 (m, 1H), 4.72 - 4.47 (m, 3H), 2.79 - 2.75 m, 1H), 2.48 - 2.44 (m, 1H), 1.64 - 1.42 (m, 4H), 1.38 - 1.25 (m, 10H), 0.93 - 0.88 (m, 6H). |
| 247 | 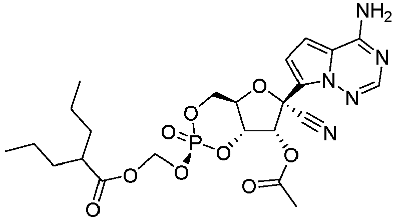 | ¹H NMR: (400MHz, CD₃CN) |
| | | δ 7.97 (s, 1H), 6.92 (d, J = 4.8 Hz, 1H), 6.81 (d, J = 4.8 Hz, 1H), 6.45 (s, 2H), 6.00 (d, J = 5.2 Hz, 1H), 5.67 - 5.63 (m, 2H), 5.02 - 4.98 (m, 1H), 4.75 - 4.69 (m, 1H), 4.52 - 4.48 (m, 1H), 4.40 - 4.35 (m, 1H), 2.43 - 2.39 (m, 1H), 2.25 (s, 3H), 1.56 - 1.38 (m, 4H), 1.27 - 1.22 (m, 4H), 0.83 - 0.78 (m, 6H). |
| 248 | 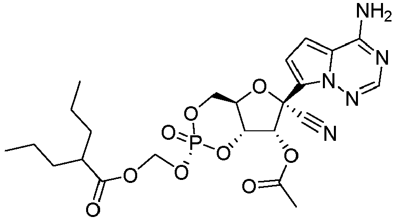 | ¹H NMR: (400MHz, CD₃CN) |
| | | δ 7.96 (s, 1H), 6.92 (d, J = 4.8 Hz, 1H), 6.80 (d, J = 4.8 Hz, 1H), 6.43 (s, 2H), 6.05 (d, J = 5.2 Hz, 1H), 5.67 - 5.64 (m, 2H), 5.13 - 5.09 (m, 1H), 4.73 - 4.47 (m, 3H), 2.47 - 2.46 (m, 1H), 2.26 (s, 3H), 1.63 - 1.56 (m, 2H), 1.51 - 1.45 (m, 2H), 1.37 - 1.29 (m, 4H), 0.92 - 0.89 (m, 6H). |
| 249 | 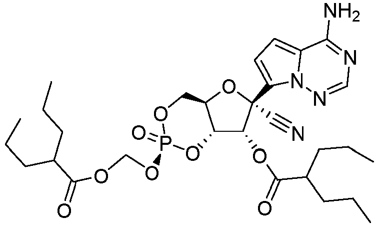 | ¹H NMR: (400 MHz, CD₃CN) |
| | | δ 7.82 (s, 1H), 6.78 - 6.71 (m, 2H), 6.39 (s, 2H), 5.89 (d, J = 5.2 Hz, 1H), 5.56 - 5.51 (m, 2H), 4.64 - 4.42 (m, 2H), 4.34 - 4.31 (m, 2H), 2.53 - 2.46 (m, 1H), 2.33 - 2.25 (m, 1H), 1.85 - 1.84 (m, 2H), 1.63 - 1.27 (m, 10H), 1.16 - 1.10 (m, 4H), 0.85 - 0.81 (m, 6H), 0.74 - 0.67 (m, 6H). |
| 255 | 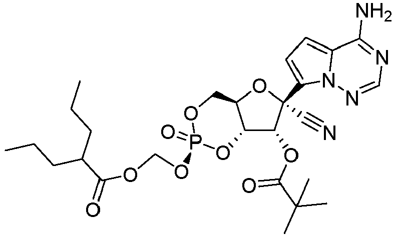 | ¹H NMR: (300 MHz, CD₃CN) |
| | | δ 7.97 (s, 1H), 6.93 (d, J = 4.5 Hz, 1H), 6.83 (d, J = 4.5 Hz, 1H), 6.47 (s, 2H), 6.00 (d, J = 5.1 Hz, 1H), 5.69 - 5.63 (m, 2H), 4.96 - 4.92 (m, 1H), 4.79 - 4.65 (m, 1 H), 4.48 - 4.45 (m, 1H), 4.42 - 4.38 (m, 1H), 2.44 - 2.18 (m, 1H), 1.98 - 1.95 (m, 4H), 1.57 - 1.42 (m, 9H), 1.37 - 1.23 (m, 4H), 0.87 - 0.79 (m, 6H). |
| 261 | 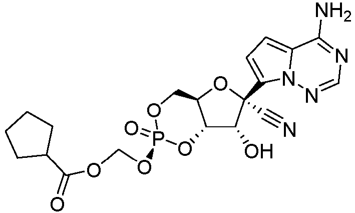 | ¹H NMR: (400MHz, CD₃CN) |
| | | δ 7.99 (s, 1H), 6.83 - 6.79 (m, 2H), 6.42 (s, 2H), 5.65 - 5.56 (m, 2H), 4.87 (d, J = 4.4 Hz, 1H), 4.74 - 4.68 (m, 1H), 4.53 - 4.47 (m, 2H), 4.40 - 4.35 (m, 1H), 2.74 - 2.70 (m, 1H), 1.80 - 1.75 (m, 2H), 1.66 - 1.49 (m, 6H). |
| 262 | 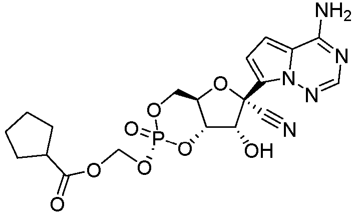 | ¹H NMR: (400 MHz, CD₃CN) |
| | | δ 7.97 (s, 1H), 6.83 - 6.78 (m, 2H), 6.39 (s, 2H), 5.66 - 5.63 (m, 2H), 4.91 - 4.90 (m, 1H), 4.74 - 4.64 (m, 3H), 4.49 - 4.45 (m, 1H), 2.89 - 2.84 (m, 1H), 1.97 - 1.94 (m, 1H), 1.94 - 1.90 (m, 1H), 1.90 - 1.80 (m, 2H), 1.71 - 1.59 (m, 3H). |
| 263 | 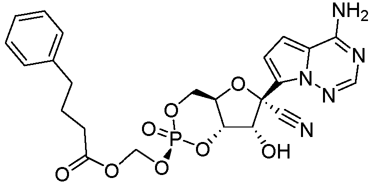 | ¹H NMR: (400 MHz, CD₃CN) |
| | | δ 7.86 (s, 1H), 7.16 - 7.12 (m, 2H), 7.07 - 7.03 (m, 3H), 6.74 (d, J = 4.8 Hz, 1H), 6.70 (d, J = 4.8 Hz, 1H), 6.32 (s, 2H), 5.56 - 5.48 (m, 2H), 4.73 - 4.72 (m, 1H), 4.66 - 4.56 (m, 1H), 4.49 - 4.40 (m, 2H), 4.30 - 4.24 (m, 1H), 2.50 - 2.46 (m, 2H), 2.25 - 2.21 (m, 2H), 1.87 - 1.84 (m, 2H). |
| 264 | 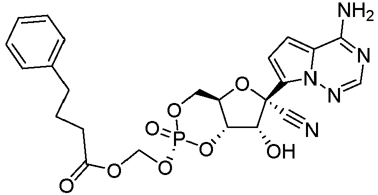 | ¹H NMR: (400 MHz, CD₃CN) |
| | | δ 7.86 (s, 1H), 7.22 - 7.19 (m, 2H), 7.15 - 7.09 (m, 3H), 6.74 - 6.69 (m, 2H), 6.32 (s, 2H), 5.56 (d, J = 5.6, 1H), 5.54 (d, J = 5.6, 1H), 4.80 (d, J = 3.6, 1H), 4.66 - 4.53 (m, 3H), 4.40 - 4.33 (m, 1H), 2.60 - 2.56 (m, 2H), 2.36 - 2.33 (m, 2H), 1.88 - 1.80 (m, 2H). |

### Preparation Example 6: preparation of compounds 126 and 140

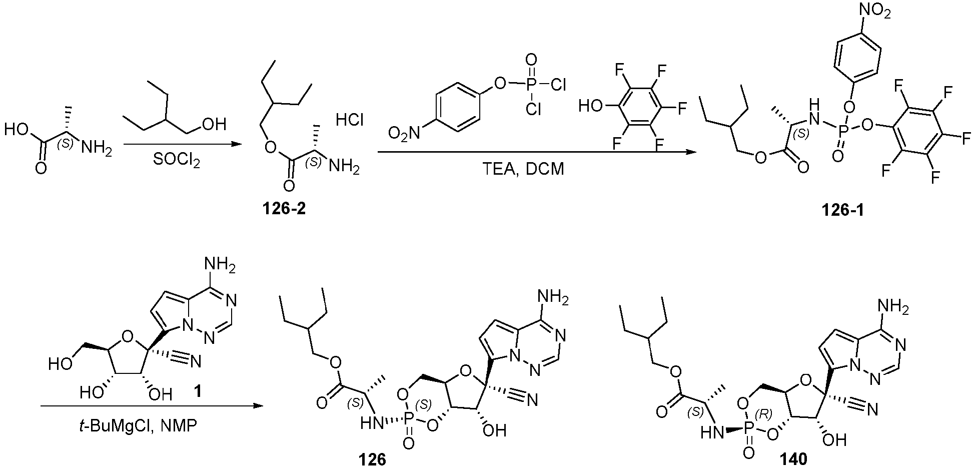

### Preparation of compound 126-2:

To a stirred mixture of L-alanine (10 g, 112.241 mmol, 1 equiv.) in 2-ethylbutanol (100 mL) was added SOCl₂ (26.70 g, 224.482 mmol, 2 equiv.) at 0 °C under Ar atmosphere. The mixture was stirred at 90 °C for 15 h. Major desired product was detected on TLC. The solvent was removed under reduced pressure, the residue was co-evaporated with toluene (4 x 100 mL) and triturated with Et₂O (100 mL) to give compound 126-2 (11.5 g, 55.0 mmol, 48.86% yield) as a white solid.

### Preparation of compound 126-1:

To a stirred mixture of 4-nitrophenyl chlorophosphonochloridate (610.30 mg, 2.384 mmol, 1 equiv.) and compound 126-2 (500 mg, 2.384 mmol, 1 equiv.) in DCM (10 mL) was added TEA (241.26 mg, 2.384 mmol, 1 equiv.) at 0 °C under Ar atmosphere. The mixture was stirred at 20 °C for 2 h. Then to the mixture were added pentafluorophenol (438.86 mg, 2.384 mmol, 1 equiv.) and TEA (241.26 mg, 2.384 mmol, 1 equiv.) at 0 °C. The mixture was stirred at 20 °C for 2 h. 70% desired product was detected by LCMS. Et₂O (15 mL) was added to the reaction mixture, filtered, the filtrate was concentrated under reduced pressure to afford compound 126-1 (834.87 mg) as a crude product which was used in next step directly without purification.

### Preparation of compound 126 and compound 140:

To a stirred mixture of (2R,3R,4S,5R)-2-{4-aminopyrrolo[2,1-f][1,2,4]triazin-7-yl}-3,4-dihydroxy-5-(hydroxymethyl)oxola ne-2-carbonitrile (300 mg, 1.030 mmol, 1.00 equiv.) in NMP (9 mL) was added tert-butylmagnesium chloride (1.29 mL, 2.575 mmol, 2.5 equiv.) at -10 °C under Ar atmosphere. The mixture was stirred at -10 °C for 30 min. Then a solution of compound 126-1 (834.87 mg, 1.545 mmol, 1.5 equiv.) in NMP (9 mL) was added to the above mixture. The resulting mixture was stirred at 0 °C for 15 h. 14% desired product was detected by LCMS. The reaction was quenched by saturated NH₄Cl and diluted with EA (100 mL), the organic layer was washed with water (2 x 50 mL), NaCl (2 x 50 mL), dried over anhydrous Na2SO4, filtered and the filtrate was concentrated under reduced pressure. The residue was purified by silica gel column chromatography, eluted with MeOH in DCM (0 ~ 8%) to give a crude product. The crude product was purified by prep-HPLC used the following condition: Column: XBridge Prep C18 OBD Column, 19 x 150 mm, 5 µm; Mobile Phase A: 10 mM aq. NH₄HCO₃, Mobile Phase B: ACN; Flow rate: 20 mL / min; Gradient: 30% B to 50% B in 5.5 min, Detector: 254 / 210 nm; RT1: 4.8 min to give compound 140 (14.1 mg, 0.028 mmol, 2.81% yield) as a white solid and RT2: 5.2 min to give compound 126 (4.1 mg, 0.008 mmol, 0.76% yield) as a white solid. Confirmed by LCMS, H NMR, P NMR.

Compound 126: ¹H NMR: (400 MHz, CD₃OD) δ 7.82 (s, 1H), 6.90-6.84 (m, 2H), 4.89-4.88 (m, 1H), 4.71-4.69 (m, 2H), 4.69-4.59 (m, 2H), 4.12-4.04 (m, 2H), 3.99-3.95 (m, 1H), 1.56-1.51 (m, 1H), 1.44-1.41 (m, 6H), 1.39-1.37 (m, 1H), 0.94-0.87 (m, 6H).

Compound 140: ¹H NMR: (400 MHz, CD₃OD) δ 7.82 (s, 1H), 6.91-6.84 (m, 2H), 4.89-4.88 (m, 1H), 4.72-4.68 (m, 2H), 4.66-4.60 (m, 1H), 4.58-4.55 (m, 1H), 4.07-4.05 (m, 2H), 3.89-3.85 (m, 1H), 1.52-1.51 (m, 1H), 1.38-1.28 (m, 7H), 0.93-0.82 (m, 6H).

### Preparation Example 7: preparation of compounds 143 and 144

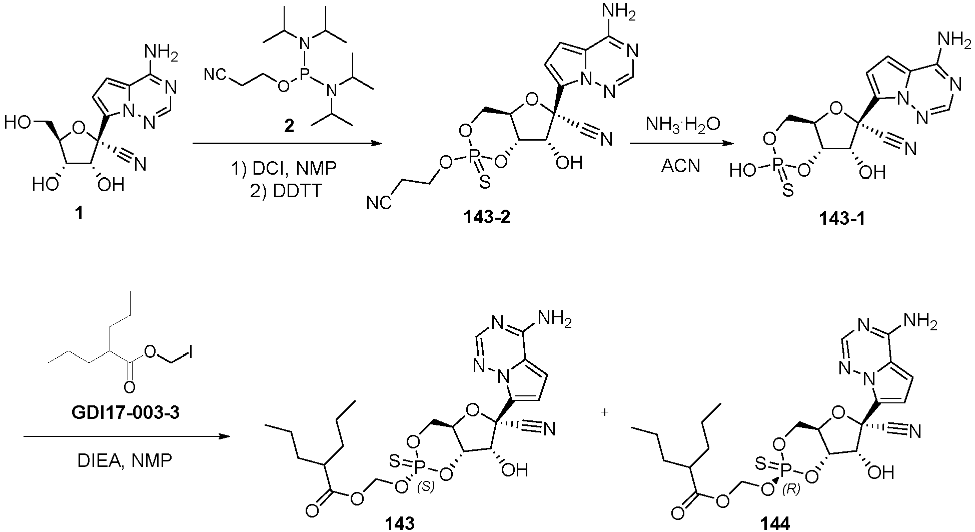

### Preparation of compound 143-2:

To a stirred mixture of compound 1 (1 g, 3.433 mmol, 1.00 equiv.) in NMP (35 mL) were added 1H-imidazole-4,5-dicarbonitrile (0.81 g, 6.866 mmol, 2 equiv.) and a solution of compound 2 (1.45 g, 4.806 mmol, 1.4 equiv.) in ACN (5 mL) at room temperature under Ar atmosphere. The mixture was stirred at room temperature for 1 h. Then (*E*)-*N*,*N*-dimethyl-*N*'-(5-sulfanylidene-1,2,4-dithiazol-3-yl)methanimidamide (3.52 g, 17.165 mmol, 5 equiv.) was added to the above mixture. The mixture was stirred at the same temperature for 30 min. 55% product was detected by LCMS. The solvent was removed under reduced pressure, the residue was purified by RP-flash chromatography, eluted with 0-50% MeCN in water to give compound 143-2 (650 mg, 30.48% yield) as a white solid.

### Preparation of compound 143-1:

To a stirred mixture of compound 143-2 (600 mg, 1.421 mmol, 1 equiv.) in ACN (6 mL) was added NH₃.H₂O (6.00 mL) at 0 °C under Ar atmosphere. The mixture was stirred at room temperature for 1 h. Major desired product was detected by LCMS. The mixture was concentrated under reduced pressure. The residue was purified by RP-flash chromatography, eluted with 0-50% MeCN in 5 mM aq. NH₄HCO₃ to give compound 143-1 (300 mg, 0.813 mmol, 57.31% yield) as a white solid. Confirmed by LC-MS, H NMR and P NMR.

### Preparation of compound 143 and compound 144:

A solution of compound 143-1 (80 mg, 0.217 mmol, 1 equiv.) and molecular sieves (12 mg) in NMP (1.4 mL) was stirred for 5 min at room temperature under Ar atmosphere. To the above mixture was added DIEA (84.00 mg, 0.651 mmol, 3 equiv.) in NMP (0.3 mL) and compound 003-3 (184.66 mg, 0.651 mmol, 3 equiv.) in NMP (0.3 mL) dropwsie over 5 min at 0 °C. The resulting mixture was stirred at room temperature for 2 h. 42.9% desired product was detected by LCMS. The reaction mixture was concentrated under reduced pressure. The residue was purified by *prep-*HPLC with the following condition: Column: XBridge Shield RP18 OBD Column, 19*150 mm, 5µm; Mobile Phase A: Water (10 mM ammonium acetate), Mobile Phase B: ACN; Flow rate: 20 mL/min; Gradient: 40% B to 60% B in 6.5 min, Wave Length: 254/210 nm; RT1: 5.2 min to afford compound 143 (5 mg, 0.010 mmol, 4.39% yield) as a white solid. And RT2: 6.5 min to afford compound 144 (8 mg, 0.015 mmol, 7.04% yield) as a white solid. Confirmed by SFC-MS, H NMR), P NMR.

Compound 143: ¹H NMR: (300 MHz, CD₃OD) δ 7.88 (s, 1H), 6.92-6.86 (m, 2H), 5.57-5.41 (m, 2H), 4.76-4.56 (m, 5H), 2.32-2.26 (m, 1H), 1.54-1.15 (m, 8H), 0.90-0.73 (m, 6H).

Compound 144: ¹H NMR: (400 MHz, CD₃OD) δ 7.88 (s, 1H), 6.91-6.87 (m, 2H), 5.56-5.49 (m, 2H), 4.96-4.80 (m, 3H), 4.69-4.64 (m, 2H), 2.48-2.46 (m, 1H), 1.64-1.61 (m, 2H), 1.50-1.43 (m, 2H), 1.37-1.31 (m, 4H), 0.94-0.91 (m, 6H).

According to the invention, additional exemplary compounds were synthesized with reference to the methods in Preparation Example 7 above and are shown in Table 6 below:

**Table 6**

| No. | Structural Formula | Structural Characterization |
|---|---|---|
| 149 | 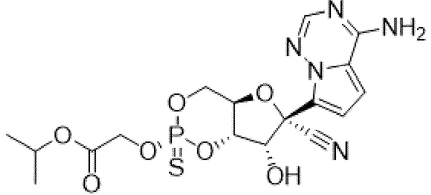 | ¹H NMR: (300 MHz, CD₃OD) |
| | | δ 7.91 (s, 1H), 6.91-6.85 (m, 2H), 5.08-5.01 (m, 1H), 4.99-4.89 (m, 2H), 4.78-4.71 (m, 1H), 4.69-4.60 (m, 3H), 4.57-4.50 (m, 1H), 1.28-1.18 (m, 6H). |
| 150 | 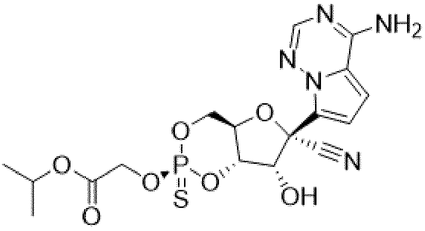 | ¹H NMR: (300 MHz, CD₃OD) δ 7.89 (s, 1H), 6.91-6.86 (m, 2H), 5.14-5.08 (m, 1H), 4.90-4.88 (m, 2H), 4.82-4.65 (m, 4H), 4.54-4.45 (m, 1H), 1.30 (d, J = 6.0 Hz, 6H). |

### Preparation Example 8: preparation of compounds 156 and 157

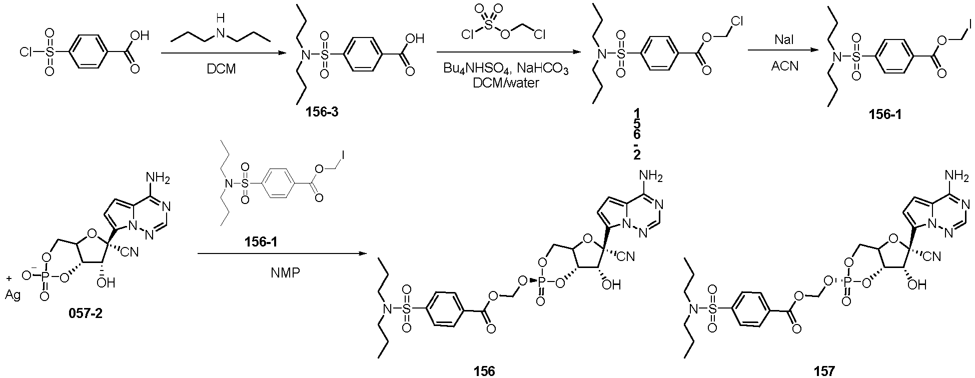

### Preparation of compound 156-3:

To a stirred solution of 4-(chlorosulfonyl)benzoic acid (10 g, 45.454 mmol, 1 equiv.) in DCM (150 mL) was added a solution of dipropylamine (13.77 g, 136.4 mmol, 3 equiv.) in DCM (30 mL) dropwise at 0 °C under Ar atmosphere, the resulting mixture was stirred for 18 h at 25 °C. 95% desired product could be detected by LCMS. The resulting mixture was washed with aq. HCl (1M, 100 mL) and water (2 x 100 mL), dried over anhydrous Na₂SO₄, filtered, and the filtrate was concentrated under reduced pressure to give compound 156-3 (11 g, 38.6 mmol, 84.9% ) as a white solid which was used to next step directly without purification. Confirmed by LCMS, H NMR.

### Preparation of compound 156-2:

To a stirred mixture of compound 156-3 (2.5 g, 8.761 mmol, 1 equiv.), Bu₄NHSO₄ (0.15 g, 0.438 mmol, 0.05 equiv.) and NaHCO₃ (2.32 g, 21.902 mmol, 2.5 equiv.) in DCM (38 mL) and H₂O (38 mL) was added a solution of chloromethyl sulfurochloridate (1.73 g, 10.513 mmol, 1.2 equiv.) in DCM (38 mL) at 0 °C under Ar atmosphere, the mixture was stirred at room temperature for 18 h. Major desired product was detected by LCMS. The reaction mixture was diluted with DCM (100 mL), washed with sat. NaHCO₃ (2 x 50 mL), brine (sat., 50 mL), dried over anhydrous Na₂SO₄. After filtration, the filtrate was concentrated under reduced pressure. The residue was purified by silica gel column chromatography, eluted with (0-18%) EA in PE to afford compound 156-2 (2.3 g, 6.91 mmol, 78.64%) as a white solid. Confirmed by LCMS, H NMR.

### Preparation of compound 156-1:

To a stirred mixture of compound 156-2 (1.8 g, 5.392 mmol, 1 equiv.) in ACN (8 mL) was added NaI (2.42 g, 16.176 mmol, 3 equiv.) at room temperature under Ar atmosphere, the resulting mixture was stirred at 60 °C for 3 h. Major desired product was detected by LCMS. The reaction mixture was diluted with DCM (100 mL), washed with aq. Na₂S₂O₃ (sat., 2 x 50 mL), dried over anhydrous MgSO4, filtered, the filtrate was concentrated under reduced pressure to give compound 156-1 (2.2 g, 5.17 mmol, 95.94%) as a white solid. Confirmed by LCMS, H NMR.

### Preparation of compound 156 and compound 157:

To a stirred mixture of compound 003-1 (300 mg, 0.652 mmol, 1 equiv.) in NMP (6 mL) was added a solution of compound 156-1 (831.90 mg, 1.956 mmol, 3 equiv.) in NMP (6 mL) at 0 °C under Ar atmosphere, the resulting mixture was stirred at room temperature for 3 h. 43% desired product was detected by LCMS. The mixture was purified by RP-flash chromatography, eluted with 0-80% ACN in with the 5 mM aq. NH₄HCO₃ to give a racemic mixture (220 mg, 0.338 mmol, 46.67%) as a white solid. The racemic mixture was separated by prep-HPLC used the following condition: Column: XBridge Prep C18 OBD Column, 19 x 150 mm, 5 µm; Mobile Phase A: 10 mM aq. NH₄HCO₃, Mobile Phase B: ACN; Flow rate: 20 mL / min; Gradient: 45% B to 60% B in 6 min, Detector: UV 254 / 210 nm; RT1: 5.55 min to give compound 156 (50 mg, 22.73% yield) as a white solid; and RT2: 6.17 min to give compound 157 (41 mg, 18.64% yield) as a white solid. Confirmed by LCMS, H NMR, P NMR.

Compound 156: ¹H NMR: (300 MHz, CD₃OD) δ 8.25-8.09 (m, 2H), 7.97-7.88 (m, 2H), 3.16-3.02 (m, 4H), 1.61-1.48 (m, 4H), 0.90-0.84 (m, 6H).

Compound 157: ¹H NMR: (300 MHz, CD₃OD) δ 8.32-8.28 (m, 2H), 7.98-7.95 (m, 2H), 7.88 (s, 1H), 6.90-6.86 (m, 2H), 6.03-6.02 (m, 1H), 5.98-5.95 (m, 1H), 4.92-4.89 (m, 2H), 4.85-4.73 (m, 2H), 4.58-4.52 (m, 1H), 3.15-3.10 (m, 4H), 1.62-1.49 (m, 4H), 0.90-0.85 (m, 6H).

### Preparation Example 9: preparation of compounds 208 and 209

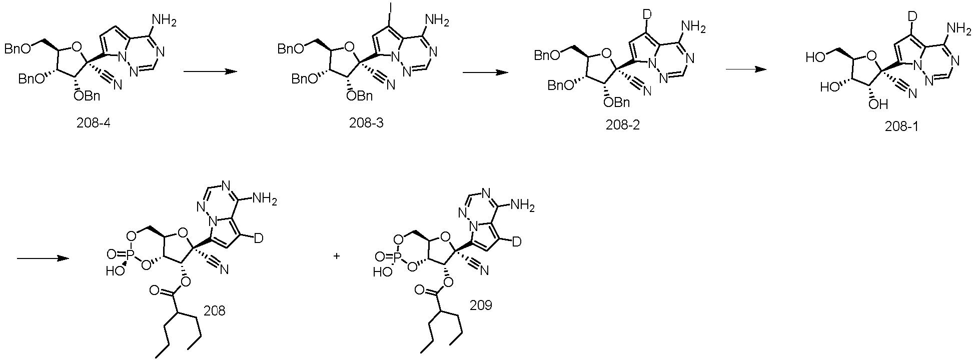

### Synthesis of compound 208-3:

Compound 208-4 (2 g, 1 equiv.) was dissolved in DMF (20 mL), into which iodine (2 equiv.) was added in portions, and the mixture was reacted at room temperature overnight. TLC/LCMS results indicated there were 80% product with a small amount of starting material remaining. The reaction solution was added to the mixed solution of sodium sulfite and sodium carbonate. The mixture was extracted with EA, and the organic phase was separated. The organic phase was washed with water and saturated brine successively, dried over anhydrous sodium sulfate, and separated by silica gel column chromatography to afford pure product 208-3 (1.7 g) as a white solid. There was also 600 mg of crude product with 70% purity. The yield is 81%.

### Synthesis of compound 208-2:

Compound 208-3 (200mg, 1equiv.) was added to a mixed solution of dry THF (15mL) and heavy water (3mL). The solution was concentrated, and then re-dissolved with the same volume of THF and heavy water, and then Pd(dppf)Cl₂-DCM (0.1equiv.) and TMEDA (0.2equiv.) were added successively and the mixture was stirred for 10min, and then NaBD₄ (5equiv.) was added in batches. After 2 hours of reaction at room temperature, tLC indicated that the starting materials was reacted completely. The reaction solution was added to water, extracted with EA, and the organic phase was separated out. The organic phase was washed with saturated brine, dried over anhydrous sodium sulfate, and separated by silica gel column chromatography to obtain the product compound 208-2 (130 mg) with a yield of 80%.

### Synthesis of compound 208-1:

Compound 208-2 (130 mg) was dissolved in DCM (15 mL), BCl₃ (1M in DCM, 3.5 equiv.) was added dropwise at -60 °C. After the addition was completed, and the mixture was stirred at -40 °C for 3 hours. TLC indicated that the reaction was complete, and LCMS indicated mainly the target product signal. Methanol (0.15 mL) was added to the reaction solution, followed by TEA until the pH of the reaction solution was 7-8. The reaction liquid was concentrated and purified by silica gel column chromatography to obtain compound 208-1.

### Synthesis of compounds 208 and 209:

In a way similar with Preparation Example 4, except that compound 094 was replaced with compound 208-1, a crude product was obtained, which was purified by preparative HPLC using the following conditions: Column: XBridge Prep C18 OBD Column, 19 x 150 mm, 5 µm; Mobile Phase A: 10 mM aq. HCOONH₄, Mobile Phase B: ACN; Flow rate: 20 mL / min; Gradient: 25% B to 40% B in 5.5 min, Detector: UV 254 / 210 nm; RT1: 5.2 min to give compound 209 (1.0 mg, 0.002 mmol, 0.92% yield) as a white solid; and RT2: 5.3 min to give compound 208 (2.9 mg, 0.006 mmol, 2.67% yield) as a white solid. Confirmed by ¹H NMR.
compound 208: ¹H NMR: (300 MHz, D₂O)
δ 7.82 (d, J = 3.3 Hz, 1H), 6.86-6.82 (m, 1H), 5.89 (d, J = 4.2 Hz, 1H), 4.41-4.35 (m, 3H), 4.20-4.14 (m, 1H), 2.70-2.60 (m, 1H), 1.95-1.75 (m, 2H), 1.62-1.50 (m, 2H), 1.47-1.42 (m, 4H), 0.84-0.79 (m, 6H).
compound 209: ¹H NMR: (300 MHz, D₂O)
δ 7.92 (s, 1H), 6.94 (d, J = 3.0 Hz, 1H), 5.84-5.80 (m, 1H), 5.58-5.53 (m, 1H), 4.45-4.27 (m, 3H), 2.06-1.96 (m, 1H), 0.96-0.84 (m, 6H), 0.63-0.53 (m, 8H).

### Preparation Example 10: preparation of compounds 236 and 237

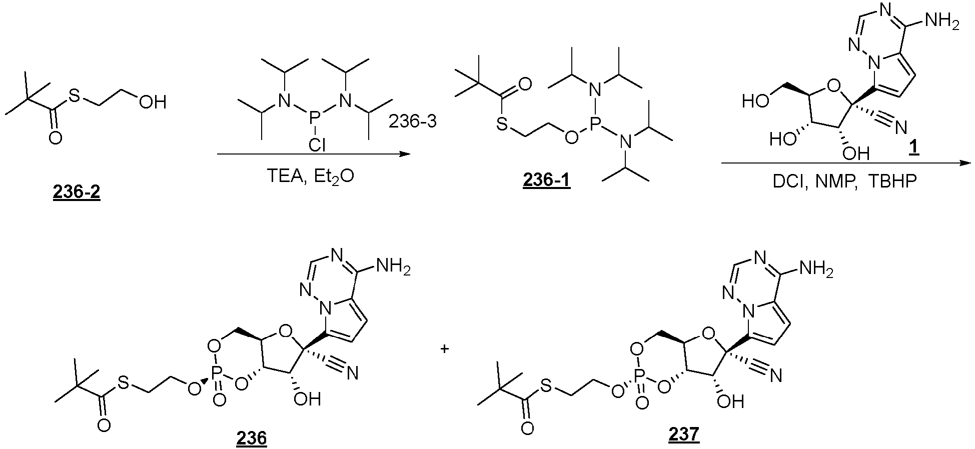

### Synthesis of S-(2-((diisopropylamino)phosphinyl)oxy)ethyl) 2,2-dimethylthiopropanoate (Compound 236-1)

To a stirred solution of 1-chloro-N,N,N',N'-tetraisopropylphosphanediamine (1.5 g, 5.622 mmol, 1.00 equiv.) in diethyl ether (5.5 mL) at -40 °C under Ar gas was added S-(2-hydroxyethyl) 2,2-dimethylpropanethioic acid (0.91 g, 5.622 mmol, 1 equiv.) and TEA (0.63 g, 6.184 mmol, 1.1 equiv.) in Et₂O (0.5 mL) and the resulting mixture was stirred at room temperature for 3 h. 80% of the product was detected by P NMR. The mixture was filtered, and the filtrate was concentrated under reduced pressure. The crude product was used directly in the next step.

### Synthesis of sodium S-(2-((((2R,4aR,6R,7R,7aS)-6-(4-aminopyrrolo[2,1-f][1,2,4]triazin-7-yl)-6-cyano-7-hydroxy-2-oxot etrahydro-4H-furo[3,2-d][1,3,2]dioxaphosphoryl-2-yloxy)ethyl) 2,2-dimethylpropyl hydrogen sulfate (compound 236/237)

To compound 1 (300 mg, 1.030 mmol, 1 equiv.) was added with NMP (1.5 mL), DCI (304.10 mg, 2.575 mmol, 2.5 equiv.) at 0 °C under Ar gas; after adding dropwise a solution of 236-1 (525.66 mg, 1.339 mmol, 1.3 equiv.) in NMP (1.5 mL), the mixture was stirred at room temperature for 2 h. Then TBHP (0.94 mL, 5.150 mmol, 5 equiv.) was then added to the above mixture at 0°C. The mixture was stirred at room temperature for 30 min. 20% of the desired mass was detected by LCMS. The mixture was purified by RP-flash chromatography under the following conditions: column, C18 silica gel; mobile phase, MeCN in water, 0% to 35% gradient, 20 min; detector, UV 254 / 210 nm, to give the crude product. The crude product was purified by preparative HPLC using the following conditions: column: XBridge Prep C18 OBD column, 19 x 150 mm, 5 µm; mobile phase A: 10 mM CH₃COONH₄, mobile phase B: ACN; flow rate: 25 ml/min; gradient: from 30% B to 50% B in 6 min, detector: 254 / 210 nm; RT1 : 5.3 min to give Compound 236 (69.3 mg, 0.139 mmol, 13.53%) as a white solid, RT2: 6.2 min to give Compound 237 (14.3 mg) , 0.029 mmol, 2.79%) as a white solid.
236 ¹H-NMR: (300 MHz, CD₃CN)
δ 7.90 (d, J = 3.0 Hz, 1H), 6.84 - 6.78 (m, 2H), 6.37 (s, 2H), 4.89 - 4.71 (m, 2H), 4.66 - 4.63 (m, 1H), 4.50 - 4.41 (m, 2H), 4.38 - 4.32 (m, 1H), 4.13 - 4.05 (m, 2 H), 3.14 - 3.10 (m, 2H), 1.11 (d, J = 3.0 Hz, 9H).
237 ¹H-NMR: (300 MHz, CD₃CN)
δ 7.94 (d, J = 3.0 Hz, 1H), 6.83 - 6.77 (m, 2H), 6.36 (s, 2H), 4.89 - 4.84 (m, 2H), 4.75 - 4.63 (m, 3H), 4.47 - 4.38 (m, 1H), 4.18 - 4.11 (m, 2H), 3.19 - 3.15 (m, 2H), 1.23 - 1.19 (m, 9H).

### Preparation of compounds 278 and 279:

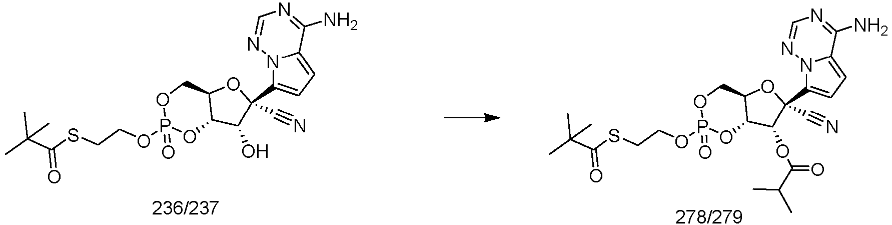

To a solution of compound 236/237 (107 mg, 0.216 mmol, 1 equiv.) in DMF (2.5 mL) was added with isobutyric acid (57.07 mg, 0.648 mmol, 3 equiv.) and DIC (81.75 mg, 0.648 mmol, 3 equiv.) at 0 °C under argon. The resulting mixture was stirred for 15 min at room temperature, added with DMAP (13.19 mg, 0.108 mmol, 0.5 equiv.). After the mixture was stirred for 15 h at room temperature, a major amount of desired compound was detected by LCMS. The mixture was purified by RP-flash chromatography under the following conditions: Column: C18 silica gel; Mobile Phase: MeCN in water, Gradient: 0% B to 70% B in 25 min, Detector: 254 / 210 nm, to afford compound 278/279.

According to the invention, additional exemplary compounds were synthesized with reference to the methods in Preparation Example 10 above and are shown in Table 7 below:

**Table 7**

| No. | Structural Formula | Structural Characterization |
|---|---|---|
| 230 | 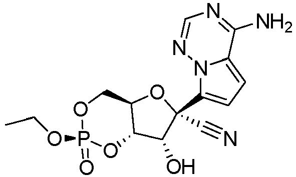 | ¹H NMR: (300MHz, CD₃OD) δ 7.88 (s, 1H), 6.92 - 6.86 (m, 2H), 4.92 (d, J = 4.5 Hz, 1H), 4.85 - 4.63 (m, 2H), 4.57 - 4.49 (m, 1H), 4.45 - 4.38 (m, 1H), 4.25 - 4.15 (m, 2H), 1.39 - 1.34 (m, 3H). |
| 231 | 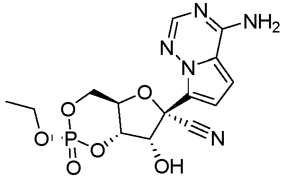 | ¹H NMR: (300MHz, CD₃OD) δ 7.90 (s, 1H), 6.91 - 6.86 (m, 2H), 4.92 (d, J = 4.8 Hz, 1H), 4.85 - 4.74 (m, 2H), 4.68 - 4.59 (m, 1H), 4.54 - 4.45 (m, 1H), 4.31 - 4.21 (m, 2H), 1.41 - 1.36 (m, 3H). |
| 232 | 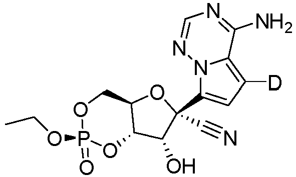 | ¹H NMR: (300 MHz, CD₃CN) δ 7.95 (d, J = 3.0, 1H), 6.82 (d, J = 7.5 Hz, 1H), 6.37 (s, 2H), 4.89 - 4.71 (m, 1H), 4.68 - 4.53(m, 1H), 4.47 - 4.44 (m, 1H), 4.38 - 4.30 (m, 1H), 4.14 - 4.09 (m, 2H), 2.16 (s, 1H), 1.32-1.27 (m, 3H). |
| 233 | 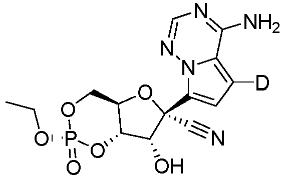 | ¹H NMR: (300 MHz, CD₃CN) δ 7.94 (d, J = 3.0, 1H), 6.82 (s, 1H), 6.35 (s, 2H), 4.82 - 4.70 (m, 2H), 4.66 - 4.56 (m, 3H), 4.46 - 4.38 (m, 1H), 4.23 - 4.13 (m, 2H), 1.36 - 1.27 (m, 3H). |
| 236 | 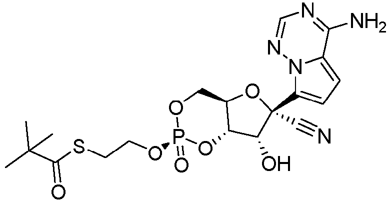 | ¹H NMR: (300 MHz, CD₃CN) δ 7.90 (d, J = 3.0 Hz, 1H), 6.84 - 6.78 (m, 2H), 6.37 (s, 2H), 4.89 - 4.71 (m, 2H), 4.66 - 4.63 (m, 1H), 4.50 - 4.41 (m, 2H), 4.38 - 4.32 (m, 1H), 4.13 - 4.05 (m, 2 H), 3.14 - 3.10 (m, 2H), 1.11 (d, J = 3.0 Hz, 9H). |
| 237 | 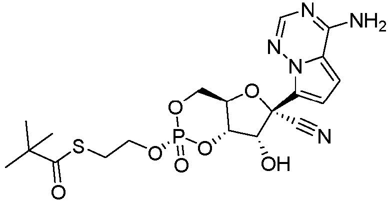 | ¹H NMR: (300 MHz, CD₃CN) δ 7.94 (d, J = 3.0 Hz, 1H), 6.83 - 6.77 (m, 2H), 6.36 (s, 2H), 4.89 - 4.84 (m, 2H), 4.75 - 4.63 (m, 3H), 4.47 - 4.38 (m, 1H), 4.18 - 4.11 (m, 2H), 3.19 - 3.15 (m, 2H), 1.23 - 1.19 (m, 9H). |
| 238 | 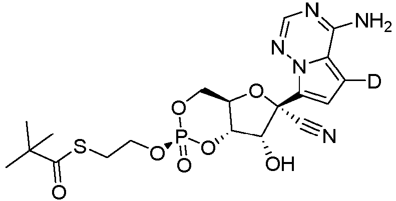 | ¹H NMR: (300 MHz, CD₃CN) δ 7.97 (d, J = 1.8 Hz, 1H), 6.83 (d, J = 3.0 Hz, 1H), 6.39 (s, 2H), 4.90 - 4.89 (m, 1H), 4.74 - 4.66 (m, 1H), 4.63 - 4.50 (m, 2H), 4.41 - 4.36 (m, 1H), 4.13 - 4.05 (m, 2H), 3.14 - 3.10 (m, 2H), 1.22 - 1.11 (m, 9H). |
| 239 | 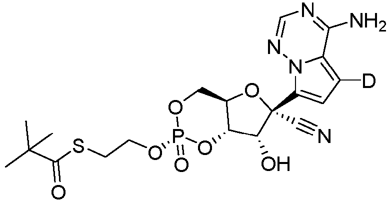 | ¹H NMR: (300 MHz, CD₃CN) δ 7.94 (d, J = 1.8 Hz, 1H), 6.83 - 6.82 (m, 1H), 6.38 (s, 2H), 4.91 - 4.89 (m, 1H), 4.76 - 4.72 (m, 3H), 4.67 - 4.63 (m, 1H), 4.47 - 4.39 (m, 2H), 3.19 - 3.16 (m, 2H), 1.18 - 1.12 (m, 9H). |
| 278 | 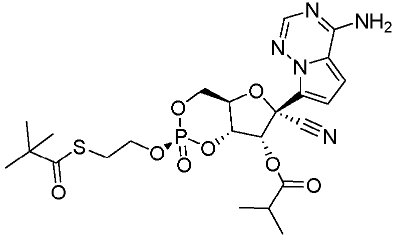 | ¹H NMR: (300 MHz, CD₃CN) δ 7.87 (s, 1H), 6.83 (d, J = 4.5 Hz, 1H), 6.73 (d, J = 4.8 Hz, 1H), 6.36 (s, 2H), 5.94 (d, J = 5.4 Hz, 1H), 4.85 - 4.80 (m, 1H), 4.66 - 4.55 (m, 1H), 4.46 - 4.38 (m, 1H), 4.33 - 4.27 (m, 1H), 4.07 - 4.00 (m, 2H), 3.09 - 3.05 (m, 2H), 2.72 - 2.65 (m, 1H), 1.20 - 1.15 (m, 6H), 1.06 (s, 9H). |
| 279 | 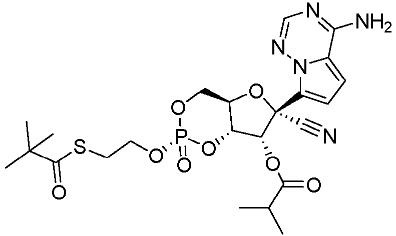 | ¹H NMR: (300 MHz, CD₃CN) δ 7.95 (s, 1H), 6.94 (d, J = 4.8 Hz, 1H), 6.83 (d, J = 4.5 Hz, 1H), 6.45 (s, 2H), 6.06 - 6.05 (m, 1H), 5.09 - 5.04 (m, 1H), 4.80 - 4.66 (m, 2H), 4.53 - 4.47 (m, 1H), 4.21 - 4.14 (m, 2H), 3.21 - 3.17 (m, 2H), 2.85 - 2.76 (m, 1H), 1.32 - 1.28 (m, 6H), 1.26 (s, 9H). |
| 289 | 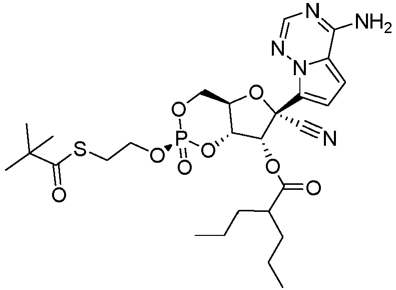 | ¹H NMR: (300 MHz, CD₃CN) δ 7.96 (s, 1H), 6.92 - 6.83 (m, 2H), 6.47 (s, 2H), 6.05 (d, J = 5.1 Hz, 1H), 4.80 - 4.76 (m, 2H), 4.52 - 4.43 (m, 2H), 4.15 - 4.11 (m, 2H), 3.18 - 3.14 (m, 2H), 2.63 - 2.58 (m, 1H), 1.78 - 1.72 (m, 2H), 1.60 - 1.37 (m, 6H), 1.16 (s, 9H), 0.98 - 0.93 (m, 6H). |
| 290 | 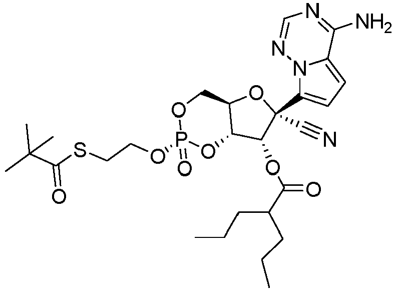 | ¹H NMR: (300 MHz, CD₃CN) δ 7.93 (s, 1H), 6.91 (d, J = 4.5 Hz, 1H), 6.83 (d, J = 4.8 Hz, 1H), 6.43 (s, 2H), 6.06 - 6.04 (m, 1H), 4.97 - 4.92 (m, 1H), 4.78 - 4.71 (m, 1H), 4.68 - 4.66 (m, 1H), 4.54 - 4.48 (m, 1H), 4.20 - 4.13 (m, 2H), 3.21 - 3.16 (m, 2H), 2.62 - 2.58 (m, 1H), 1.79 - 1.73 (m, 2H), 1.61 - 1.57 (m, 2H),1.48 - 1.40 (m, 4H), 1.27 (s, 9H), 0.99 - 0.93 (m, 6H). |

### Preparation Example 11: preparation of compounds 214/215

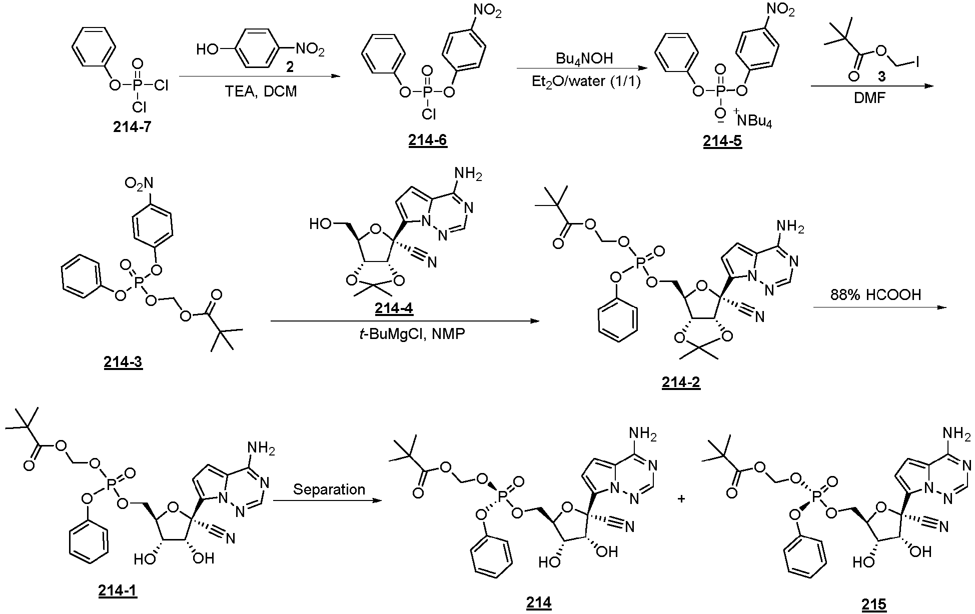

### Synthesis of 4-nitrophenyl phenyl phosphorochloridate (compound 214-6)

To a stirred mixture of compound 214-7 (2 g, 9.480 mmol, 1 equiv.) and compound 2 (1.32 g, 9.480 mmol, 1 equiv.) in DCM (20 mL) was added TEA (1.92 g, 18.960 mmol, 2 equiv.) dropwise at -78°C. The resulting mixture was stirred at -78°C for 1 h and at room temperature for a further for 2 h, 56% of the mixture was the desired product as monitored by LCMS. The resulting mixture was concentrated under reduced pressure. The residue was dissolved in Et₂O (100 mL) and stirred for 30 min. The resulting mixture was filtered, and the filter cake was washed with Et₂O (50 mL). The filtrate was concentrated under reduced pressure to give compound 214-6 (2.8 g, crude). The crude product was used directly in the next step without further purification.

### Synthesis of tetrabutylammonium 4-nitrophenyl phenyl phosphate (compound 214-5)

To a stirred mixture of compound 214-6 (2.8 g, 8.928 mmol, 1 equiv.) in diethyl ether (28 mL) was added with a solution of n-Bu₄NOH (2.32 g, 8.928 mmol, 1 equiv.) in diethyl ether (28 mL). Water (28 mL) was added dropwise at 0 °C. The resulting mixture was stirred for 2 h at room temperature and 56% of the mixture was the desired product as monitored by LCMS. The resulting mixture was concentrated under vacuum. The residue was purified by RP-flash chromatography under the following conditions: column, C18 silica gel; mobile phase, ACN in water (10 mM NH₄HCO₃), 0% to 50% gradient, 30 min; detector, UV 254 nm, to give the compound 215-5 (3.5 g, 73.05%) as a yellow oil. Confirmed by LC-MS.

### Synthesis of methyl ((4-nitrophenoxy)(phenoxy)phosphoryl)oxy)pivalate (compound 214-3)

To a stirred solution of compound 214-5 (1 g, 1.863 mmol, 1 equiv.) in DMF (10 mL) at 0°C was added compound 3 (0.68 g, 2.795 mmol, 1.5 equiv.). The mixture was stirred for 2 h at room temperature, 73% of the mixture was the desired product as monitored by LCMS. The reaction mixture was quenched with a solution of hydrochloric acid (0.01%, 100 mL). The aqueous layer was extracted with ethyl acetate (150 mL). The organic layer was washed with brine (2 x 20 mL) and dried over anhydrous Na₂SO₄. The mixture was concentrated under reduced pressure. The residue was purified by silica gel column chromatography, eluting with PE/EA (0-30%) to give compound 214-3 (436 mg, 57.16%) as a colourless oil. Confirmed by LCMS.

### Synthesis of methyl (((((3aR,4R,6R,6aR)-6-(4-aminopyrrolo[2,1-f][1,2,4]triazin-7-yl)-6-cyano-2,2-dimethyltetrahydrofur an[3,4-d][1,3]dioxy-4-yl)methoxy)(phenoxy)phosphoryl)oxy)pivalate (compound 214-2)

To a stirred solution of compound 214-4 (100 mg, 0.302 mmol, 1.00 equiv.) in NMP (2 mL) was added with tert-butylmagnesium chloride (0.60 mL, 0.604 mmol, 2 equiv.) dropwise at 0° C under argon and the resulting mixture was stirred for 30 min at 0°C under Ar gas. A solution of compound 214-3 (185.31 mg, 0.453 mmol, 1.5 equiv.) in NMP (2 mL) was added dropwise at 0° to the above mixture. The resulting mixture was stirred for a further 2 h at room temperature and 20% of the mixture was the desired product as monitored by LCMS. The resulting mixture was diluted with water (50 mL) and extracted with EA (2 x 50 mL). The organic layer was washed with water (3 x 50 mL) and brine (2 x 25 mL) and dried over anhydrous Na₂SO₄. After filtration, the filtrate was concentrated under reduced pressure. The crude product is used directly in the next step without further purification.

### Synthesis of methyl (((((2R,3S,4R,5R)-5-(4-aminopyrrolo[2,1-f][1,2,4]triazin-7-yl)-5-cyano-3,4-dihydroxytetrahydrofura n-2-yl)methoxy)(phenoxy)phosphoryl)oxy)pivalate (compound 214-1)

A solution of compound 214-2 (200 mg, 0.332 mmol, 1 equiv.) in HCOOH (2 mL, 46.653 mmol, 140.32 eq., 88%) was stirred for 6 h at room temperature under argon and monitored by LCMS, 34 % being the desired product. The resulting mixture was azeotropically boiled with toluene (3x3 mL) and concentrated under reduced pressure. The residue was purified by silica gel column chromatography, eluting with a DCM solution of MeOH (0-10 %) to give compound 214-1 (50 mg, 26.78 %) as a pale yellow solid. It was confirmed by LC-MS.

### Preparation of methyl ((S)-(2R,3S,4R,5R)-5-(4-aminopyrrolo[2,1-f][1,2,4]triazin-7-yl)-5-cyano-3,4-dihydroxytetrahydrofu ran-2-yl)methoxy)(phenoxy)phosphoryl)oxy)pivalate (Compound 214)

Compound 214-1 (55 mg, 0.098 mmol, 1 equiv.) was purified by preparative HPLC under the following conditions (column: Lux 3µ Cellulose-4, 4.6*50mm, 3µm; mobile phase A: Hex (0.1% DEA):EtOH = 70:30; flow rate: 1ml/min; gradient: 0%B to 0%B; injection volume: 5µl mL; RT:3.836) gave compound 214 (11.9 mg; RT:5.589 and a white solid compound 215 (14.1 mg, 25.64%) as a white solid. Confirmed by ¹H NMR.
214 ¹H-NMR
¹H NMR: (300 MHz, CD₃OD)
δ 7.88 - 7.81 (m, 1H), 7.31 (t, J = 7.6 Hz, 2H), 7.24 - 7.11 (m, 3H), 6.91 - 6.82 (m, 2H), 5.67 (ddd, J = 13.2, 7.3, 5.0 Hz, 2H), 4.56 - 4.36 (m, 3H), 4.18 (t, J = 5.4 Hz, 1H), 3.31 (s, 4H), 1.17 - 1.11 (m, 8H).
215 ¹H-NMR
¹H NMR: (300 MHz, CD₃OD)
δ 7.84 (s, 1H), 7.30 (s, 5H), 7.15 (d, J = 8.1 Hz, 2H), 7.05 (d, J = 8.1 Hz, 2H), 6.90 - 6.84 (m, 2H), 5.71 - 5.63 (m, 2H), 5.12 - 5.04 (m, 3H), 4.49 - 4.31 (m, 4H), 4.20 - 4.16 (m, 1H), 3.08 - 3.04 (m, 1H), 2.94 - 2.91 (m, 1H), 2.39 - 2.37 (m, 1H), 1.79 - 1.21 (m, 17H), 0.84 (t, J = 7.2 Hz, 6H).

According to the invention, additional exemplary compounds were synthesized with reference to the methods in Preparation Example 11 above and are shown in Table 8 below:

**Table 8**

| No. | Structural Formula | Structural Characterization |
|---|---|---|
| 214 | 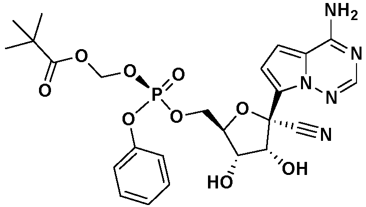 | ¹H NMR: (300 MHz, CD₃OD) δ 7.88 - 7.81 (m, 1H), 7.31 (t, J = 7.6 Hz, 2H), 7.24 - 7.11 (m, 3H), 6.91 - 6.82 (m, 2H), 5.67 (ddd, J = 13.2, 7.3, 5.0 Hz, 2H), 4.56 - 4.36 (m, 3H), 4.18 (t, J = 5.4 Hz, 1H), 3.31 (s, 4H), 1.17 - 1.11 (m, 8H). |
| 215 | 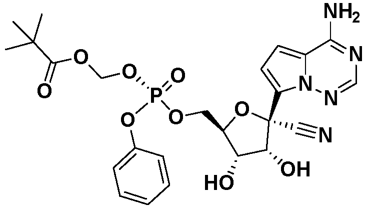 | ¹H NMR: (300 MHz, CD₃OD) δ 7.83 (s, 1H), 7.30 - 7.25 (m, 2H), 6.88 - 6.85(m, 2H), 7.19 - 7.14 (m, 3H), 5.70 - 5.65 (m, 2H), 4.85 (s, 1H), 4.54 - 4.38 (m, 3H), 4.17 - 4.14 (m, 1H), 1.28 - 1.16 (m, 9H). |
| 216 | 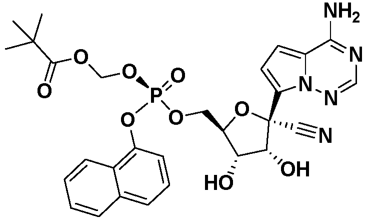 | ¹H NMR: (300 MHz, CD₃OD) δ 8.06 (d, J = 7.5 Hz, 1H), 7.88 (d, J = 7.8 Hz, 1H), 7.81 (s, 1H), 7.71 (d, J = 6.9 Hz, 1H), 7.55 - 7.47 (m, 2H), 7.36 - 7.31 (m, 2H), 6.85 - 6.78 (m, 2H), 5.77 - 5.66 (m, 2H), 4.80 - 4.78 (m, 1H), 4.56 - 4.45 (m, 2H), 4.41 (s, 1H), 4.22 - 4.19 (m, 1H), 1.33 - 1.25 (m, 1H), 1.08 (s, 9H). |
| 217 | 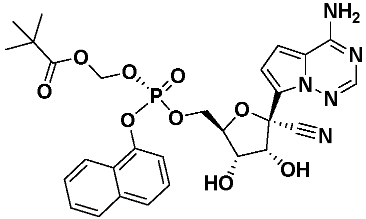 | ¹H NMR: (300 MHz, CD₃OD) δ 8.05 (d, J = 8.1 Hz, 1H), 7.86 (d, J = 8.1 Hz, 1H), 7.78 (s, 1H), 7.71 - 7.68 (m, 1H), 7.53 - 7.47 (m, 2H), 7.44 - 7.28 (m, 2H), 6.84 (s, 2H), 5.73 (d, J = 13.8 Hz, 2H), 4.77 - 4.76 (m, 1H), 4.60 - 4.54 (m, 1H), 4.50 - 4.40 (m, 2H), 4.16 - 4.13 (m, 1H), 1.10 (s, 9H). |
| 218 | 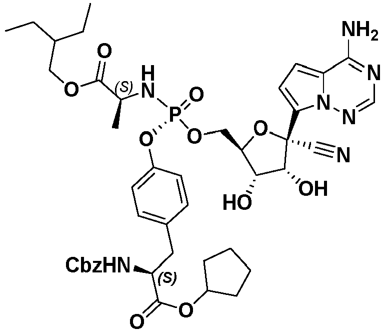 | ¹H NMR: (300 MHz, CD₃OD) δ 7.83 (s, 1H), 7.30 (s, 5H), 7.13 (d, J = 8.4 Hz, 2H), 7.04 (d, J = 8.4 Hz, 2H), 6.91 - 6.87 (m, 2H), 5.12 - 5.03 (m, 3H), 4.78 (d, J = 5.7 Hz, 1H), 4.44 - 4.16 (m, 5H), 4.02 - 3.81 (m, 3H), 3.10 - 2.88 (m, 2H), 1.81 - 1.44 (m, 9H), 1.38 - 1.22 (m, 7H), 0.86 (t, J = 7.5 Hz, 6H). |
| 219 | 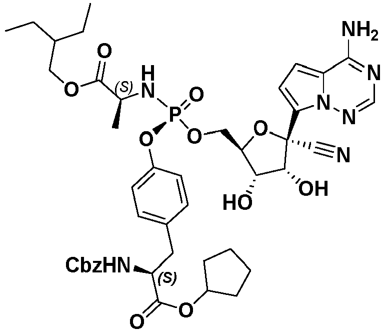 | ¹H NMR: (300 MHz, CD₃OD) δ 7.85 (s, 1H), 7.30 (s, 5H), 7.16 - 7.08 (m, 4H), 6.91 - 6.85 (m, 2H), 5.12 - 5.06 (m, 3H), 4.79 (d, J = 5.1 Hz, 1H), 4.37 - 4.27 (m, 4H), 4.18 - 4.14 (m, 1H), 4.05 - 4.00 (m, 1H), 3.95 - 3.85 (m, 2H), 3.06 - 3.04 (m, 1H), 2.94 - 2.89 (m, 1H), 1.81 - 1.58 (m, 8H), 1.47 - 1.41 (m, 1H), 1.36 - 1.27 (m, 7H), 0.85 (t, J = 7.5 Hz, 6H). |
| 220 | 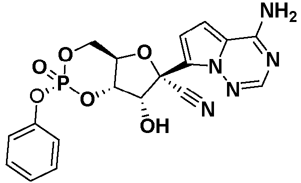 | ¹H NMR: (300 MHz, CD₃OD) δ 7.86 (s, 1H), 7.43 - 7.38 (m, 2H), 7.28 - 7.22 (m, 3H), 6.91 (s, 2H), 4.95 - 4.85 (m, 3 H), 4.65 - 4.60 (m, 2H). |
| 221 | 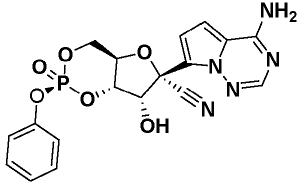 | ¹H NMR: (300 MHz, CD₃OD) δ 7.90 (s, 1H), 7.45 - 7.40 (m, 2H), 7.33 - 7.25 (m, 3H), 6.91 - 6.87 (m, 2H), 4.97 - 4.95 (m, 3 H), 4.74 - 4.56 (m, 2H). |
| 222 | 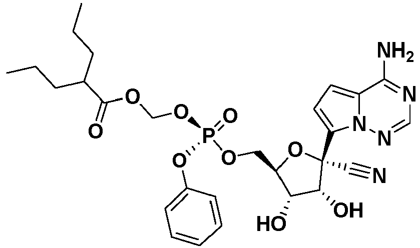 | ¹H NMR: (300MHz, CD₃OD) δ 7.85 (s, 1H), 7.34 - 7.29 (m, 2H), 7.21 - 7.14 (m, 3H), 6.90 - 6.85 (m, 2H), 5.72 - 5.62 (m, 2H), 4.53 - 4.39 (m, 3H), 4.18 (t, J = 5.1 Hz, 1H), 2.40 - 2.35 (m, 1H), 1.61 - 1.45 (m, 2H), 1.45 - 1.34 (m, 2H), 1.30 - 1.18 (m, 4H), 0.84 (t, J = 7.2 Hz, 6H). |
| 223 | 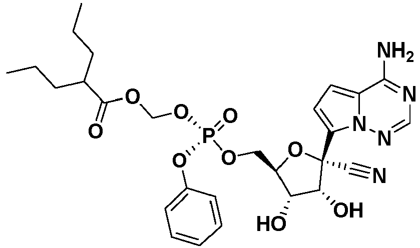 | ¹H NMR: (300MHz, CD₃OD) δ 7.83 (d, J = 1.8 Hz, 1H), 7.30 - 7.25 (m, 2H), 7.19 - 7.14 (m, 3H), 6.88 (s, 2H), 5.69 (d, J = 13.5 Hz, 2H), 4.54 - 4.39 (m, 3H), 4.16 (t, J = 5.4 Hz, 1H), 2.44 - 2.35 (m, 1H), 1.62 - 1.50 (m, 2H), 1.47 - 1.35 (m, 2H), 1.32 - 1.20 (m, 4H), 0.85 (t, J = 7.2 Hz, 6H). |
| 224 | 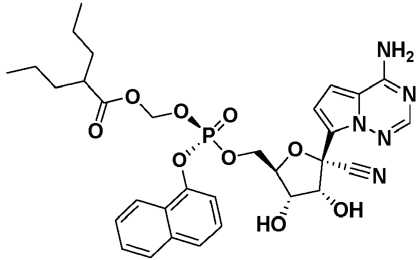 | ¹H NMR: (300 MHz, CD₃OD) δ 8.05 (m, 1H), 7.87 (d, J = 7.5 Hz, 1H), 7.81 (s, 1H), 7.71 (d, J = 7.2 Hz, 1H), 7.53 - 7.50 (m, 2H), 7.39 - 7.34 (m, 2H), 6.84 (d, J = 4.2 Hz, 1H), 6.77 (d, J = 4.5 Hz, 1H), 5.78 - 5.67 (m, 2H), 4.81 - 4.79 (m, 1H), 4.56 - 4.49 (m, 2H), 4.41 (s, 1H), 4.22 - 4.19 (m, 1H), 2.30 - 2.28 (m, 1H), 1.51 - 1.37 (m, 2H), 1.35 - 1.28 (m, 2H), 1.23 - 1.14 (m, 4H), 0.81 - 0.75 (m, 6H). |
| 225 | 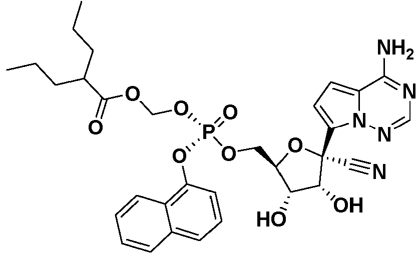 | ¹H NMR: (300 MHz, CD₃OD) δ 8.04 (m, 1H), 7.85 (d, J = 8.1 Hz, 1H), 7.78 (s, 1H), 7.69 (d, J = 8.1 Hz, 1H), 7.53 - 7.37 (m, 3H), 7.33 - 7.28 (m, 1H), 6.86 - 6.82 (m, 2H), 5.77 - 5.72 (m, 2H), 4.78 - 4.76 (m, 1H), 4.56 - 4.41 (m, 3H), 4.16 - 4.13 (m, 1H), 2.34 - 2.31 (m, 1H), 1.54 - 1.47 (m, 2H), 1.44 - 1.37 (m, 2H), 1.35 - 1.22 (m, 4H), 0.81 - 0.77 (m, 6H). |
| 226 | 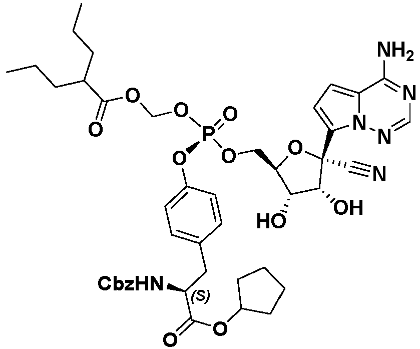 | ¹H NMR: (300 MHz, CD₃OD) δ 7.82 (s, 1H), 7.29 (s, 5H), 7.08 (q, J = 8.4 Hz, 4H), 6.87 (s, 2H), 5.68 (d, J = 13.5 Hz, 2H), 5.11 - 5.03 (m, 3H), 4.49 - 4.17 (m, 4H), 4.15 (s, 1H), 3.31 - 2.85 (m, 2H), 2.42 - 2.38 (m, 1H), 1.80 - 1.20 (m, 17H), 0.87 - 0.83 (m, 6H). |
| 227 | 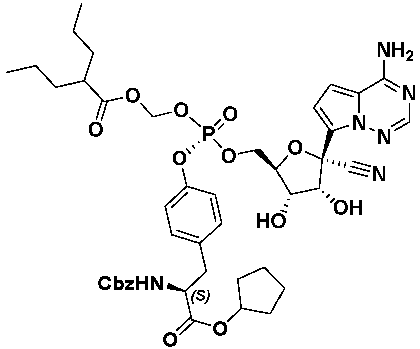 | ¹H NMR: (300 MHz, CD₃OD) δ 7.84 (s, 1H), 7.30 (s, 5H), 7.15 (d, J = 8.1 Hz, 2H), 7.05 (d, J = 8.1 Hz, 2H), 6.90 - 6.84 (m, 2H), 5.71 - 5.63 (m, 2H), 5.12 - 5.04 (m, 3H), 4.49 - 4.31 (m, 4H), 4.20 - 4.16 (m, 1H), 3.07 (dd, J = 14.0, 6.2 Hz, 1H), 2.97 - 2.84 (m, 1H), 2.46 - 2.29 (m, 1H), 1.95 - 1.10 (m, 17H), 0.84 (t, J = 7.2 Hz, 6H). |

### Preparation Example 11: preparation of compounds 155

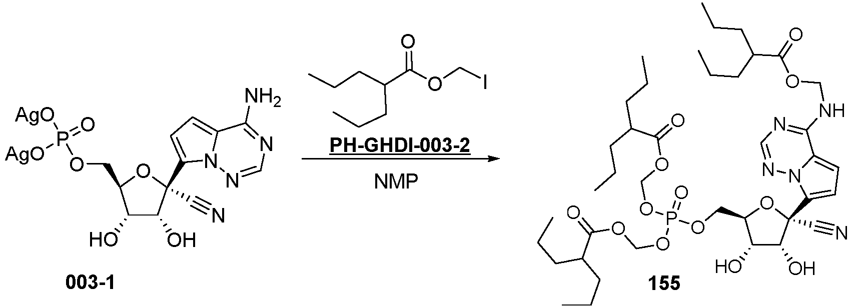

A mixture of compound 003-1 (100 mg, 0.17 mmol, 1 equiv) and molecular sieves (4A) (40 mg) in NMP (2 mL) was stirred for 5 min at 25 °C under Ar atmosphere. To the above mixture was added a solution of PH-GHDI-003-2 (500 mg, 1.7 mmol, 10 equiv) in NMP (0.5 mL) over 5 min at 0 °C. The resulting mixture was stirred for additional 2 h at 25 °C. The reaction was quenched with water (0.5 mL), extracted with EA (150 mL), the organic layer was washed with aq. NaHCO₃ (sat., 50 mL) and brine (sat., 5 x 20 mL), dried over anhydrous Na₂SO₄. After filtration, the filtrate was concentrated under reduced pressure. The residue was purified by prep-HPLC with the following condition: Column: SunFire Prep C18 OBD Column, 19*150 mm, 5µm; Mobile Phase A: Water (0.1% FA), Mobile Phase B: ACN; Flow rate: 20 mL/min; Gradient: 50% B to 70% B in 5.5 min, 70% B; Wave Length: 254/210 nm; RT: 5.2 min to afford 155-0 (10 mg, 61.33% yield) as an off-white semi-solid.

**Table 9**

| | | |
|---|---|---|
| 155 | 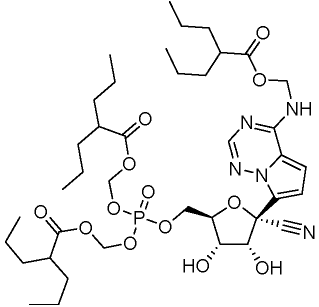 | ¹H NMR: (300 MHz, CD₃OD) δ 8.20 (s, 1H), 7.28 (d, J = 3.3 Hz, 1H), 6.96 (d, J = 2.7 Hz, 1H), 6.00 (s, 2H), 5.67 - 5.62 (m, 4H), 4.74 - 4.72 (m, 1H), 4.43 - 4.30 (m, 3H), 4.14 - 4.11 (m, 1H), 2.47 - 2.43 (m, 3H), 1.66 - 1.10 (m, 24H), 0.91 - 0.82 (m, 18H). |

### Biological Example

### Biological Example 1: Cytopathic Experiment (CPE)

Cells were seeded into microplates at a certain density and cultured overnight in a incubator at 5% CO₂, 37°C. The next day, double-diluted compound and virus were added. It was set up a cell control group (cells without treatment with a compound or virus infection), a virus control group (cells infected with virus, no compound treatment) and a culture medium control group (medium only). Cells were cultured in the incubator for 3 days (229E) or 7 days (OC43). Cytotoxicity experiments were performed under the same conditions as antiviral experiments, but without virus infection. Cell viability was tested using a cell viability assay kit Cell Titer Glo (Promega).

The antiviral activity and cytotoxicity of the compounds were expressed by the inhibition rate (%) and cell viability (%) of the compounds against virus-induced cytopathic effects at different concentrations, respectively. Nonlinear fitting analysis on the inhibition rate and cell viability of the compounds were carried out with the GraphPad Prism, and the half effective concentration (EC₅₀), the 90% effective concentration (EC₉₀), the half cytotoxic concentration (CC₅₀) and the 90% cytotoxic concentration of the compound (EC₉₀) values were calculated.

Activity results for exemplary compounds were shown in Table I below.

**Table I: activity results of examplary compounds**

| Compound No. | 229E MRC5 CPE assay (µM) | | OC43 Huh7 CPE assay (µM) | |
|---|---|---|---|---|
| | EC₅₀ | EC₉₀ | EC₅₀ | EC₉₀ |
| 057 | C | C | B | C |
| 077 | A | C | C | C |
| 145 | A | B | B | B |
| 094 | C | C | C | C |
| 141 | C | C | C | C |
| 142 | C | C | C | C |
| 129 | A | A | A | C |
| 130 | A | B | A | B |
| 146 | C | C | C | C |
| 147 | C | C | C | C |
| 126 | A | B | A | B |
| 140 | C | C | C | C |
| 143 | C | C | C | C |
| 144 | C | C | C | C |
| 149 | C | C | C | C |
| 150 | C | C | C | C |
| 156 | B | C | C | C |
| 157 | B | C | C | C |
| 158 | C | C | C | C |
| 179 | C | C | C | C |
| 180 | C | C | C | C |
| 181 | C | C | C | C |
| 208 | C | C | B | B |
| 209 | C | C | C | B |
| 210 | A | A | A | B |
| 211 | A | A | B | C |
| 197 | B | C | C | C |
| 198 | B | C | C | C |
| 199 | C | C | C | C |
| 200 | C | C | C | C |
| 001 | A | A | A | A |
| 070 | B | B | B | C |
| 071 | A | A | A | A |
| 034 | B | C | B | C |
| 074 | A | A | A | B |
| 075 | B | B | B | C |
| 122 | B | B | B | B |
| 123 | B | B | B | C |
| 036 | B | B | B | C |
| 095 | A | B | A | B |
| 096 | A | A | B | B |
| 097 | A | B | B | B |
| 098 | A | A | A | B |
| 120 | A | B | A | B |
| 121 | B | B | B | C |
| 137 | A | B | A | A |
| 138 | A | B | A | A |
| 139 | A | B | A | A |
| 028 | C | C | C | C |
| 060 | C | C | C | C |
| 065 | C | C | C | C |
| 076 | A | B | B | B |
| 068 | C | C | C | C |
| 069 | C | C | C | C |
| 103 | C | C | C | C |
| 104 | C | C | C | C |
| 107 | C | C | C | C |
| 128 | C | C | C | C |
| 016 | C | C | C | C |
| 062 | C | C | C | C |
| 063 | C | C | C | C |
| 086 | C | C | C | C |
| 087 | C | C | C | C |
| 088 | C | C | C | C |
| 003 | B | C | C | C |
| 160 | C | C | C | C |
| 161 | C | C | C | C |
| 162 | C | C | C | C |
| 163 | C | C | C | C |
| 164 | C | C | C | C |
| 174 | C | C | C | C |
| 175 | C | C | C | C |
| 176 | C | C | C | C |
| 177 | C | C | C | C |
| 192 | B | C | B | C |
| 193 | B | C | B | C |
| 194 | B | C | B | C |
| 195 | B | C | B | C |
| 196 | B | C | B | C |
| 212 | B | C | B | C |
| 214 | C | C | C | C |
| 215 | C | C | C | C |
| 216 | C | C | C | C |
| 217 | C | C | C | C |
| 218 | C | C | C | C |
| 219 | C | C | C | C |
| 220 | C | C | C | C |
| 221 | C | C | C | C |
| 222 | B | C | C | C |
| 223 | B | C | C | C |
| 224 | C | C | C | C |
| 225 | C | C | C | C |
| 226 | C | C | C | C |
| 227 | C | C | C | C |
| 228 | C | C | C | C |
| 229 | C | C | C | C |
| 230 | C | C | C | C |
| 231 | C | C | C | C |
| 232 | C | C | C | C |
| 233 | C | C | C | C |
| 236 | A | A | A | A |
| 237 | A | A | A | B |
| 238 | A | A | A | A |
| 239 | A | A | A | B |
| 260 | B | B | C | C |
| 261 | B | C | C | C |
| 262 | B | C | C | C |
| 263 | A | A | B | C |
| 264 | A | B | C | C |
| 265 | C | C | | |
| 266 | C | C | C | C |
| 267 | C | C | C | C |
| 268 | C | C | C | C |
| 269 | C | C | C | C |
| 270 | C | C | C | C |
| 276 | B | C | B | C |
| 277 | A | B | | |
| 155 | B | C | C | C |
| Remdesivir | A | B | A | A |

wherein, A indicates < 0.1 µM, B indicates > 0.1 µM and < 0.5 µM, C indicates > 0.5 µM.

Embodiments of the present invention have been described above. However, the present invention is not limited to the above-described embodiments. Any modification, equivalents, improvement, etc. made within the spirit and principle of the present invention shall be comprised within the protection scope of the present invention.

## Claims

1. A compound of formula (X) or (XI) or an isotopically-labeled compound, optical isomer, geometric isomer, tautomer or mixture of isomers, or a pharmaceutically acceptable salt, prodrug, metabolite, polymorph or solvate thereof, 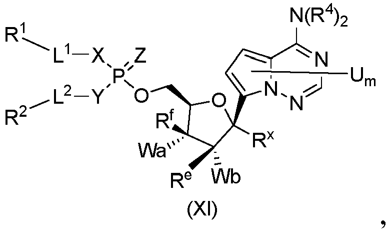 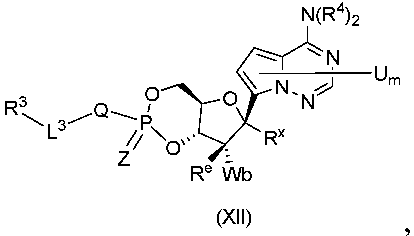 wherein,
U, independently of each other, represents H, D, halogen, C₁-C₁₂ alkyl or C₁-C₁₂ haloalkyl; m, independently of each other, represents an integer from 0 to 3, preferably represents 0, 1 or 2, more preferably represents 0 or 1;
R^{x}, independently of each other, represents H, OH, -OC₁-C₁₂ alkyl, CN, nitro, amino, halogen, C₁-C₁₂ haloalkyl;
Wa and Wb, independently of each other, represent H, OH, the following groups unsubstituted or substituted with one, two or more R^{w}: C₁-C₁₂ alkyl, C₂-C₁₂ alkenyl, C₂-C₁₂ alkynyl, C₁-C₁₂ alkyloxy, C₂-C₁₂ alkenyloxy, C₂-C₁₂ alkynyloxy, C₁-C₁₂ alkylcarbonyloxy, C₂-C₁₂ alkenylcarbonyloxy, C₂-C₁₂ alkynylcarbonyloxy, 5-12 membered heteroarylamino, benzimidamido, C₆-C₁₄ aryl, C₁-C₁₂ alkyloxycarbonyloxy, C₁-C₁₂ alkylcarbonylthio, C₆-C₂₀ aryl-C₁-C₁₂ alkylcarbonyloxy, C₃-C₁₂ cycloalkylcarbonyloxy, C₆-C₁₄ arylcarbonyloxy, C₃-C₁₂ cycloalkyloxycarbonyl, carboxyl, amino, 5-12 membered heteroarylcarbonyloxy, C₆-C₂₀ arylcarbonyloxy, 5-12 membered heterocyclylcarbonyloxy; wherein, R^{w}, independently of each other, represents di(C₁-C₆ alkyl)aminosulfonyl, C₁-C₁₂ alkyl, hydroxyl, carboxyl, amino, halogen;
Q, X, Y and Z, independently of each other, represent O, S or NH,
L¹ and L², independently of each other, represents a single bond, the following groups unsubstituted or optionally substituted with one, two or more R^{a}: C₁-C₁₂ alkylene, C₂-C₁₂ alkenylene, C₂-C₁₂ alkynylene, C₃-C₁₂ cycloalkylene, C₆-C₁₄ arylene, 5-12 membered heteroarylene, -(O-C₁-C₁₂ alkylene)ₙ-, -(C₁-C₁₂ alkylene-O-)ₙ-, C₁-C₁₂ alkylene-NH-C₁-C₁₂ alkylene, C₁-C₁₂ alkylene-C₆-C₁₄ arylene, C₆-C₁₄ arylene-C₁-C₁₂ alkylene; wherein, R^{a}, independently of each other, represents C₁-C₆ alkyl, C₁-C₆ haloalkyl, phenyl, aminoC₁-C₆ alkyl, di(C₁-C₆ alkyl)aminoC₁-C₆ alkyl, benzyloxycarbonylamino; n, independently of each other, represents an integer from 1 to 6, preferably an integer from 4 to 6;
R¹ and R², independently of each other, represents H, the following groups unsubstituted or substituted with one, two or more R^{b}: C₁-C₁₂ alkyl, C₂-C₁₂ alkenyl, C₂-C₁₂ alkynyl, C₁-C₁₂ alkyloxy, C₂-C₁₂ alkenyloxy, C₂-C₁₂ alkynyloxy, C₁-C₁₂ alkylcarbonyloxy, C₂-C₁₂ alkenylcarbonyloxy, C₂-C₁₂ alkynylcarbonyloxy, C₁-C₁₂ alkyloxycarbonyl, C₂-C₁₂ alkenyloxycarbonyl, C₂-C₁₂ alkynyloxycarbonyl, 5-12 membered heteroarylamino, benzimidamido, C₆-C₁₄ aryl, C₁-C₁₂ alkyloxycarbonyloxy, C₁-C₁₂ alkylcarbonylthio, C₆-C₂₀ aryl-C₁-C₁₂ alkylcarbonyloxy, C₃-C₁₂ cycloalkylcarbonyloxy, C₆-C₁₄ arylcarbonyloxy, C₃-C₁₂ cycloalkyloxycarbonyl, carboxyl, amino, 5-12 membered heteroarylcarbonyloxy, C₆-C₂₀ arylcarbonyloxy, 5-12 membered heterocyclylcarbonyloxy; wherein, R^{b}, independently of each other, represents benzyloxycarbonylamino, C₁-C₆ alkyl, C₁-C₆ haloalkyl, hydroxyl, carboxyl, amino, halogen, preferably Cl;
L³ represents a single bond, C₁-C₁₂ alkylene unsubstituted or optionally substituted with one, two or more group R^{c}; wherein R^{c}, independently of each other, represents C₁-C₆ alkyl, C₁-C₆ haloalkyl, hydroxyl, carboxyl, amino, halogen;
R³ represents H, the following groups unsubstituted or substituted with one, two or more group R^{d}: C₁-C₁₂ alkyl, C₂-C₁₂ alkenyl, C₂-C₁₂ alkynyl, C₁-C₁₂ alkyloxy, C₂-C₁₂ alkenyloxy, C₂-C₁₂ alkynyloxy, C₁-C₁₂ alkylcarbonyloxy, C₁-C₁₂ alkyloxycarbonyl, 5-12 membered heteroarylcarbonyloxy, C₆-C₂₀ arylcarbonyloxy, C₁-C₁₂ alkyloxycarbonyloxy, 5-12 membered heterocyclylcarbonyloxy, carboxyl, amino, C₁-C₁₂ alkylcarbonylthio, C₆-C₂₀ aryl-C₁-C₁₂ alkylcarbonyloxy, C₃-C₁₂ cycloalkylcarbonyloxy, C₆-C₁₄ aryloxycarbonyloxy, C₃-C₁₂ cycloalkyloxycarbonyl, C₃-C₁₂ cycloalkyloxycarbonyloxy; wherein, R^{d}, independently of each other, represents di(C₁-C₆ alkyl)aminosulfonyl, preferably di-(n-propyl)aminosulfonyl, C₁-C₆ alkyl, hydroxyl, carboxyl, amino, halogen, preferably Cl;
R⁴ represents H, R¹-L¹-X or R²-L²-Y;
R^{e} and R^{f}, independently of each other, represents C₁-C₁₂ alkyl, C₂-C₁₂ alkenyl, C₂-C₁₂ alkynyl, C₁-C₁₂ alkyloxy, C₂-C₁₂ alkenyloxy, C₂-C₁₂ alkynyloxy, halogen, OH, CN, -NO₂.

2. A compound of formula (I) or (II) or an isotopically-labeled compound, optical isomer, geometric isomer, tautomer or mixture of isomers, or a pharmaceutically acceptable salt, prodrug, metabolite, polymorph or solvate thereof, 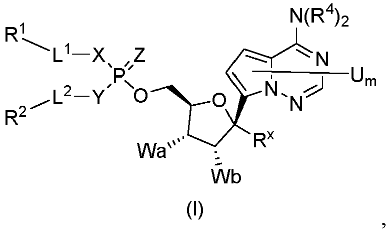 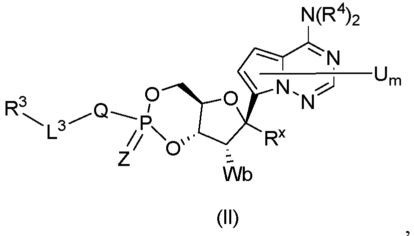 wherein,
U, independently of each other, represents H, D, halogen, C₁-C₁₂ alkyl or C₁-C₁₂ haloalkyl; m, independently of each other, represents an integer from 0 to 3, preferably represents 0, 1 or 2, more preferably represents 0 or 1;
R^{x}, independently of each other, represents H, OH, -OC₁-C₁₂ alkyl, CN, nitro, amino, halogen, C₁-C₁₂ haloalkyl;
Wa and Wb, independently of each other, represent H, OH, the following groups unsubstituted or substituted with one, two or more R^{w}: C₁-C₁₂ alkyl, C₂-C₁₂ alkenyl, C₂-C₁₂ alkynyl, C₁-C₁₂ alkyloxy, C₂-C₁₂ alkenyloxy, C₂-C₁₂ alkynyloxy, C₁-C₁₂ alkylcarbonyloxy, C₂-C₁₂ alkenylcarbonyloxy, C₂-C₁₂ alkynylcarbonyloxy, 5-12 membered heteroarylamino, benzimidamido, C₆-C₁₄ aryl, C₁-C₁₂ alkyloxycarbonyloxy, C₁-C₁₂ alkylcarbonylthio, C₆-C₂₀ aryl-C₁-C₁₂ alkylcarbonyloxy, C₃-C₁₂ cycloalkylcarbonyloxy, C₆-C₁₄ arylcarbonyloxy, C₃-C₁₂ cycloalkyloxycarbonyl, carboxyl, amino, 5-12 membered heteroarylcarbonyloxy, C₆-C₂₀ arylcarbonyloxy, 5-12 membered heterocyclylcarbonyloxy; wherein, R^{w}, independently of each other, represents di(C₁-C₆ alkyl)aminosulfonyl, C₁-C₁₂ alkyl, hydroxyl, carboxyl, amino, halogen;
Q, X, Y and Z, independently of each other, represents O, S or NH,
L¹ and L², independently of each other, represents a single bond, the following groups unsubstituted or optionally substituted with one, two or more R^{a}: C₁-C₁₂ alkylene, C₂-C₁₂ alkenylene, C₂-C₁₂ alkynylene, C₃-C₁₂ cycloalkylene, C₆-C₁₄ arylene, 5-12 membered heteroarylene, -(O-C₁-C₁₂ alkylene)ₙ-, -(C₁-C₁₂ alkylene-O-)ₙ-, C₁-C₁₂ alkylene-NH-C₁-C₁₂ alkylene, C₁-C₁₂ alkylene-C₆-C₁₄ arylene, C₆-C₁₄ arylene-C₁-C₁₂ alkylene; wherein, R^{a}, independently of each other, represents C₁-C₆ alkyl, C₁-C₆ haloalkyl, phenyl, aminoC₁-C₆ alkyl, di(C₁-C₆ alkyl)aminoC₁-C₆ alkyl, benzyloxycarbonylamino; n, independently of each other, represents an integer from 1 to 6, preferably an integer from 4 to 6;
R¹ and R², independently of each other, represents H, the following groups unsubstituted or substituted with one, two or more R^{b}: C₁-C₁₂ alkyl, C₂-C₁₂ alkenyl, C₂-C₁₂ alkynyl, C₁-C₁₂ alkyloxy, C₂-C₁₂ alkenyloxy, C₂-C₁₂ alkynyloxy, C₁-C₁₂ alkylcarbonyloxy, C₂-C₁₂ alkenylcarbonyloxy, C₂-C₁₂ alkynylcarbonyloxy, C₁-C₁₂ alkyloxycarbonyl, C₂-C₁₂ alkenyloxycarbonyl, C₂-C₁₂ alkynyloxycarbonyl, 5-12 membered heteroarylamino, benzimidamido, C₆-C₁₄ aryl, C₁-C₁₂ alkyloxycarbonyloxy, C₁-C₁₂ alkylcarbonylthio, C₆-C₂₀ aryl-C₁-C₁₂ alkylcarbonyloxy, C₃-C₁₂ cycloalkylcarbonyloxy, C₆-C₁₄ arylcarbonyloxy, C₃-C₁₂ cycloalkyloxycarbonyl, carboxyl, amino, 5-12 membered heteroarylcarbonyloxy, C₆-C₂₀ arylcarbonyloxy, 5-12 membered heterocyclylcarbonyloxy; wherein, R^{b}, independently of each other, represents benzyloxycarbonylamino, C₁-C₆ alkyl, C₁-C₆ haloalkyl, hydroxyl, carboxyl, amino, halogen, preferably Cl;
L³ represents a single bond, C₁-C₁₂ alkylene unsubstituted or optionally substituted with one, two or more group R^{c}; wherein R^{c}, independently of each other, represents C₁-C₆ alkyl, C₁-C₆ haloalkyl, hydroxyl, carboxyl, amino, halogen;
R³ represents H, the following groups unsubstituted or substituted with one, two or more group R^{d}: C₁-C₁₂ alkyl, C₂-C₁₂ alkenyl, C₂-C₁₂ alkynyl, C₁-C₁₂ alkyloxy, C₂-C₁₂ alkenyloxy, C₂-C₁₂ alkynyloxy, C₁-C₁₂ alkylcarbonyloxy, C₁-C₁₂ alkyloxycarbonyl, 5-12 membered heteroarylcarbonyloxy, C₆-C₂₀ arylcarbonyloxy, C₁-C₁₂ alkyloxycarbonyloxy, 5-12 membered heterocyclylcarbonyloxy, carboxyl, amino, C₁-C₁₂ alkylcarbonylthio, C₆-C₂₀ aryl-C₁-C₁₂ alkylcarbonyloxy, C₃-C₁₂ cycloalkylcarbonyloxy, C₆-C₁₄ aryloxycarbonyloxy, C₃-C₁₂ cycloalkyloxycarbonyl, C₃-C₁₂ cycloalkyloxycarbonyloxy; wherein, R^{d}, independently of each other, represents di(C₁-C₆ alkyl)aminosulfonyl, preferably di-(n-propyl)aminosulfonyl, C₁-C₆ alkyl, hydroxyl, carboxyl, amino, halogen, preferably Cl;
R⁴ represents H, R¹-L¹-X or R²-L²-Y.

3. The compound of formula (I) or (II) or an isotopically-labeled compound, optical isomer, geometric isomer, tautomer or mixture of isomers, or a pharmaceutically acceptable salt, prodrug, metabolite, polymorph or solvate thereof according to claim 2, wherein, the compound of formula (I) or (II) have a structure of the following formula (IA) or (IIA): 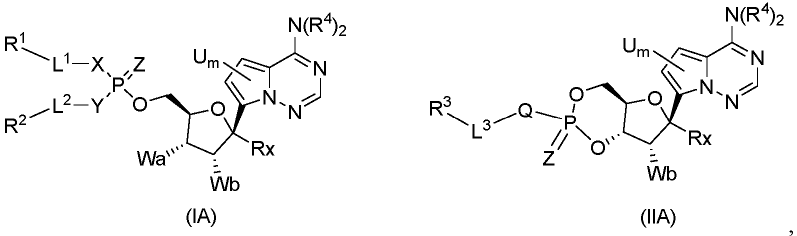 wherein, R¹, R², R³, R⁴, R^{x}, L¹, L², L³, Q, U, Wa, Wb, X, Y, Z are as defined in formula (I) or (II).

4. The compound of formula (I) or (II) or an isotopically-labeled compound, optical isomer, geometric isomer, tautomer or mixture of isomers, or a pharmaceutically acceptable salt, prodrug, metabolite, polymorph or solvate thereof according to claim 2 or 3, wherein, the compound of formula (I) or (II) have a structure of the following formula (IB) or (IIB): 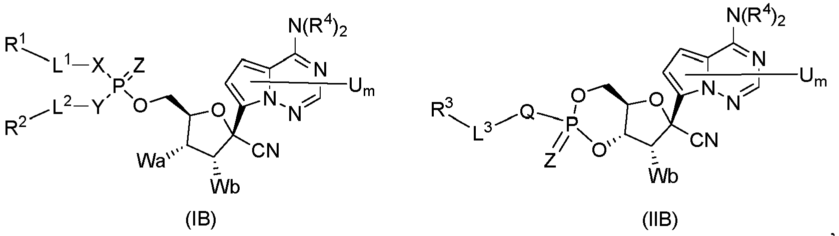 wherein, R¹, R², R³, R⁴, L¹, L², L³, Q, U, Wa, Wb, X, Y, Z are as defined in formula (I) or (II).

5. The compound of formula (I) or (II) or an isotopically-labeled compound, optical isomer, geometric isomer, tautomer or mixture of isomers, or a pharmaceutically acceptable salt, prodrug, metabolite, polymorph or solvate thereof according to any one of claims 1 to 4, wherein, the compound of formula (I) or (II) have a structure of the following formula (IC) or (IIC): 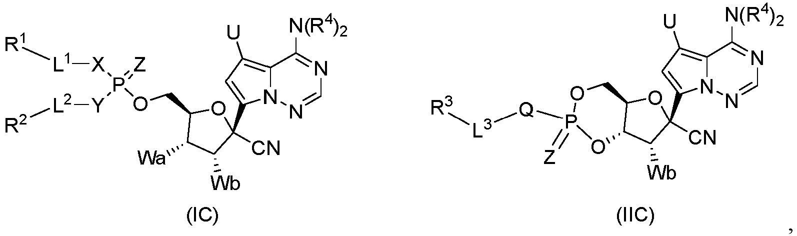 wherein, R¹, R², R³, R⁴, L¹, L², L³, Q, U, Wa, Wb, X, Y, Z are as defined in formula (I) or (II).

6. The compound of formula (I) or (II) or an isotopically-labeled compound, optical isomer, geometric isomer, tautomer or mixture of isomers, or a pharmaceutically acceptable salt, prodrug, metabolite, polymorph or solvate thereof according to any one of claims 1 to 5, wherein, Wa and Wb, independently of each other, represent H, OH, the following groups unsubstituted or substituted with one, two or more R^{w}: C₁-C₆ alkylcarbonyloxy, C₂-C₁₂ alkenylcarbonyloxy, C₂-C₁₂ alkynylcarbonyloxy, C₃-C₁₂ cycloalkylcarbonyloxy, 5-12 membered heteroarylcarbonyloxy, C₆-C₂₀ arylcarbonyloxy, 5-12 membered heterocyclylcarbonyloxy; wherein, R^{w}, independently of each other, represents di(C₁-C₆ alkyl)aminosulfonyl, preferably di(n-propyl)aminosulfonyl, C₁-C₆ alkyl, hydroxyl, carboxyl, amino, halogen; preferably Wa and Wb, independently of each other, represent OH, acetoxy, isopropanoyloxy, isobutyryloxy, 2-propylpentanoyloxy, 2,2-dimethylpropanoyloxy, 4-(di(n-propyl)aminosulfonyl)benzoyloxy, pyridine-3-carbonoyloxy, N-methylpyrrolidin-2-ylcarbonoyloxy, prolyloxy, cyclopentylcarbonoyloxy.

7. The compound of formula (I) or (II) or an isotopically-labeled compound, optical isomer, geometric isomer, tautomer or mixture of isomers, or a pharmaceutically acceptable salt, prodrug, metabolite, polymorph or solvate thereof according to any one of claims 1 to 5, wherein, L¹ and L², independently of each other, preferably represents a single bond, C₁-C₆ alkylene, C₆-C₁₀ arylene, C₃-C₆ cycloalkylene, 5-8 membered heteroarylene, -(O-C₁-C₆ alkylene)ₙ-, C₁-C₆ alkylene-NH-C₁-C₆ alkylene; more preferably, L¹ and L², independently of each other, represents a single bond, methylene, ethylene, propylene, phenylene, cyclopentylene, cyclohexylene, oxadiazolylene, pyrazolylene, imidazolylene, thiazolylene, -(OCH₂CH₂)ₙ-, C₁-C₆ alkylene-NH-C₁-C₃ alkylene; most preferably, L¹ and L², independently of each other, represents a single bond, methylene, 1,1-ethylene, 2,2-propylene, 2-phenyl-1,1-ethylene, 1,4-phenylene, 1,2,4-oxadiazol-3,5-diyl, -(OCH₂CH₂)₄-, pentylene-N(C₂H₅)-CH₂CH₂-; and/or
R¹ and R², independently of each other, preferably represents H, the following groups unsubstituted or substituted with one, two or more R^{b}: C₁-C₆ alkyl, C₆-C₁₂ alkylcarbonyloxy, C₁-C₆ alkyloxycarbonyl, C₃-C₆ cycloalkylcarbonyloxy, C₃-C₆ cycloalkyloxycarbonyl, 5-12 membered heteroarylamino, benzimidamido, C₆-C₁₄ aryl, C₁-C₆ alkyloxycarbonyloxy, C₃-C₆ cycloalkylcarbonyloxy, C₃-C₆ cycloalkyloxycarbonyl, carboxyl, amino, 5-12 membered heteroarylcarbonyloxy, 5-12 membered heterocyclylcarbonyloxy, wherein R^{b}, independently of each other, represents benzyloxycarbonylamino, C₁-C₆ alkyl, C₁-C₆ haloalkyl, hydroxy, carboxyl, amino, fluoro, chloro or bromo; more preferably, R¹ and R², independently of each other, represents H, methyl, heptylcarbonyloxy, methyloxycarbonyl, ethyloxycarbonyl, propyloxycarbonyl, butyloxycarbonyl, hexyloxycarbonyl, quinolinylamino, benzimidamido, propyloxycarbonyloxy, carboxyl, amino, nicotinoyloxy, N-methyl-hydroxylpyrrolidinylcarbonyl; most preferably, R¹ and R² are independently of each other selected from the following atoms or groups:
H, phenyl, methyl, carboxyl, amino, 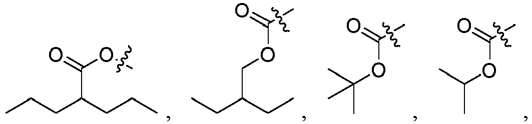 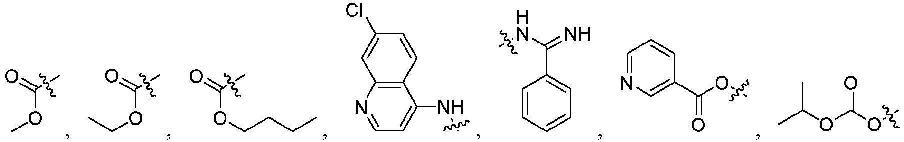 or 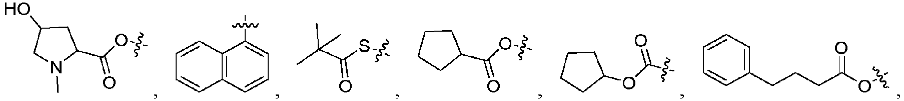 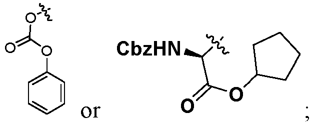 and/or
L³ preferably represents a single bond or C₁-C₆ alkylene unsubstituted or optionally substituted with one, two or more group R^{c}, wherein R^{c}, independently of each other, represents C₁-C₆ alkyl, C₁-C₆ haloalkyl, hydroxy, carboxyl, amino, halogen; more preferably, L³ represents a single bond, methylene or ethylene; most preferably, L³ represents a single bond, methylene, 1,1-ethylene; and/or
R³ preferably represents H, the following groups unsubstituted or substituted with one, two or more group R^{d}: C₁-C₆ alkyl, C₆-C₁₂ alkylcarbonyloxy, C₁-C₆ alkyloxycarbonyl, 5-12 membered heteroarylcarbonyloxy, benzimidamido, C₆-C₁₄ arylcarbonyloxy, C₁-C₆ alkyloxycarbonyloxy, 5-12 membered heterocyclylcarbonyloxy, N-methyl-hydroxylpyrrolidinylcarbonyl, carboxyl, amino, C₁-C₆ alkylcarbonylthio, C₆-C₁₄ aryl-C₁-C₆ alkylcarbonyloxy, C₆-C₁₄ aryloxycarbonyloxy, C₃-C₆ cycloalkyloxycarbonyloxy; more preferably, R³ represents H, methyl, heptylcarbonyloxy, methyloxycarbonyl, ethyloxycarbonyl, propyloxycarbonyl, butyloxycarbonyl, hexyloxycarbonyl, propyloxycarbonyloxy, isopropyloxycarbonyloxy, benzoyl, nicotinoyloxy, carboxyl, amino, tert-butylcarbonylthio, phenylpropylcarbonyloxy, phenoxycarbonyloxy, cyclobutyloxycarbonyloxy, cyclopentyloxycarbonyloxy; most preferably, R³ is selected from the following atoms or groups: H, phenyl, methyl, carboxyl, amino, 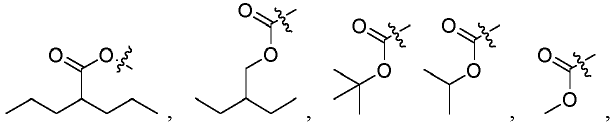 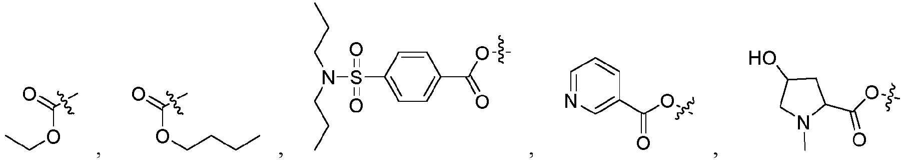 or 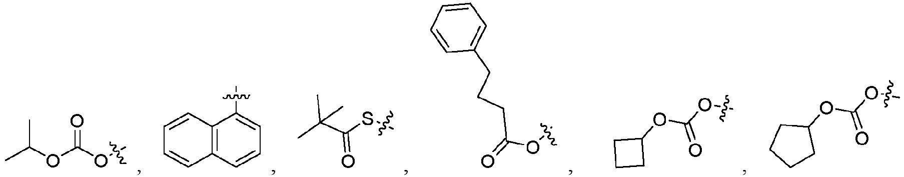 or

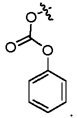

8. The compound of formula (I) or (II) or an isotopically-labeled compound, optical isomer, geometric isomer, tautomer or mixture of isomers, or a pharmaceutically acceptable salt, prodrug, metabolite, polymorph or solvate thereof according to any one of claims 1 to 6, wherein, R¹-L¹-X- and R²-L²-Y-, independently of each other, represents the following groups:
OH, 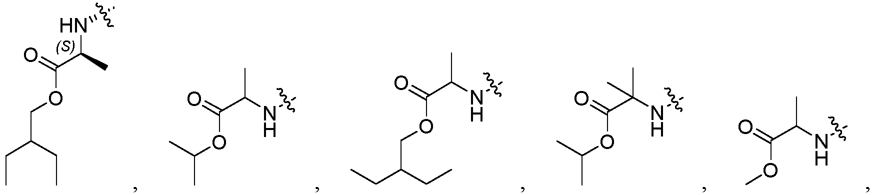 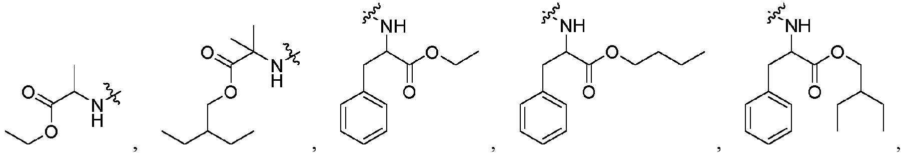 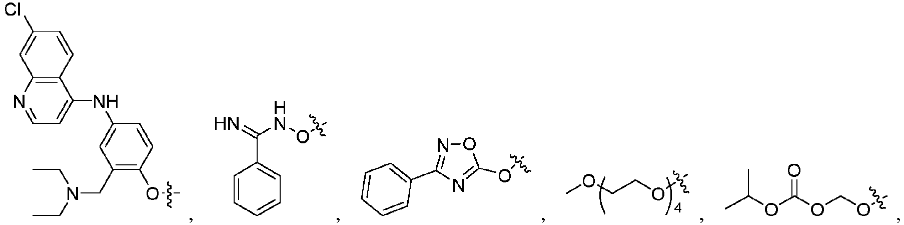 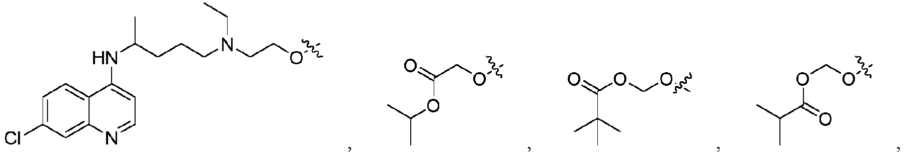 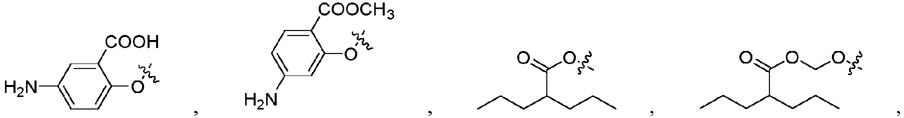 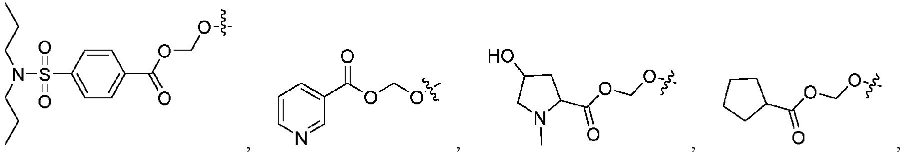 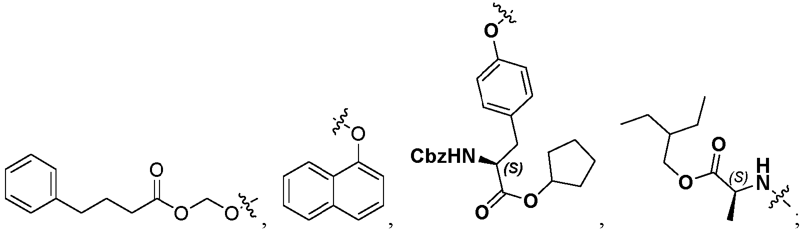 and/or
R³-L³-Q- represents the following groups:
OH,

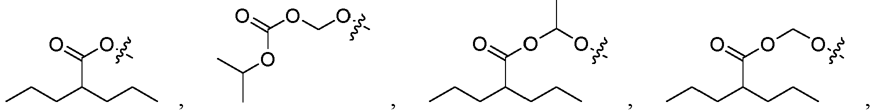

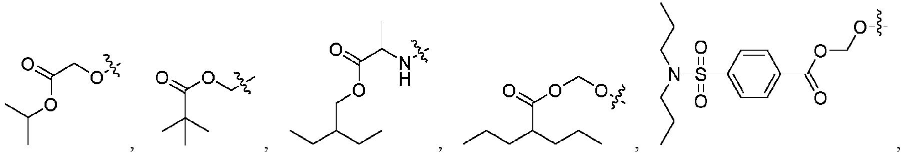

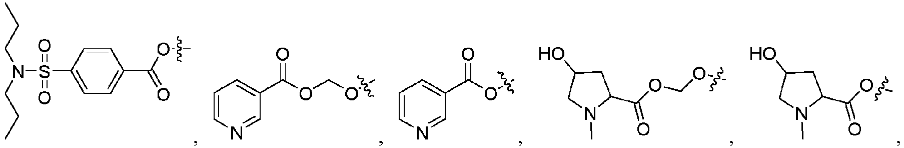

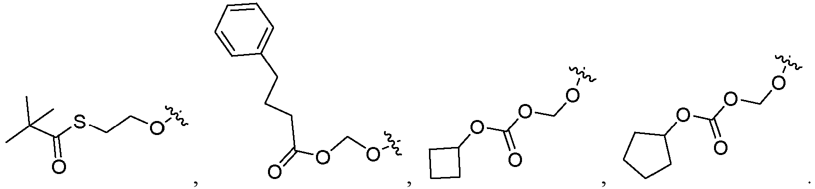

9. A pharmaceutical composition comprising a compound of formula (I) or (II), or an isotopically-labeled compound, optical isomer, geometric isomer, tautomer isomer or a mixture of isomers, or a pharmaceutically acceptable salt, prodrug, metabolite, polymorph or solvate thereof according to any one of claims 1 to 7, and/or optionally contain at least one physiologically/pharmaceutically acceptable excipient and/or optionally comprise additional active ingredients; preferably, the additional active ingredient is, for example, a fusion inhibitor, a viral entry inhibitor, a protease inhibitor, a polymerase inhibitor, an antiviral nucleoside drug and derivates thereof other than remdesivir, a viral maturation inhibitor, JAK inhibitor, angiotensin converting enzyme 2 (ACE2) inhibitor, a SARS-CoV specific human monoclonal antibody, a compound for inhibiting cytokine storm, preferably ribavirin, sofosbuvir, GS-441524, palivizumab, motavizumab.

10. Use of the compound of formula (I) or (II), or an isotopically-labeled compound, optical isomer, geometric isomer, tautomer isomer or a mixture of isomers, or a pharmaceutically acceptable salt, prodrug, metabolite, polymorph or solvate thereof according to any one of claims 1 to 7 in the manufacture of a medicament; preferably, the medicament optionally comprises additional active ingredient; the additional active ingredient is preferably selected from a fusion inhibitor, a viral entry inhibitor, a protease inhibitor, a polymerase inhibitor, an antiviral nucleoside drug and derivates thereof other than remdesivir, a viral maturation inhibitor, JAK inhibitor, angiotensin converting enzyme 2 (ACE2) inhibitor, a SARS-CoV specific human monoclonal antibody, and a compound for inhibiting cytokine storm, more preferably ribavirin, sofosbuvir, GS-441524, palivizumab, or motavizumab; preferably, the medicament is used for inhibiting the replication of RNA virus, preferably orthomyxovirus (for example, influenza virus) or paramyxovirus (for example, coronavirus or respiratory syncytial virus) and/or for preventing or treating a disease or symptom caused by RNA virus, preferably orthomyxovirus (for example, influenza virus) or paramyxovirus (for example, coronavirus or respiratory syncytial virus); more preferably, the coronavirus is selected from severe acute respiratory syndrome coronavirus (SARS-CoV), novel coronavirus (SARS-CoV-2), Middle East respiratory syndrome coronavirus (MERS-CoV), coronavirus 229E (HCoV-229E), coronavirus OC43 (HCoV-OC43), murine hepatitis coronavirus (MHV), human coronavirus NL63, human coronavirus HKUl, and coronaviruses with more than 85% homology and viral activity with any of the above coronaviruses, or the coronavirus is selected from porcine epidemic diarrhea virus (PEDV) or feline infectious peritonitis virus (FIPV); most preferably, the coronavirus is the novel coronavirus (SARS-CoV-2).

11. A method of forming a prodrug of a nucleoside drug, optionally comprising the steps of:
(S-A) reacting the p-nitrophenyl phosphate compound (V) with a (deoxy)nucleoside drug (VI) having free hydroxyl groups at positions C5 to obtain a prodrug compound of formula (VII), 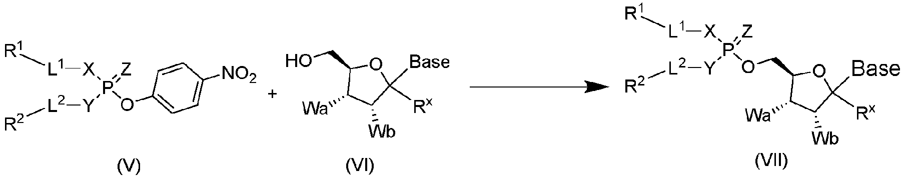
wherein, R¹, R², R^{x}, L¹, L², Wa, Wb, X, Y, Z are as defined in any one of claims 1 to 6, the base may be selected from purine or pyrimidine bases or any basic (hetero)aryl group, preferably selected from guanine, adenine, thymine, uracil, cytosine bases or 4-aminopyrrolo[2,1-f][1,2,4]triazin-7-yl;
preferably, step (S-A) is performed under basic conditions; more preferably, step (S-A) is performed under basic conditions and in the presence of a Grignard reagent; most preferably, step (S-A) is performed in the presence of tert-butylmagnesium bromide;
or,
(S-B) reacting the (deoxy)nucleoside drug phosphate (IX) with a phenolic compound (VIII) to obtain a prodrug compound of formula (X), 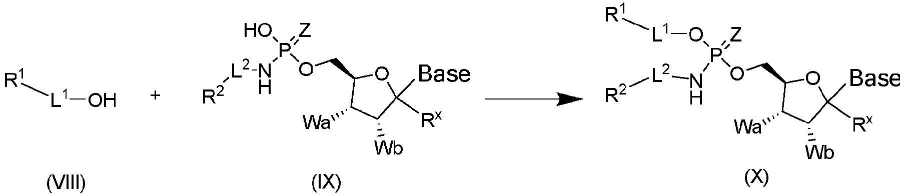 wherein, R¹, R², R^{x}, L¹, L², Wa, Wb, Z are as defined in any one of claims 1 to 6, L¹ represents C₆-C₁₄ arylene or 5-12 membered heteroarylene, preferably C₆-C₁₀ arylene or 5-8 membered heteroarylene, more preferably 1,4-phenylene or 1,2,4-oxadiazol-3,5-diyl, the base may be selected from purine or pyrimidine bases or any basic (hetero)aryl group, preferably selected from guanine, adenine, thymine, uracil, cytosine bases or 4-aminopyrrolo[2,1-f][1,2,4]triazin-7-yl;
or,
(S-C) converting the (deoxy)nucleoside drug phosphate (XI) into a silver salt (XII) firstly and then reacting the silver salt (XII) with the halides R¹-L¹-Hal and/or R²-L²-Hal, to obtain a prodrug compound of formula (XIII), 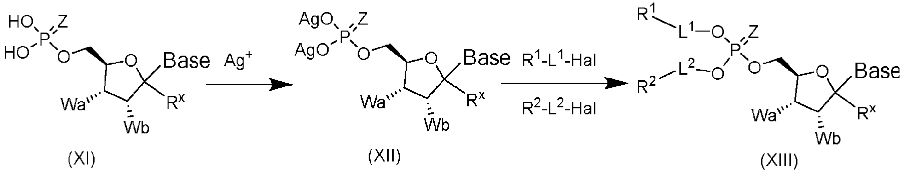 wherein, R¹, R², R^{x}, L¹, L², Wa, Wb, X, Y, Z are as defined in any one of claims 1 to 6, the base may be selected from purine or pyrimidine bases or any basic (hetero)aryl group, preferably selected from guanine, adenine, thymine, uracil, cytosine bases or 4-aminopyrrolo[2,1-f][1,2,4]triazin-7-yl; Hal represents halogen, preferably Br or I, more preferably I; preferably, the silver salt is silver carbonate;
or,
(S-D) reacting cyclic (deoxy)nucleoside drug phosphate (XIV) with halide R³-L³-Hal in the presence of a basic compound, to obtain a prodrug compound of formula (XV), 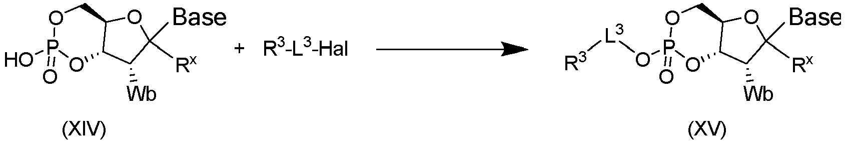 wherein, R^{x}, R³, L³ and Wb are as defined in any one of claims 1 to 6, the base may be selected from purine or pyrimidine bases or any basic (hetero)aryl group, preferably selected from guanine, adenine, thymine, uracil, cytosine bases or 4-aminopyrrolo[2,1-f][1,2,4]triazin-7-yl; Hal represents halogen, preferably Br or Cl, more preferably Cl; the basic compound is preferably an alkali metal carbonate, more preferably cesium carbonate;
or,
(S-E) converting the (deoxy)nucleoside drug phosphate (XVI) into a silver salt (XVII) firstly and then reacting the silver salt (XVII) with the halide R³-L³-Hal, to obtain a prodrug compound of formula (XV), 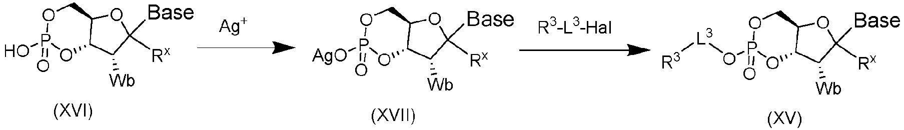 wherein, R^{x}, R³, L³ and Wb are as defined in any one of claims 1 to 6, the base may be selected from purine or pyrimidine bases or any basic (hetero)aryl group, preferably selected from guanine, adenine, thymine, uracil, cytosine bases or 4-aminopyrrolo[2,1-f][1,2,4]triazin-7-yl; Hal represents halogen, preferably Br or I, more preferably I;
or,
(S-F) reacting the phosphoramidite compound (XVIII) with a (deoxy)nucleoside drug (VI) having free hydroxyl groups at positions C3 and C5 to obtain a prodrug compound of formula (XIX), 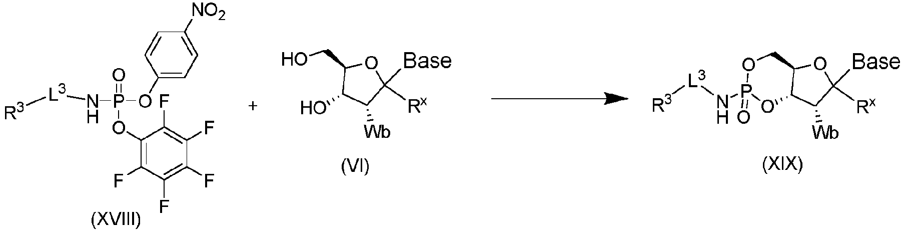 wherein, R^{x}, R³, L³ and Wb are as defined in any one of claims 1 to 6, the base may be selected from purine or pyrimidine bases or any basic (hetero)aryl group, preferably selected from guanine, adenine, thymine, uracil, cytosine bases or 4-aminopyrrolo[2,1-f][1,2,4]triazin-7-yl; preferably, step (S-F) is performed under basic conditions; more preferably, step (S-F) is performed under basic conditions and in the presence of a Grignard reagent; most preferably, step (S-F) is performed in the presence of tert-butylmagnesium bromide.

12. The method according to claim 10, wherein, Wb represents OH**,** R^{x} represents cyano and the base is 4-aminopyrrolo[2,1-f][1,2,4]triazin-7-yl.
